# EUROPEAN PATENT APPLICATION

(11) **EP 4 089 184 A1**
(43) Date of publication of application: **16.11.2022**
(21) Application number: 21738110.2
(22) Date of filing: 08.01.2021
(51) Int. Cl.: C12Q 1/6886, C12N 15/11

(54) **DISPOSABLE REAGENT KIT FOR DETECTING MULTIPLE GENETIC MUTATIONS OF LUNG CANCER**

(30) Priority: 10.01.2020 CN 202010029160
(71) Applicant: Amoy Diagnostics Co., Ltd, Xiamen, Fujian 361027 (CN)
(72) Inventor: JIANG, Fengge, Xiamen, Fujian 361027 (CN); HUANG, Gemu, Xiamen, Fujian 361027 (CN); CHEN, Ning, Xiamen, Fujian 361027 (CN); LI, Shuoqing, Xiamen, Fujian 361027 (CN); CAI, Asha, Xiamen, Fujian 361027 (CN); ZHENG, Weiwei, Xiamen, Fujian 361027 (CN); LIN, Xiaohong, Xiamen, Fujian 361027 (CN); SONG, Qingtao, Xiamen, Fujian 361027 (CN); ZHENG, Limou, Xiamen, Fujian 361027 (CN)
(74) Representative: Hoppe, Georg Johannes
(86) International application number: PCT/CN2021/070914
(87) International publication number: WO 2021/139783

(57) **Abstract**

Disclosed is a disposable composition for detecting multiple genetic mutations of lung cancer and applications of the composition, comprising primers and probes used for detecting EGFR, KRAS, BRAF, HER2, ALK, ROS1, RET, NTRK1, NTRK2, NTRK3, and MET genetic mutations of lung cancer, and distribution schemes of the primers and probes. The present invention employs a PCR 8-tube strip design, every two PCR 8-tube strips detect 11 genetic mutation/fusion states of one sample, where one PCR 8-tube strip holds a corresponding fusion detection reagent and an internal control reagent, while the other PCR 8-tube strip holds a corresponding mutation detection reagent.

## Description

### FIELD OF THE DISCLOSURE

The present disclosure relates to a field of biotechnology, and more specifically relates to a primer, a probe, a test system and a reagent kit for testing multiple lung cancer genetic mutations at one time.

### BACKGROUND OF THE DISCLOSURE

Lung cancers are one of conventional malignant tumors that seriously endanger human health, and 80-85% of lung cancers are non-small cell lung cancer (NSCLC). Many types of genetic mutations are present in NSCLC patients, among which mutation rates of EGFR, HER2, KRAS, and BRAF genes are about 10-50%, 1-4%, 5-25%, 1-2%, fusion rates of ALK, ROS1, RET, NTRK1, NTRK2, and NTRK3 genes are about 3-7%, 1%, 1%, 0.12%, 0.02%, 0.08%, and a mutation rate of MET exon 14 skipping genetic is about 1%. A large number of clinical studies have shown that a status of driver mutations is an important predictor of a curative effect of a targeted drug therapy. Patients with EGFR, HER2, KRAS, and BRAF genetic mutations can benefit from a corresponding treatment with tyrosine kinase inhibitors; patients with ALK, ROS 1, RET, NTRK1, NTRK2, and NTRK3 genetic fusions can benefit from a treatment with tyrosine kinase inhibitors; patients with a MET exon 14 skipping genetic mutation can benefit from a treatment with MET inhibitors. The NCCN Guidelines for Non-Small Cell Lung Cancer clearly pointed out that a status of genetic mutation needs to be tested before targeted therapy, and it is strongly recommended to conduct a wider range of a status test of a status of effective genes. Therefore, a combination test of multiple genetic mutations for NSCLC patients can provide patients more precise treatment. Test results of the reagent kit are only used for clinical reference and should not be used as an only basis for individualized treatment of patients. Clinicians should comprehensively judge test results based on patient's condition, drug indications, treatment response and other laboratory test characteristics.

The genetic mutations or fusion status are related to curative efficiencies of targeted drugs, and a combined test of multiple genes can further improve an accuracy of prediction and prognosis. However, most available reagent kits can only test one or more genetic mutations at one time, a test throughput is low, time-consuming is long, and a demand for the samples is large. Although NGS Panel currently on the market for scientific research purposes may also be used to test the aforementioned 11 genes at one time, a high price of NGS, cumbersome operation, complex data analysis, large usage of samples, and other factors also restrict a popularization and an application of NGS methods. At present, there is no PCR reagent kit that can complete the detection of these 11 hotspot genes at the same time and at one time in clinical practice. The present disclosure provides a primer, a probe, a kit and a distribution method of the primer for testing 11 lung cancer genes at one time with high-sensitivity and high-specificity. The test kit is used to test genetic mutations and genetic fusions respectively using DNA and RNA samples, specificity and sensitivity of the test are high, a reproducibility of test results is good, an operation is simple and efficient, and an efficient and reliable test method for clinical tests of 11 genetic mutations/fusions of EGFR, BRAF, HER2, KRAS, ALK, ROS1, RET, NTRK1, NTRK2, NTRK3 and MET. This method can be used to test 11 genes of lung cancer at one time using a PCR platform, which covers all core genetic targets of targeted drugs that are currently marketed and potential marketed in the next 3-5 years of lung cancer and benefit clinics to enable patients to be benefited from precision medicine using a shortest time and least samples.

### BRIEF SUMMARY OF THE DISCLOSURE

An objective of the present disclosure provides a reagent kit for testing a lung cancer genetic mutation, it comprises:
A reagent for testing sites of an A1 group for a lung cancer genetic fusion, the sites of the A1 group comprise EML4-ALK-1 to EML4-ALK-3, EML4-ALK-6 to EML4-ALK-14, EML4-ALK-17 To EML4-ALK-21; KIF5B-ALK-1; KIF5B-ALK-2; KLC1-ALK; TFG-ALK; STRN-ALK-1; HIP 1-ALK-1; HIP1-ALK-2; HIP1-ALK-3; PRKAR1A-ALK-1; PRKARIA-ALK-2; NBAS-ALK-1; TFG-ALK-2; PPM1B-ALK-1; EIF2AK3-ALK-1; BCLIIA-ALK-1; BIRC6-ALK-1; CEBPZ-ALK-1; CLIPI-ALK-1; COL25AI-ALK-1; GCC2-ALK-1; GCC2-ALK-2; LM07-ALK-1; PICALM-ALK-1; PHACTR1-ALK-1; TPR-ALK-1; MPRIP-ALK-1; TNIP2-ALK-1; DCTN1-ALK-1; SQSTMI-ALK-1;
A reagent for testing sites of an A5 group for the lung cancer genetic fusion, the sites of the A5 group comprise ROS1-M1 to ROSI-MIO;
A reagent for testing sites of a B 1 group of a lung cancer mutation gene, the sites of the B 1 group comprise E-19-M1 to E-19-M21; E-19-M24 to E-19-M27; E-20-M2;
A reagent for testing sites of a B2 group of the lung cancer mutation gene, the sites of the B2 group comprise E-21-Ml; E-18-M1; E-18-M2; E-18-M3; and
A reagent for testing sites of a B3 group of the lung cancer mutation gene, the sites of the B3 group comprise E-20-M1; E-21-M2.

The five combinations of the present disclosure divide fusion genes (a RNA part, A1 and A5, wherein A1 covers 46 fusions of ALK gene of lung cancer genes, A5 covers 10 fusions of ROS1 gene) and mutation genes (B1, B2 and B3 of a DNA part cover 34 mutations of EGFR gene), so that DNA mutations and RNA fusions can be independently tested on a device. For samples that only the DNA mutations or the RNA fusions need to be tested, this design method is more personalized, and waste of reaction reagents is avoided; the operation is more convenient, and wrong additions and missing additions caused by continuous switching of the samples in the same reaction strip are avoided.

ALK and ROS1 fusions are independently tested in one independent tube, and EGFR is divided into three tubes to be tested, precious clinical samples are saved to the greatest extent possible, and an effective distinction of different mutation types between different genes and the same gene is achieved at the same time to meet clinical application better.

An objective of the present disclosure provides a reagent kit for testing a lung cancer genetic mutation, it comprises:
It comprises 15 combinations:
A reagent for testing the following sites of an A1 group comprises EML4-ALK-1 to EML4-ALK-3, EML4-ALK-6 to EML4-ALK-14, EML4-ALK-17 to -EML4-ALK-21; KIF5B-ALK-1; KIF5B-ALK-2; KLC1-ALK; TFG-ALK; STRN-ALK-1; HIP1-ALK-1; HIP1-ALK-2; HIP1-ALK-3; PRKAR1A-ALK-1; PRKAR1A -ALK-2; NBAS-ALK-1; TFG-ALK-2; PPM1B-ALK-1; EIF2AK3-ALK-1; BCL11A-ALK-1; BIRC6-ALK-1; CEBPZ-ALK-1; CLIP1-ALK-1; COL25A1-ALK-1; GCC2-ALK-1; GCC2-ALK-2; LMO7-ALK-1; PICALM-ALK-1; PHACTR1-ALK-1; TPR-ALK-1; MPRIP-ALK-1 ; TNIP2-ALK-1; DCTN1-ALK-1; SQSTM1-ALK-1;
A reagent for testing the following sites of an A2 group comprises NTRK1-E9-M1; NTRK1-E10-M1; NTRK1-E10-M3; NTRK1-E10-M5 to NTRK1-E10-M9; NTRK1-E10-M12; NTRK1-E10-M14; NTRK1-E10-M15; NTRK1-E10-M17; NTRK1-E12-M1; NTRK1-E12-M3; NTRK1-E12-M4; NTRK1-E12-M11; NTRK1-E12-M12; NTRK1-E12- M14;
A reagent for testing the following sites of an A3 group comprises NTRK2-E15-M1; NTRK2-E16-M1; NTRK2-E16-M3; NTRK2-E16-M7; NTRK2-E17-M2;
A reagent for testing the following sites of an A4 group comprises NTRK3-EX14-M1; NTRK3-EX14-M2; NTRK3-EX14-M3; NTRK3-EX14-M4; NTRK3-EX14-M7; NTRK3-EX15-M1; NTRK3- EX15-M2; NTRK3-EX15-M3;
A reagent for testing the following sites of an A5 group comprises ROS1-M1 to ROS1-M10;
A reagent for testing the following sites of an A6 group comprises ROS1-M1 1; ROS1-M12; ROS1-M13; ROS1-EX35-M1 to ROS1-EX35-M4; ROS1-EX35-M6; ROS1-EX35-M8 to ROS1-EX35-M11; ROS1-EX35-M13;
A reagent for testing the following sites of an A7 group comprises MET-M1;
A reagent for testing the following sites of an A8 group comprises RET-M2; RET-M5; RET-M15; RET-M16; RET-M17; RET-M19; LRET-M22; LRET-M32; LRET-M40; LRET-M41; LRET-M42; LRET-M44; LRET-M45; LRET-M49; LRET-M55; LRET-M57; LRET-M58; LRET-M59;
A reagent for testing the following sites of a B1 group comprises E-19-M1 to E-19-M21; E-19-M24 to E-19-M27; E-20-M2;
A reagent for testing the following sites of a B2 group comprises E-21-M1; E-18-M1; E-18-M2; E-18-M3;
A reagent for testing the following sites of a B3 group comprises E-20-M1; E-21-M2;
A reagent for testing the following sites of a B4 group comprises E-20-M4; E-20-M5; E-20-M8; E-20-M9; E-20-M13; E-20-M14; E-20-M20; E-20-M21; E-20-M23; E-20-M24; E-20-M28 to E-20-M31; E-20-M36 to E-20-M41; E-20- M44; E-20-M48; E-20-M50 to E-20-M53; BRAF-M1;
A reagent for testing the following sites of a B5 group comprises E-20-M3; E-20-M10; E-20-M12; E-20-M15 to E-20-M19; E-20-M22; E-20-M25 to E-20-M27; E- 20-M32 to E-20-M35; E-20-M42; E-20-M43; E-20-M45 to E-20-M47; E-20-M49; E-20-M55 to E-20- M57; KRAS-M6;
A reagent for testing the following sites of a B6 group comprises HER2-M1 to HER2-M3; HER2-M8; HER2-M9; HER2-M15 to HER2-M26; KRAS-M2; KRAS-M3; KRAS-M5; KRAS - M14;
A reagent for testing the following sites of a B7 group comprises HER2-M4; HER2-M6; HER2-M7; HER2-M10; KRAS-M1; KRAS-M4.

The 15 reagent combinations of the reagent kit of the present disclosure cover 7 fusion genes comprising ALK, ROS1, RET, NTRK1, NTRK2, NTRK3 and MET and 4 mutation genes comprising EGFR, KRAS, BRAF and HER2 and comprise a total of 119 genetic fusions and 112 genetic mutations, it specially comprises 83 mutations of EGFR gene, 7 mutations of KRAS gene, 1 mutation of BRAF gene, 21 mutations of HER2 gene, 46 fusions of ALK gene, 23 fusions of ROS1 gene, 18 fusions of RET gene, 18 fusions of NTRK1 gene, 5 fusions of NTRK2 gene, 8 fusions of NTRK3 gene and 1 jumping mutation of MET gene (see Tables 5 and 6 for details).

All licensed multiple-lung-cancer-gene combination reagent kits (a PCR method) or authorized multiple-gene combination reagent kits for lung cancer on the market use PCR 8-strips or 12-strips, and tests for mutation and fusion are placed on different positions of the same reaction strip. An entity of the reaction strip needs to be put into a PCR device for amplification in a machine test.

The 15 combinations of the reagent kit of the present disclosure plus an external control can be divided into two PCR reaction 8-strips, that is, a reagent in the reagent kit for one person can comprises the two PCR reaction 8-strips, the fusion genes (the RNA part) ) and the mutation genes (the DNA part) respectively have an independent PCR reaction 8-strips, so that DNA mutations and the RNA fusions can be independently tested on a device. For samples that only the DNA mutations or the RNA fusions need to be tested, this design method is more personalized, and waste of reaction reagents is avoided; the operation is more convenient, wrong additions and missing additions caused by continuous switching of the sample in the same reaction strip are avoided. Further, consumables of the reaction 8-strips have wider applicability, a needed device is simpler, and clinical use is easily promoted.

When the reagent kit is combined at the test sites, clinical purposes of the gene, needs from the pharmaceutical company, ease of operation, interaction between different genes, methodological feasibility, best performance indicators of each gene site and other aspects are comprehensively considered. For example, it is necessary to consider whether different genes, different exons of the same gene, and different codons have different clinical purposes. The genes/sites with the same clinical purpose should be combined as much as possible, and the sites with different clinical purposes should be independently distinguished. For example, objective response rate and disease-free progression survival of patients with EGFR T790M mutation using first-generation EGFR-TKIs have significantly worse than other EGFR-sensitive mutations, but are sensitive to a third-generation EGFR inhibitor, Osimertinib; For another example, KRAS genetic mutation, it is an unfavorable factor affecting a curative efficacy of TKI targeted drugs. As long as there is the KRAS genetic mutation, conventional targeted drugs against EGFR, ALK, ROS1 and other genes cannot be used. In addition, lung cancer patients with the KRAS genetic mutation using chemotherapy drugs are often ineffective, a chemotherapy of Pemetrexed, Paclitaxel and other first-line drugs combined with a platinum system is less effective than patients without KRAS mutation. However, in recent years, a number of targeted or immunotherapy drugs have shown excellent clinical data in the patients with the KRAS mutation, especially KRAS G12C. AMG 510 is the first KRAS G12C inhibitor that reaches clinical stage in recent years. Results of Phase I study of the AMG 510 shows that a total ORR (objective response rate) of the AMG 510 in KRAS-mutant NSCLC (non-small cell lung cancer) patients is 48%, DCR (disease control rate) is 96%. In 13 NSCLC patients having a received dosage of 960 mg of the AMG 510, ORR is 54%, DCR is 100%, and a clinical trial is about to be officially launched. Therefore, when the reagent kit is designed, the sites need to be isolated to function as an independent tube to be tested, and can be distinguished.

| | | | | | | | | | |
|---|---|---|---|---|---|---|---|---|---|
| Fusion reaction strip | Fluorescence signal | ① | ② | ③ | ④ | ⑤ | ⑥ | ⑦ | ⑧ |
| | FAM | ALK | NTRK1 | NTRK2 | NTRK3 | ROS1 | ROS1 | cMet-skipping | RET |
| | | High frequency of hot-spot, independent predictor | collectively called as NTRK genes, test purposes are same | | | test purposes are same | | Independent predictor | Independent predictor |

| | Fluorescence signal | ① | ② | ③ | ④ | ⑤ | | ⑥ | | ⑦ | ⑧ |
|---|---|---|---|---|---|---|---|---|---|---|---|
| Mutation reaction strip | FAM | EGFR 19del | EGFR L858R | EGFR T790M | EGFR 20-Ins | EGFR 20Ins | | HER2 20-ins | | HER2 20-ins | external control |
| | | Exon19, high frequency of hot-spot | Exon21, high frequency of hot-spot | Exon20, high frequency of hot-spot, independent predictor | collectively called as 20ins, test purposes are same | | | | | | A quality of a quality control sample |
| | ROX | EGFR S768I | EGFR G719X | EGFR L861Q | BRAF V600E | | KRAS G12C | | KRA | S KRAS | external control |
| | | Exon20 | Exon18 | Exon21 | Independent predictor | | G12C independent predictor, test purposes of other KRAS sites are same | | | | A quality of a quality control sample |
| | | test purposes of other sites besides T790M are same | | | | | | | | | |

Another objective of the present disclosure is to provide a composition for testing multiple lung cancer genetic mutations at one time, it comprises primers and probes for EGFR, KRAS, BRAF, HER2, ALK, ROS1, RET, NTRK1, NTRK2, NTRK3, MET, other mutation genes, and the primers and the probes are grouped and sub-packaged by the two PCR 8-tube strips, as shown in Table 1:

**Table 1 Special primers, new probes and a distribution method of the lung cancer mutation**

| React ion tube | Name of primers and probes | Sequences of mutant primers and probes | Sequence number |
|---|---|---|---|
| LET PCR 8-tube | LET-A1-F1 | CAGAAGCCCATTATTCAGAGC | SEQ ID NO.001 |
| | LET-A1-R1 | ACTGCTCTAGCAGACTTTTCTG | SEQ ID NO.002 |
| | LET-A1-P1 | VIC-5 '-TTCACCAGGATCCACCTC-3 '-MGB | SEQ ID NO.003 |
| | LET-A1-F2 | TACTGTAGAGCCCACACCTGG | SEQ ID NO.004 |
| strips-geneti c fusion -1 | LET-A1-F3 | TCAACCAAGCAAAAATGTCAACTCG | SEQ ID NO.005 |
| | LET-A1-F4 | GTTACCAAAACTGCAGACAAGCATA | SEQ ID NO.006 |
| | LET-A1-F5 | CCAGACAACAAGTATATAATGTCTAACTCG | SEQ ID NO.007 |
| | LET-A1-F6 | ACACAGACGGGAATGAACAGC | SEQ ID NO.008 |
| | LET-A1-F7 | CTGAAGATCATGTGGCCTCAGT | SEQ ID NO.009 |
| | LET-A1-F8 | TAGGAACGCACTCAGGCAG | SEQ ID NO.010 |
| | LET-A1-F9 | AGGACACTGTGCAGATTTTCATCC | SEQ ID NO.011 |
| | LET-A1-F10 | CATAGGAACGCACTCAGGCA | SEQ ID NO.012 |
| | LET-A1-F11 | AAAGAAACTCTTTCATCTGCTGCTAAAAG | SEQ ID NO.013 |
| | LET-A1-F12 | AGGTCAAAGAATATGGCCAGAAGAG | SEQ ID NO.014 |
| | LET-A1-F13 | CGATGCCCTCAGTGAAGAACTA | SEQ ID NO.015 |
| | LET-A1-F14 | CTCACTCGTGCACATGAAAGG | SEQ ID NO.016 |
| | LET-A1-F15 | ACCTGGAGAACCAGGACCTT | SEQ ID NO.017 |
| | LET-A1-F16 | AGAATTGAATCAGGGAGATATGAAGCC | SEQ ID NO.018 |
| | LET-A1-F17 | ATGGTGCCACCACCTGC | SEQ ID NO.019 |
| | LET-A1-F18 | TGGCCTCAACCATTTCCGG | SEQ ID NO.020 |
| | LET-A1-F19 | AGCACTACGAGCTTGCTGG | SEQ ID NO.021 |
| | LET-A1-F20 | TTTCGGTCTCCTTTATCGCAGG | SEQ ID NO.022 |
| | LET-AL -F21 | GAGTTTGTTGAAGTGGGAAGATTGG | SEQ ID NO.023 |
| | LET-A1-F22 | GGGGAAAATATCAGTGCTTCACCAT | SEQ ID NO.024 |
| | LET-A1-F23 | CAGCCACCATATACAGGAGCTC | SEQ ID NO.025 |
| | LET-A1-F24 | CGAGGCGCCCTAGACATC | SEQ ID NO.026 |
| | LET-A1-F25 | GATCCGGTTCCTTGGTGTCAT | SEQ ID NO.027 |
| | LET-A1-F26 | CGGGTTGGTATCCCTTCAGG | SEQ ID NO.028 |
| | LET-A1-F27 | TGATTTACTGTTCTGGCACAGACA | SEQ ID NO.029 |
| | LET-A1-F28 | ATTCTCAGCAGACAATATCGGATCG | SEQ ID NO.030 |
| | LET-A1-F29 | CTTCAACTCAAAGAAAACAAGAGGCAG | SEQ ID NO.031 |
| | LET-A1-F30 | AAGAATCGCAAGAGAAGCACCTT | SEQ ID NO.032 |
| | LET-A1-F31 | TAAAAATTCAAAAACAGAAACAAGAAACCC TACA | SEQ ID NO.033 |
| | LET-A1-F32 | AAGCACAGCTCTTCCAGCTTAA | SEQ ID NO.034 |
| | LET-A1-F33 | ATGGGATATTCCTGGGATCTTCGTA | SEQ ID NO.035 |
| | LET-A1-F34 | CATGAGACCTCCAAACCCCTTT | SEQ ID NO.036 |
| | LET-A1-F35 | GCCTCCCAAACCCACTACC | SEQ ID NO.037 |
| | LET-A1-F36 | CGTATTTTATTGTCACAAACAACAGGAGT | SEQ ID NO.038 |
| | LET-A1-F37 | GGGGAGAAGTCCCCTGACA | SEQ ID NO.039 |
| | LET-A1-F38 | GTCGCCTCTTTGCTGCAC | SEQ ID NO.040 |
| | LET-A1-F39 | TCCTTCAGGCATTGCTACTCTG | SEQ ID NO.041 |
| | LET-A1-F40 | AAGAACGTTGGGGAGAGTGTG | SEQ ID NO.042 |
| | LET-A1-R2 | GGGCTCTGCAGCTCCAT | SEQ ID NO.043 |
| | LET-A1-P2 | FAM-5'-CGGAAGCACCAGGAG-3 '-MGB | SEQ ID NO.044 |
| LET PCR 8-tube strips-geneti c fusion -2 | LET-A2-F1 | GAAGCCCATTATTCAGAGCG | SEQ ID NO.045 |
| | LET -A2-R1 | TGGTACTGCTCTAGCAGACTT | SEQ ID NO.046 |
| | LET-A2-P1 | VIC-5 '-TTCACCAGGATCCACCTC-3 '-MGB | SEQ ID NO.047 |
| | LET -A2-F2 | GATCGGTAGCCAAGCTGGAA | SEQ ID NO.048 |
| | LET -A2-F3 | GTTGGGGAGAGTGTGGCA | SEQ ID NO.049 |
| | LET -A2-F4 | TACATCACAGACTGTTTCCACTCC | SEQ ID NO.050 |
| | LET -A2-F5 | GAAGTCCAAGTTGCTTCTCAGTCT | SEQ ID NO.051 |
| | LET -A2-F6 | TTCTGGGCTGGGTGTGAC | SEQ ID NO.052 |
| | LET -A2-F7 | TTGTTGGGTTTCGAGGCCAA | SEQ ID NO.053 |
| | LET -A2-F8 | TCGAGAGCAAGTTTAAGAAGGAGC | SEQ ID NO.054 |
| | LET -A2-F9 | TATGTCAGCGTTTGGCTTAACAGA | SEQ ID NO.055 |
| | LET-A2-F10 | GCTATGGGGAGGTCAAGGTC | SEQ ID NO.056 |
| | LET-A2-F 11 | GAGGAAGATCGCCTTGGAGT | SEQ ID NO.057 |
| | LET-A2-F12 | CTTGCGGGAAAAGGAGAGCT | SEQ ID NO.058 |
| | LET-A2-F13 | GAAGTCCCCTGACAGTGCC | SEQ ID NO.059 |
| | LET-A2-F14 | CCACCAGGAACACCCATCAT | SEQ ID NO.060 |
| | LET-A2-F15 | ACACACGAGCTGACCTCTCT | SEQ ID NO.061 |
| | LET-A2-F16 | TGCCAGCGTGAGAACCAG | SEQ ID NO.062 |
| | LET -A2-R2 | CCCAAAAGGTGTTTCGTCCTTC | SEQ ID NO.063 |
| | LET -A2-R3 | CAATGTCATGAAATGCAGGGACAT | SEQ ID NO.064 |
| | LET -A2-P2 | FAM-5'-CAGCACATCTGGAGACC-3'-MGB | SEQ ID NO.065 |
| | LET -A2-P3 | FAM-5'-GGCTCCAGAGGATG-3'-MGB | SEQ ID NO.066 |
| LET PCR 8-tube strips-geneti c fusion -3 | LET-A3-F1 | CCATTATTCAGAGCGAGTATGGA | SEQ ID NO.067 |
| | LET -A3-R1 | TGGTACTGCTCTAGCAGACTT | SEQ ID NO.068 |
| | LET-A3-PI | VIC-5 '-TTCACCAGGATCCACCTC-3 '-MGB | SEQ ID NO.069 |
| | LET-A3-F2 | CAGTCACCAAATTCATCAGTGCC | SEQ ID NO.070 |
| | LET-A3-F3 | AGAGTGTGGCAGCTGCC | SEQ ID NO.071 |
| | LET-A3-F4 | ATCAGGGAGATATGAAGCCTCCAA | SEQ ID NO.072 |
| | LET -A3-R2 | GGGCCAACACCTTGTCTTGA | SEQ ID NO.073 |
| | LET -A3-R3 | CATTGGAGATGTGATGGAGTGGG | SEQ ID NO.074 |
| | LET -A3-R4 | TCCTTCGCCTAGCTCCCTTT | SEQ ID NO.075 |
| | LET -A3-P2 | FAM-5'-TTTGGATTTGGGAAAGTA-3 '-MGB | SEQ ID NO.076 |
| | LET -A3-P3 | FAM-5'-CGTTATCAGCAATGAT-3'-MGB | SEQ ID NO.077 |
| | LET -A3-P4 | FAM-5'-AGCGACATAACATTGTTC-3 '-MGB | SEQ ID NO.078 |
| LET PCR 8-tube strips-geneti c fusion -4 | LET-A4-F1 | CCATTATTCAGAGCGAGTATGGA | SEQ ID NO.079 |
| | LET -A4-R1 | ATGGTACTGCTCTAGCAGAC | SEQ ID NO.080 |
| | LET-A4-P1 | VIC-5 '-TTCACCAGGATCCACCTC-3 '-MGB | SEQ ID NO.081 |
| | LET -A4-F2 | ACAGCCGGAGGTCATACTG | SEQ ID NO.082 |
| | LET -A4-F3 | CTCCCCGCCTGAAGAGC | SEQ ID NO.083 |
| | LET -A4-F4 | AGATCATGTGGCCTCAGTGAAAA | SEQ ID NO.084 |
| | LET -A4-F5 | AACGTTGGGGAGAGTGTGG | SEQ ID NO.085 |
| | LET -A4-F6 | GCCCAACACTGTACCTCAGTT | SEQ ID NO.086 |
| | LET -A4-F7 | AGCAGATGAGGAAGATCGCC | SEQ ID NO.087 |
| | LET -A4-R2 | GATGTGGTGCAGTGGGC | SEQ ID NO.088 |
| | LET -A4-R3 | CTCACCCAGTTCTCGCTTCA | SEQ ID NO.089 |
| | LET -A4-P2 | FAM-5 '-TGGCTGTCATCAGTGGT-3 '-MGB | SEQ ID NO.090 |
| | LET -A4-P3 | FAM-5'-CATTAAGAGGAGAGACATC-3'-MGB | SEQ ID NO.091 |
| LET PCR | LET -A5-F1 | TATTCAGAGCGAGTATGGAGC | SEQ ID NO.092 |
| | LET -A5-R1 | GTACTGCTCTAGCAGACTTTTCT | SEQ ID NO.093 |
| 8-tube strips-geneti c fusion -5 | LET -A5-P1 | VIC-5 '-TTCACCAGGATCCACCTC-3 '-MGB | SEQ ID NO.094 |
| | LET -A5-F2 | TCGTGTGCTCCCTGGATATTCTTA | SEQ ID NO.095 |
| | LET -A5-F3 | GTCAAGGCTCCTGAGACCTTTG | SEQ ID NO.096 |
| | LET-A5-F4 | CAGGCACTCCTTGGAGCA | SEQ ID NO.097 |
| | LET -A5-F5 | ATCTGATGACTTTGAGCTGTCTGG | SEQ ID NO.098 |
| | LET -A5-F6 | AGGGCAGCAACATCTTTGAGAG | SEQ ID NO.099 |
| | LET-A5-F7 | CGGCTGCAGGACTATGAGG | SEQ ID NO.100 |
| | LET -A5-R2 | TTGAATTTTTACTCCCTTCTAGTAATTTGGGA AT | SEQ ID NO.101 |
| | LET -A5-R3 | TTGTAACAACCAGAAATATTCCAACTATAAT AGTAAGT | SEQ ID NO.102 |
| | LET-A5-P2 | FAM-5'-CCCAAATAAACCAGGCAT-3 '-MGB | SEQ ID NO.103 |
| | LET-A5-P3 | FAM-5′-CCAGAAACAAGTTTCATACTT-3′- MGB | SEQ ID NO.104 |
| LET PCR 8-tube strips-geneti c fusion -6 | LET-A6-F1 | CAGAAGCCCATTATTCAGAGC | SEQ ID NO.105 |
| | LET -A6-R1 | ACTGCTCTAGCAGACTTTTCTG | SEQ ID NO.106 |
| | LET-A6-P1 | VIC-5 '-TTCACCAGGATCCACCTC-3 '-MGB | SEQ ID NO.107 |
| | LET -A6-F2 | CTGAGAGATCGGTAGCCAAGC | SEQ ID NO.108 |
| | LET -A6-F3 | GTCACATCTTCAGGTGCTGGATT | SEQ ID NO.109 |
| | LET -A6-F4 | CCTGGTGCTAGTTGCAAAGACA | SEQ ID NO.110 |
| | LET -A6-F5 | ACGGCTGAAGAAGCAACTGAG | SEQ ID NO.111 |
| | LET -A6-F6 | CAGGCACTCCTTGGAGCAAA | SEQ ID NO.112 |
| | LET -A6-F7 | CACTGAGAAAAGAAGAAGAACAAGCTACA | SEQ ID NO.113 |
| | LET -A6-F8 | CTGCGGCTGCAGGACTAT | SEQ ID NO.114 |
| | LET -A6-F9 | GTTTCACAGTTACTTTCACTTCCCGA | SEQ ID NO.115 |
| | LET-A6-F10 | GAAAAAGAAGCCCTCAATCACCG | SEQ ID NO.116 |
| | LET-A6-F11 | CAAGAAAAATTGGTTTGCAAGATGAAAGG | SEQ ID NO.117 |
| | LET-A6-F12 | GAGATAGTGTCACCTGCCCTACT | SEQ ID NO.118 |
| | LET-A6-F13 | TCTCAAAAAGAAGATAAATATGAGGAAGAA ATCAAGAT | SEQ ID NO.119 |
| | LET-A6-F14 | CTTGGAACCACCTGGAGAACC | SEQ ID NO.120 |
| | LET -A6-R2 | CTGTCACCCCTTCCTTGGC | SEQ ID NO.121 |
| | LET -A6-P2 | FAM-5'-CATAGAAGATTAAAGAATC-3'-MGB | SEQ ID NO.122 |
| LET PCR 8-tube strips-geneti c fusion -7 | LET-A7-F1 | GAAGCCCATTATTCAGAGCG | SEQ ID NO.123 |
| | LET-A7-R1 | ACTGCTCTAGCAGACTTTTCTG | SEQ ID NO.124 |
| | LET-A7-P1 | VIC-5 '-TTCACCAGGATCCACCTC-3 '-MGB | SEQ ID NO.125 |
| | LET-A7-F2 | AATGCCTGAGCGGGACATG | SEQ ID NO.126 |
| | LET-A7-R2 | GCCTTGTCCCTCCTTCAAGG | SEQ ID NO.127 |
| | LET-A7-F3 | CAACAGCACTGTTATTACTACTTGGGT | SEQ ID NO.128 |
| | LET-A7-R3 | GATACTGCACTTGTCGGCATG | SEQ ID NO.129 |
| | LET-A7-P2 | FAM-5'-AAGCAAATTAAAGATCAGT-3 '-MGB | SEQ ID NO.130 |
| | LET-A7-Block1 | TTCGCTACGATGCAAGAGTACACACTCCTCA TTTGGATAGGC-NH2 | SEQ ID NO.131 |
| LET PCR 8-tube strips-geneti c fusion -8 | LET-A8-F1 | CAGAAGCCCATTATTCAGAGC | SEQ ID NO.132 |
| | LET -A8-R1 | GTACTGCTCTAGCAGACTTTTCT | SEQ ID NO.133 |
| | LET -A8-P1 | VIC-5 '-TTCACCAGGATCCACCTC-3 '-MGB | SEQ ID NO.134 |
| | LET -A8-F2 | TAAAAGACCTTGCAGAAATAGGAATTGCT | SEQ ID NO.135 |
| | LET -A8-F3 | GGAAGAAAATGAAAAGGAGTTAGCAGC | SEQ ID NO.136 |
| | LET -A8-F4 | CTCCTTTCTTGAAAATAATCTTGAACAGCTC | SEQ ID NO.137 |
| | LET -A8-F5 | CCTGCGCAAACTCTTTGTTCAG | SEQ ID NO.138 |
| | LET -A8-F6 | AGGCACTGCAGGAGGAGA | SEQ ID NO.139 |
| | LET -A8-F7 | TATTGGGCCCTTCCTGGAGAA | SEQ ID NO.140 |
| | LET -A8-F8 | TGTAAAACAAGAAAAAACAGAGGATGGC | SEQ ID NO.141 |
| | LET -A8-F9 | CACTCCCTCCAGCTGGC | SEQ ID NO.142 |
| | LET-A8-F10 | CTGCCTGCCACACATTGTTC | SEQ ID NO.143 |
| | LET-A8-F11 | AAGTGACTCTTCAGATCCCTGCT | SEQ ID NO.144 |
| | LET-A8-F12 | CCACTGGGTCACCCCTTG | SEQ ID NO.145 |
| | LET-A8-F13 | CTGATGGCTTGAAGGCGGAA | SEQ ID NO.146 |
| | LET-A8-F14 | CCTGTCATGAGACCTCCAAACC | SEQ ID NO.147 |
| | LET-A8-F15 | CGAAGTGAAAATAAATTGATTTTAATGAAAA CACAGC | SEQ ID NO.148 |
| | LET-A8-F16 | AAAGGTCGCCTCTTTGCT | SEQ ID NO.149 |
| | LET-A8-F17 | CAGTAAGGCCAAAAATGTGCATACTC | SEQ ID NO.150 |
| | LET-A8-F18 | CCATGAAATGGAAAAGGAGCACTTAAATAA G | SEQ ID NO.151 |
| | LET-A8-F19 | ACCAATCCAGAAAACCTTCCATCG | SEQ ID NO.152 |
| | LET -A8-R2 | TCTCCTAGAGTTTTTCCAAGAACCAAG | SEQ ID NO.153 |
| | LET -A8-P2 | FAM-5'-CCAAAGTGGGAATTC-3 '-MGB | SEQ ID NO.154 |
| LET PCR 8-tube strips-geneti c mutat ion-1 | LET-B1-F1 | GTCTCCAGATCTCAGTAAGGTACG | SEQ ID NO.155 |
| | LET-B1-R1 | GGGAAAGAGTGGTCTCTCATCTC | SEQ ID NO.156 |
| | LET-B1-P1 | VIC-5 '-CTGTCCAAAAGCCATGAA-3 '-MGB | SEQ ID NO.157 |
| | LET-B 1-F2 | CCATGCGAAGCCACACTGACG | SEQ ID NO.158 |
| | LFT-B1-R2 | GGCACACGTGGGGGTTGTGCACCA | SEQ ID NO.159 |
| | LET-B 1-R3 | GGCACACGTGGGGGTTGTGCAAGA | SEQ ID NO.160 |
| | LET-B1-R4 | GGCACACGTGGGGGTTGTGAACGA | SEQ ID NO.161 |
| | LET-B 1-R5 | GGCACACGTGGGGGTTGTGCATGA | SEQ ID NO.162 |
| | \LET-B1-Block1 | CAGCAGGCGGCACACGTGGGGGTTGTCCAC GA-NH2 | SEQ ID NO.163 |
| | LET-B 1-P2 | ROX-5 '-CCATCACGTAGGCTTCCTGG-3 '-BHQ2 | SEQ ID NO.164 |
| | LET-B1-R6 | ATGAGAAAAGGTGGGCCTGAGGT | SEQ ID NO.165 |
| | LET-B 1-F3 | TTCCCGTCGCTATCAAGACATCTCCGAAA | SEQ ID NO.166 |
| | LET-B 1-F4 | CGTCGCTATCAAGGCATCTCCG | SEQ ID NO.167 |
| | LET-B1-F5 | TCCCGTCGCTATCAAAACATCTCCGAAA | SEQ ID NO.168 |
| | LET-B 1-F6 | AATTCCCGTCGCTATCAAGGCTCCGAAAGCC | SEQ ID NO.169 |
| | LET-B 1-F7 | CCCGTCGCTATCAAGGAGCAATCTCCGAA | SEQ ID NO.170 |
| | LET-B1-F8 | CCCGTCGCTATCAAGGAAGCAACATCTCC | SEQ ID NO.171 |
| | LET-B 1-F9 | CCCGTCGCTATCAAGGAACCGAAAGCC | SEQ ID NO.172 |
| | LET-B1-Block2 | TCCCGTCGCTATCAAGGAATTAAGAGAAGCA AC-NH2 | SEQ ID NO.173 |
| | LET-B 1-P3 | FAM-5'-AAGCCAACAAGGAAATCCT-3'-MGB | SEQ ID NO.174 |
| LET PCR 8-tube strips-geneti c mutat ion-2 | LET-B2-F1 | CAACCCTGCCCTGTGCAA | SEQ ID NO.175 |
| | LET-B2-R1 | GGTGGTTCTGGAAGTCCAT | SEQ ID NO.176 |
| | LET-B2-P1 | HEX-5′-AGCATCCAGTGGCGGACATAGTC-3′- BHQ1 | SEQ ID NO.177 |
| | LET -B2-F2 | GCAGCATGTCAAGATCACAGATTTTGTGCG | SEQ ID NO.178 |
| | LET -B2-R2 | ACTTTGCCTCCTTCTGCATGGTATT | SEQ ID NO.179 |
| | LET -B2-P2 | FAM-5′-CAGAAGGAGGCAAAGTAAGGAG-3′- BHQ1 | SEQ ID NO.180 |
| | LET-B2-Block1 | AGATCACAGATTTTGGGCTGGCCAAACTGCT GG-NH2 | SEQ ID NO.181 |
| | LET -B2-R3 | CACCGTGCCGAACGCACCGGAGCT | SEQ ID NO.182 |
| | LET -B2-R4 | TATACGTGCCGAACGCACCGGACCA | SEQ ID NO.183 |
| | LET -B2-F3 | CTGAGGTGACCCTTGTCTCTGTGTTCT | SEQ ID NO.184 |
| | LET -B2-P3 | ROX-5 '-CTCTCTTGAGGATCTTG-3 '-MGB | SEQ ID NO.185 |
| | LET -B2-P4 | ROX-5′-CCCAACCAAGCTCTCTTGAGGATCTT- 3′-BHQ2 | SEQ ID NO.186 |
| | LET-B2-Block2 | ACGCACCGGAGCCCAGCACTTTGT -NH2 | SEQ ID NO.187 |
| LET PCR 8-tube strips-geneti c mutat ion-3 | LET-B3-F1 | GTCTCCAGATCTCAGTAAGGTACG | SEQ ID NO.188 |
| | LET-B3 -R1 | GGGAAAGAGTGGTCTCTCATCTC | SEQ ID NO.189 |
| | LET-B3-P1 | VIC-5 '-CTGTCCAAAAGCCATGAA-3 '-MGB | SEQ ID NO.190 |
| | LET -B3-F2 | CAACAGATTTTGGGCTGGCCAGACA | SEQ ID NO.191 |
| | LET -B3-R2 | GTTAAACAATACAGCTAGTGGGAAGGC | SEQ ID NO.192 |
| | LET -B3-P2 | ROX-5′-CAGAAGGAGGCAAAGTAAGGAGG-3′- BHQ2 | SEQ ID NO.193 |
| | LET-B3-F3 | CTACTCCACCGTGCAGCTCATCCT | SEQ ID NO.194 |
| | LET -B3-F4 | ACTACTCCACCGTGCAACTCATCCT | SEQ ID NO.195 |
| | LET -B3-R3 | TATCTCCCTTCCCTGATTACC | SEQ ID NO.196 |
| | LET-B3-Block1 | CTCACCTCCACCGTGCAGCTCATCAC-NH2 | SEQ ID NO.197 |
| | LET-B3-P3 | FAM-5′-CTCATGCCCTTCGGCTGCCTCC-3′- BHQ1 | SEQ ID NO.198 |
| | LET-B4-F1 | CGTGATGGCCAGCGTGGACGGT | SEQ ID NO.199 |
| | LET -B4-F2 | GATGGCCAGCGTGGACAGCGTGGA | SEQ ID NO.200 |
| | LET -B4-F3 | TGGCCAGCGTGATGGCCAG | SEQ ID NO.201 |
| | LET -B4-F4 | TGGCCAGCGTGGACGGGT | SEQ ID NO.202 |
| | LET -B4-F5 | TGATGGCCAGCGTGGACGGT | SEQ ID NO.203 |
| | LET -B4-F6 | CTACGTGATGGCCAGTGTGG | SEQ ID NO.204 |
| | LET -B4-F7 | TGGCCAGCGTGGACTAC | SEQ ID NO.205 |
| | LET -B4-F8 | TGGCCAGCGTGGACACCA | SEQ ID NO.206 |
| | LET -B4-F9 | ATGGCCAGCGTGGACAAACT | SEQ ID NO.207 |
| LET PCR 8-tube strips-geneti c mutat ion-4 | LET-B4-F10 | TGATGGCCAGCGTGGACGCACC | SEQ ID NO.208 |
| | LET-B4-F11 | TGGCCAGCGTGGACAGCGTCG | SEQ ID NO.209 |
| | LET-B4-F12 | TGATGGCCAGCGTGGACAGTC | SEQ ID NO.210 |
| | LET -B4-R1 | GACATAGTCCAGGAGGCAGCCG | SEQ ID NO.211 |
| | LET-B4-P1 | FAM-5'-CGCCTGCTGGGCATCTGC-3'-BHQ1 | SEQ ID NO.212 |
| | LET-B4-Block1 | GATGGCCAGCGTGGACAACCCC-NH2 | SEQ ID NO.213 |
| | LET-B4-F13 | CAGTAAAAATAGGTGATTTTGGTCTAGCTAC CGA | SEQ ID NO.214 |
| | LET -B4-R2 | GCATCTCAGGGCCAAAAATTTAATCAGTG | SEQ ID NO.215 |
| | LET -B4-P2 | ROX-5′-CTCGATGGAGTGGGTCCCATCAG-3′- BHQ2 | SEQ ID NO.216 |
| | LET-B4-Block2 | TTTTGGTCTAGCTACAGTGAAATCTCGATGG AGT-NH2 | SEQ ID NO.217 |
| | LET-B4-F14 | TCAGGCCAAAAGTGTGATCCAA | SEQ ID NO.218 |
| | LET -B4-R3 | GGTCTGAGGCTGTTCACTGACTTA | SEQ ID NO.219 |
| | LET -B4-P3 | HEX-5′-TCTGGCAGTTCTCCTCTCCTGCAC-3′- BHQ1 | SEQ ID NO.220 |
| | LET-B5-F1 | GCCAGCGTGGACAACCCCCACCAC | SEQ ID NO.221 |
| | LET-B5-F2 | CGTGGACAACCCCCAACA | SEQ ID NO.222 |
| | LET-B5-F3 | CGTGGACAACCCCCACTAC | SEQ ID NO.223 |
| | LET-B5-F4 | TGGACAACCCCCACCCCCAC | SEQ ID NO.224 |
| | LET-B5-F5 | CGTGGACAACCCGGACA | SEQ ID NO.225 |
| | LET-B5-F6 | GGACAACCCCCACGGCAACCC | SEQ ID NO.226 |
| | LET-B5-F7 | CAACCCCCACGTGCCACG | SEQ ID NO.227 |
| | LET-B5-F8 | GGCCAGCGTGGACAACACC | SEQ ID NO.228 |
| | LET-B5-F9 | GATGGCCAGCGTGGGTT | SEQ ID NO.229 |
| | LET-B5-F10 | GTGATGGCCAGCGTGGGGGTC | SEQ ID NO.230 |
| LET PCR 8-tube strips-geneti c mutat ion-5 | LET -B5-F11 | GCCTACGTGATGGCCAGTGTGGC | SEQ ID NO.231 |
| | LET -B5-F12 | CGTGATGGCCAGCGTGGAGCGTG | SEQ ID NO.232 |
| | LET -B5-F13 | CGTGATGGCCAGCGTGGCTGGT | SEQ ID NO.233 |
| | LET-B5-F14 | GACAACCCCCACGTGCACGT | SEQ ID NO.234 |
| | LET-B5-F15 | CAGCGTGGACAACCCCGTT | SEQ ID NO.235 |
| | LET -B5-R1 | GACATAGTCCAGGAGGCAGCCG | SEQ ID NO.236 |
| | LET-B 5-P1 | FAM-5'-CGCCTGCTGGGCATCTGC-3'-BHQ1 | SEQ ID NO.237 |
| | LET-B5-F16 | GATAGTGTATTAACCTTATGTGTGACATGTT C | SEQ ID NO.238 |
| | LET-B5-R2 | CAAGGCACTCTTGCCTACGCGACA | SEQ ID NO.239 |
| | LET-B5-P2 | ROX-5 '-CTGCTGAAAATGACTGAAT-3 '-MGB | SEQ ID NO.240 |
| | LET-B5-Block1 | CACTCTTGCCTACGCCACCAGCTCCAACTAC CA-NH2 | SEQ ID NO.241 |
| | LET-B5-F17 | CTCGGCCTCCCAAGGTGTT | SEQ ID NO.242 |
| | LET-B5-R3 | CCACACAGCAGCAAGTGATAGTT | SEQ ID NO.243 |
| | LET-B5-P3 | VIC-5′-TAGGTGCCTAGCCCATGGTGCCTA-3′- BHQ1 | SEQ ID NO.244 |
| LET PCR 8-tube strips-geneti c mutat ion-6 | LET -B6-F1 | GATAGTGTATTAACCTTATGTGTGACATGTT C | SEQ ID NO.245 |
| | LET-B6-R1 | CGTCAAGGCACTCTTGCCTACGCTACG | SEQ ID NO.246 |
| | LET -B6-R2 | TCGTCAAGGCACTCTTGCCTACGCGAA | SEQ ID NO.247 |
| | LET -B6-R3 | CGTCAAGGCACTCTTGCCTACGTCAG | SEQ ID NO.248 |
| | LET -B6-R4 | TATCGTCAAGGCACTCTTGCCTAAGCA | SEQ ID NO.249 |
| | LET -B6-P1 | ROX-5 '-CTCCAACTACCACAAGTT-3 '-MGB | SEQ ID NO.250 |
| | LET -B6-F2 | ATGGCTGTGGTTTGTGATGGTT | SEQ ID NO.251 |
| | LET -B6-R5 | ACCAGCCATCACGTAAGCCATCAC | SEQ ID NO.252 |
| | LET -B6-R6 | ACATATGGGGAGCCCACACACA | SEQ ID NO.253 |
| | LET -B6-R7 | GAGACATATGGGGAGCCCACACG | SEQ ID NO.254 |
| | LET -B6-R8 | GGAGACATATGGGGAGCCCACAA | SEQ ID NO.255 |
| | LET -B6-R9 | GAGACATATGGGGAGCCCACACAG | SEQ ID NO.256 |
| | LET-B6-R10 | GGAGACATATGGGGAGCCCCTC | SEQ ID NO.257 |
| | LET -B6-P2 | FAM-5′- CGTATGCTTCCTGGGGACAAGGGTACG-3′- BHQ1 | SEQ ID NO.258 |
| | LET -B6-P3 | FAM-5'-CTCTCAGCGTACCCTTG-3'-MGB | SEQ ID NO.259 |
| | LET -B6-F3 | CTCGGCCTCCCAAGGTGTT | SEQ ID NO.260 |
| | LET-B6-R11 | CCACACAGCAGCAAGTGATAGTT | SEQ ID NO.261 |
| | LET -B6-P4 | VIC-5′-TAGGTGCCTAGCCCATGGTGCCTA-3′- BHQ1 | SEQ ID NO.262 |
| LET PCR 8-tube strips-geneti c | LET-B7-F1 | TGAATATAAACTTGTGGTAGTTGGAGCCGA | SEQ ID NO.263 |
| | LET-B7-F2 | GAATATAAACTTGTGGTAGTTGGAGCGA | SEQ ID NO.264 |
| | LET-B7-R1 | CAAGATTTACCTCTATTGTTGGATCATATTC | SEQ ID NO.265 |
| | LET-B7-P1 | ROX-5'-CAGCTAATTCAGAATCAT-3'-MGB | SEQ ID NO.266 |
| | LET-B7-Block1 | GACTGAATATAAACTTGTGGTAGTTGGAGCT GG-NH2 | SEQ ID NO.267 |
| | LET-B7-F3 | CTCGGCCTCCCAAGGTGTT | SEQ ID NO.268 |
| | LET-B7-R2 | CCACACAGCAGCAAGTGATAGTT | SEQ ID NO.269 |
| mutat ion-7 | LET-B7-P2 | VIC-5′-TAGGTGCCTAGCCCATGGTGCCTA-3′- BHQ1 | SEQ ID NO.270 |
| | LET-B7-F4 | TGGTGTGGGCTCCCCTGGCTC | SEQ ID NO.271 |
| | LET-B7-R3 | TCTAAGAGGCAGCCATAGGGCATA | SEQ ID NO.272 |
| | LET-B7-P3 | FAM-5'-CCCATATGTCTCCCGCCTTCTGGGC-3'-BHQ1 | SEQ ID NO.273 |
| LET PCR 8-tube strips-geneti c mutat ion-8 | LET B8-F1 | CAACCCTGCCCTGTGCAA | SEQ ID NO.274 |
| | LET B8-R1 | GGTGGTTCTGGAAGTCCAT | SEQ ID NO.275 |
| | LET B8-P1 | FAM-5′-AGCATCCAGTGGCGGACATAGTC- 3′-BHQ1 | SEQ ID NO.276 |
| | LET B8-P2 | ROX-5′-AGCATCCAGTGGCGGACATAGTC- 3′-BHQ2 | SEQ ID NO.277 |
| | LET B8-F2 | AGCTCTCTTGAGGATCTTGAAGGAA | SEQ ID NO.278 |
| | LET B8-R2 | CCTGTGCCAGGGACCTTACCT | SEQ ID NO.279 |
| | LET B8-P3 | FAM-5'-CTCCGGTGCGTTCGGCACG-3 '-BHQ1 | SEQ ID NO.280 |
| | LET B8-P4 | ROX-5'-CTCCGGTGCGTTCGGCACG-3'-BHQ2 | SEQ ID NO.281 |

Further, the present disclosure provides a reaction system of the primers and the probes, it comprises:
(1) a reverse transcription reaction system: 5-25µL of purified water, 5-15µL of 5×reverse transcription buffer, 20-150µmol of each primer, and 100-200U of reverse transcriptase, and a total volume is 20-30µL;
(2) a PCR reaction system: 15-45µL of purified water, 5-15µL of 10×PCR buffer, 1-10mmol of MgCl₂, 1-20pmol of each pair of primers, 1-20pmol of each pair of probes, 10-100pmol of dNTPs, and 1-10U of Taq enzyme, and a total volume is 30-50µL.

Another objective of the present disclosure is to provide a reagent kit, the reagent kit comprises sequences of SEQ ID NOs: 1-281. Specifically, the reagent kit comprises two kinds of PCR 8-tube strips, and the two kinds of PCR 8-tube stripes are respectively used to test the lung cancer RNA genetic fusion and the lung cancer DNA genetic mutation. The reagent kit comprises specific primers and specific probes of EGFR, KRAS, BRAF, HER2, ALK, ROS1, RET, NTRK1, NTRK2, NTRK3, MET and other genes and further comprises PCR buffer, UNG enzyme, etc.

Further, test reagents for testing the lung cancer RNA genetic fusion in the PCR 8-tube strips comprises: 1 tube of a test reagent of each of ALK, NTRK1, NTRK2 and NTRK3 fusion genes, 2 tubes of a test reagent of ROS1 fusion gene, and 1 tube of a test reagent of MET exon 14 skipping genetic mutation, and 1 tube of a test reagent of RET fusion gene. Tubes 1-4 in the 8-tube strips consist of test reagents of the ALK, NTRK1, NTRK2 and NTRK3 fusion genes, tubes 5 and 6 consist of test reagents of the ROS1 fusion gene, and a tube 7 consists of a test reagent of the MET exon 14 skipping mutation, and a tube 8 consists of a test reagent of the RET fusion gene test reagent and an internal control. The test reagents for the fusion gene in the tubes 1-8 are indicated by FAM signals, and the test reagent of the internal control in the tubes 1-8 is used to monitor a quality and an addition of a sample RNA and is indicated by VIC signal.

The test reagents for testing the lung cancer DNA genetic mutation in the PCR 8-tube strips comprises: 3 tubes of a test reagent of EGFR mutation gene, 1 tube of a test reagent of an EGFR/BRAF mutation gene, 1 tube of a test reagent of an EGFR/KRAS mutation gene, 2 tubes of a test reagent of an HER2/KRAS mutation gene, and 1 tube of a test reagent of an external control. An FAM signal of tubes 1-5 and a ROX signal of tubes 1-3 in the 8-tube strips respectively indicate different mutation sites of the EGFR gene, a ROX signal of a tube 4 indicates a V600E mutation of the BRAF gene, a FAM signal of tubes 6 and 7 indicates different mutation sites of the HER2 gene, a ROX signal of tubes 5-7 indicates different mutation sites of the KRAS gene, the test reagent of the internal control of tubes 1-7 is used to test an addition of the samples and is indicated by a VIC signal, and a tube 8 consists of test reagents for amplifying conserved segments of human genome to monitor a quality of a sample DNA and to be indicated by FAM and ROX signals.

The two PCR 8-tube strips are needed to test one sample. Compositions of a reagent kit is shown in Tables 2, 3 and 4.

**Table 2 The compositions of the reagent kit**

| Components of the reagent kit | Main components | numbers | Volume |
|---|---|---|---|
| LET PCR 8-tube strips for genetic fusion | primers, probes, magnesium ions, dNTPs | PCR 8-tube strips, 12 strips | 3 5 µL/tube |
| LET PCR 8-tube strips for genetic mutation | primers, probes, magnesium ions, dNTPs | PCR 8-tube strips, 12 strips | 3 5 µL/tube |
| LET RT reaction solution I | primers, magnesium ions, dNTPs | 1 tube | 220 µL |
| LET RT reaction solution II | primers, magnesium ions, dNTPs | 1 tube | 220 µL |
| LET reverse transcriptase | Reverse transcriptase | 1 tube | 16 µL |
| LET mixed enzyme A | Hot-start enzyme, UNG enzyme | 1 tube | 45µL |
| LET mixed enzyme B | Hot-start enzyme, UNG enzyme | 1 tube | 45 µL |
| LET positive control | plasmid DNA | 1 tube | 500 µL |

**Table 3 Compositions of LET PCR 8-tube strips for the genetic fusion**

| Tube numbers | Test reagents | Test types | Volumes | Fluorescence signal |
|---|---|---|---|---|
| 1 | LET reaction solution A1 | ALK fusion | 35 µL | FAM/VIC |
| 2 | LET reaction solution A2 | NTRK1 fusion | 35 µL | FAM/VIC |
| 3 | LET reaction solution A3 | NTRK2 fusion | 35 µL | FAM/VIC |
| 4 | LET reaction solution A4 | NTRK3 fusion | 35 µL | FAM/VIC |
| 5 | LET reaction solution A5 | ROS1 fusion | 35 µL | FAM/VIC |
| 6 | LET reaction solution A6 | ROS1 fusion | 35 µL | FAM/VIC |
| 7 | LET reaction solution A7 | MET 14 exon skipping | 35 µL | FAM/VIC |
| 8 | LET reaction solution A8 | RET fusion | 35 µL | FAM/VIC |

**Table 4 Compositions of LET PCR 8-tube strips for the genetic mutation**

| Tube numbers | Test reagents | Test types | Volumes | Fluorescence signal |
|---|---|---|---|---|
| 1 | LET reaction solution B1 | EGFR mutation | 35µL | FAM/VIC/ROX |
| 2 | LET reaction solution B2 | EGFR mutation | 35µL | FAM/VIC/ROX |
| 3 | LET reaction solution B3 | EGFR mutation | 35µL | FAM/VIC/ROX |
| 4 | LET reaction solution B4 | EGFR/BRAF mutation | 35µL | FAM/VIC/ROX |
| 5 | LET reaction solution B5 | EGFR/KRAS mutation | 35µL | FAM/VIC/ROX |
| 6 | LET reaction solution B6 | HER2/KRAS mutation | 35µL | FAM/VIC/ROX |
| 7 | LET reaction solution B7 | HER2/KRAS mutation | 35µL | FAM/VIC/ROX |
| 8 | LET reaction solution B8 | external control | 35µL | FAM/ROX |

**Table 5 Test sites of LET PCR 8-tube strips for genetic fusion**

| Tube numbers/signal indication | Test genes | Fusion types | Fusion names |
|---|---|---|---|
| 1/FAM | ALK | EML4exon13; ALK exon20 | EML4-ALK-1 |
| | | EML4 exon6 ins33; ALK exon20 | EML4-ALK-2 |
| | | EML4 exon20; ALK exon20 | EML4-ALK-3 |
| | | EML4exon18; ALK exon20 | EML4-ALK-6 |
| | | EML4 exon2; ALK exon20 | EML4-ALK-7 |
| | | EML4exon17; ins68 ALK exon20 | EML4-ALK-8 |
| | | EML4 exon2; ins117 ALK exon20 | EML4-ALK-9 |
| | | EML4 exon13; ins69 ALK exon20 | EML4-ALK-10 |
| | | EML4 exon6; ALK exon20 | EML4-ALK-11 |
| | | EML4 exon6; ALK exon19 | EML4-ALK-12 |
| | | EML4 exon6; ins18 ALK exon20 | EML4-ALK-13 |
| | | EML4 exon20; ins18 ALK exon20 | EML4-ALK-14 |
| | | EML4 exon17de158; ins39 ALK exon20 | EML4-ALK-17 |
| | | EML4 exon17 ins65; ALK exon20 | EML4-ALK-18 |
| | | EML4 exon17; ins30 ALK exon20 | EML4-ALK-19 |
| | | EML4 exon17 ins61; ins34 ALK exon20 | EML4-ALK-20 |
| | | EML4 exon3; ins53 ALK exon20 | EML4-ALK-21 |
| | | KIFSB exon24; ALK exon20 | KIF5B-ALK-1 |
| | | KIFSB exon17; ALK exon20 | KIF5B-ALK-2 |
| | | KLC1 exon9; ALK exon20 | KLC1-ALK |
| | | TFG exon4; ALK exon20 | TFG-ALK |
| | | STRN exon3; ALK exon20 | STRN-ALK-1 |
| | | HIP1 exon21; ALK exon20 | HIP 1 -ALK- 1 |
| | | HIP1 exon28; ALK exon20 | HIP1-ALK-2 |
| | | HIP1 exon30; ALK exon20 | HIP1-ALK-3 |
| | | PRKAR1 A exon5; ALK exon20 | PRKAR1A-ALK-1 |
| | | PRKAR1A exon 10; ALK exon20 | PRKAR1A-ALK-2 |
| | | NBAS exon35; ALK exon20 | NBAS-ALK-1 |
| | | TFG exon6; ALK exon20 | TFG-ALK-2 |
| | | PPM1B exon1; ALK exon20 | PPM1B-ALK-1 |
| | | EIF2AK3 exon2; ALK exon20 | EIF2AK3-ALK-1 |
| | | BCL11A exon4 del5059; ins14 ALK exon20 | BCL11A-ALK-1 |
| | | BIRC6 exon10; ALK exon20 | BIRC6-ALK-1 |
| | | CEBPZ exon2; ALK exon20 | CEBPZ-ALK-1 |
| | | CLIP1 exon22; ALK exon20 | CLIP 1-ALK-1 |
| | | COL25A1 exon3; ALK exon20 | COL25A1-ALK-1 |
| | | GCC2 exon13; ALK exon20 | GCC2-ALK-1 |
| | | GCC2 exon19; ALK exon20 | GCC2-ALK-2 |
| | | LMO7 exon15; ALK exon20 | LMO7-ALK-1 |
| | | PICALM exon19; ALK exon20 | PICALM-ALK-1 |
| | | PHACTR1 exon6; ALK exon20 | PHACTR1-ALK-1 |
| | | TPRexon15; ALK exon20 | TPR-ALK-1 |
| | | MPRIP exon21; ALK exon20 | MPRIP-ALK-1 |
| | | TNIP2 exon5; ALK exon20 | TNIP2-ALK-1 |
| | | DCTN1 exon26; ALK exon20 | DCTN1-ALK-1 |
| | | SQSTM1 exon5; ALK exon20 | SQSTM1-ALK-1 |
| 2/FAM | NTRK1 | TFG exon5; NTRK1 exon9 | NTRK1-E9-M1 |
| | | TPM3 exon8; NTRK1 exon10 | NTRK1-E10-M1 |
| | | SQSTM1 exon5; NTRK1 exon10 | NTRK1-E10-M3 |
| | | TPRexon16 de154; NTRK1 ins13 exon10 | NTRK1-E10-M5 |
| | | TPR exon21; NTRK1 exon10 | NTRK1-E10-M6 |
| | | CD74 exon8; NTRK1 exon10 | NTRK1-E10-M7 |
| | | IRF2BP2 exon1; NTRK1 exon10 | NTRK1-E10-M8 |
| | | IRF2BP2 exon1 del48; NTRK1 exon10 | NTRK1-E10-M9 |
| | | TFG exon5; NTRK1 exon10 | NTRK1-E10-M12 |
| | | GRIPAP1 exon22; NTRK1 exon10 | NTRK1-E10-M14 |
| | | F11Rexon4; NTRK1 exon10 | NTRK1-E10-M15 |
| | | SQSTM1 exon6; NTRK1 exon10 | NTRK1-E10-M17 |
| | | TPM3 exon8; NTRK1 exon12 | NTRK1-E12-M1 |
| | | MPRIP exon21; NTRK1 exon12 | NTRK1-E12-M3 |
| | | SSBP2 exon12; NTRK1 exon12 | NTRK1-E12-M4 |
| | | MPRIP exon14; NTRK1 exon12 | NTRK1-E12-M11 |
| | | MPRIP exon18; NTRK1 exon12 | NTRK1-E12-M12 |
| | | GRIPAP1 exon22; NTRK1 exon12 | NTRK1-E12-M14 |
| 3/FAM | NTRK2 | TRIM24 exon12; NTRK2 exon15 | NTRK2-E15-M1 |
| | | TRIM24 exon12; NTRK2 exon16 | NTRK2-E16-M1 |
| | | SQSTM1 exon5; NTRK2 exon16 | NTRK2-E16-M3 |
| | | STRN exon3; NTRK2 exon16 | NTRK2-E16-M7 |
| | | SQSTM1 exon5; NTRK2 exon17 | NTRK2-E17-M2 |
| 4/FAM | NTRK3 | ETV6 exon4; NTRK3 exon14 | NTRK3-EX14-M1 |
| | | ETV6 exon5; NTRK3 exon14 | NTRK3-EX14-M2 |
| | | EML4 exon2; NTRK3 exon14 | NTRK3-EX14-M3 |
| | | SQSTM1 exon5; NTRK3 exon14 | NTRK3-EX14-M4 |
| | | RBPMS exon5; NTRK3 exon14 | NTRK3-EX14-M7 |
| | | ETV6 exon5; NTRK3 exon15 | NTRK3-EX15-M1 |
| | | ETV6 exon4; NTRK3 exon15 | NTRK3-EX15-M2 |
| | | SQSTM1 exon6; NTRK3 exon15 | NTRK3-EX15-M3 |
| 5/FAM | ROS1 | SLC34A2 exon4; ROS1 exon32 | ROS1-M1 |
| | | SLC34A2 exon13 del2046; ROS1 exon32 | ROS1-M2 |
| | | CD74 exon6; ROS1 exon32 | ROS1-M3 |
| | | SDC4 exon2; ROS1 exon32 | ROS1-M4 |
| | | SDC4 exon4; ROS1 exon32 | ROS1-M5 |
| | | SLC34A2 exon4; ROS1 exon34 | ROS1-M6 |
| | | SLC34A2 exon13 del2046; ROS1 exon34 | ROS1-M7 |
| | | CD74 exon6; ROS1 exon34 | ROS1-M8 |
| | | SDC4 exon4; ROS1 exon34 | ROS1-M9 |
| | | EZR exon10; ROS1 exon34 | ROS1-M10 |
| 6/FAM | ROS1 | TPM3 exon8; ROS1 exon35 | ROS1-M11 |
| | | LRIG3 exon16; ROS1 exon35 | ROS1-M12 |
| | | GOPC exon8; ROS1 exon35 | ROS1-M13 |
| | | CCDC6 exon5; ROS1 exon35 | ROS1-EX35-M1 |
| | | CD74 exon6; ROS1 exon35 | ROS1-EX35-M2 |
| | | CLTC exon31; ROS1 exon35 | ROS1-EX35-M3 |
| | | EZR exon10; ROS1 exon3 5 | ROS1-EX35-M4 |
| | | KDELR2 exon5; ROS1 exon35 | ROS1-EX35-M6 |
| | | MYO5A exon23; ROS1 exon35 | ROS1-EX35-M8 |
| | | PPF1BP1 exon9; ROS1 exon35 | ROS1-EX35-M9 |
| | | TMEM106B exon3; ROS1 exon35 | ROS1-EX35-M10 |
| | | TPM3 exon7; ROS1 exon35 | ROS1-EX35-M11 |
| | | TFG exon4; ROS1 exon35 | ROS1-EX35-M13 |
| 7/FAM | MET | MET exon 14 skipping mutation | MET-M1 |
| 8/FAM | RET | CCDC6 exon1; RET exon12 | RET-M2 |
| | | NCOA4 exon6; RET exon12 | RET-M5 |
| | | KIF5B exon15; RET exon12 | RET-M15 |
| | | KIFSB exon16; RET exon12 | RET-M16 |
| | | KIFSB exon23; RET exon12 | RET-M17 |
| | | KIFSB exon22; RET exon12 | RET-M19 |
| | | TRIM33 exon14; RET exon12 | LRET-M22 |
| | | CUX1 exon10; RET exon12 | LRET-M32 |
| | | KIAA1468 exon10; RET exon12 | LRET-M40 |
| | | KIF13A exon18; RET exon12 | LRET-M41 |
| | | MPRIP exon19; RET exon12 | LRET-M42 |
| | | MYO5C exon25; RET exon12 | LRET-M44 |
| | | PICALM exon19; RET exon12 | LRET-M45 |
| | | RUFY2 exon9; RET exon12 | LRET-M49 |
| | | TNIP2 exon5; RET exon12 | LRET-M55 |
| | | WAC exon3; RET exon12 | LRET-M57 |
| | | KIF5Bexon18; RET exon12 | LRET-M58 |
| | | TRIM33 exon11; RET exon12 | LRET-M59 |

**Table 6 Test sites of the LET PCR 8-tube strips for the genetic mutation**

| Tube number/ signal indicati on | test section | Mutation types | Base change | cosmic ID | mutation name |
|---|---|---|---|---|---|
| 1/FAM | EGFR Exon 19 | E746_A750del (1) | 2235_2249del15 | 6223 | E-19-M1 |
| | | E746_A750del (2) | 2236_2250del15 | 6225 | E-19-M2 |
| | | L747_P753>S | 2240_2257del18 | 12370 | E-19-M3 |
| | | E746_T751>I | 2235_2252>AAT(complex) | 13551 | E-19-M4 |
| | | E746_T751del | 2236_2253del18 | 12728 | E-19-M5 |
| | | E746_T751>A | 2237_2251del15 | 12678 | E-19-M6 |
| | | E746_S752>A | 2237_2254del18 | 12367 | E-19-M7 |
| | | E746_S752>V | 2237_2255>T(complex) | 12384 | E-19-M8 |
| | | E746_S752>D | 2238_2255del18 | 6220 | E-19-M9 |
| | | L747_A750>P | 2238_2248>GC(complex) | 12422 | E-19-M10 |
| | | L747_T751>Q | 2238_2252>GCA(complex) | 12419 | E-19-M11 |
| | | L747_E749del | 2239_2247del9 | 6218 | E-19-M12 |
| | | L747_T751del | 2239_2253del15 | 6254 | E-19-M13 |
| | | L747_S752del | 2239_2256del18 | 6255 | E-19-M14 |
| | | L747_A750>P | 2239_2248TTAAGAGAAG>C (complex) | 12382 | E-19-M15 |
| | | L747_P753>Q | 2239_2258>CA(complex) | 12387 | E-19-M16 |
| | | L747_T751>S | 2240_2251del12 | 6210 | E-19-M17 |
| | | L747_T751del | 2240 2254del15 | 12369 | E-19-M18 |
| | | L747_T751>P | 2239_2251>C(complex) | 12383 | E-19-M19 |
| | | L747_T751del | 2238_2252del15 | 23571 | E-19-M20 |
| | | L747_S752>Q | 2239_2256>CAA(Complex) | 12403 | E-19-M21 |
| | | L747_A750>P | 2239_2250>CCC(Complex) | / | E-19-M24 |
| | | L747_K754>QL | 2239_2261>CAATT(Complex) | / | E-19-M25 |
| | | E746 K754>EQH L | 2238_2261>GCAACATCT(Co mplex) | / | E-19-M26 |
| | | L747_S752>Q | 2238_2256>GCAA(Complex) | 26441 | E-19-M27 |
| 1/ROX | EGFR Exon 20 | S768I | 2303G>T | 6241 | E-20-M2 |
| 2/FAM | EGFR Exon 21 | L858R | 2573T>G | 6224 | E-21-M1 |
| 2/ROX | EGFR Exon 18 | G719A | 2156G>C | 6239 | E-18-M1 |
| | | G719S | 2155G>A | 6252 | E-18-M2 |
| | | G719C | 2155G>T | 6253 | E-18-M3 |
| 3/FAM | EGFR Exon 20 | T790M | 2369C>T | 6240 | E-20-M1 |
| 3/ROX | EGFR Exon 21 | L861Q | 2582T>A | 6213 | E-21-M2 |
| 4/FAM | EGFR Exon 20 | D770_N771insG | 2310_2311insGGT | 12378 | E-20-M4 |
| | | V769 D770insAS V | 2307_2308insGCCAGCGTG | 12376 | E-20-M5 |
| | | D770_N771insSV D | 2311_2312insGCGTGGACA | 13428 | E-20-M8 |
| | | D770_N771insSV D | 2309_2310AC>CCAGCGTGG AT13558E-20-M9 | | |
| | | V769_D770insMA SV2307_2308insATGGCCAGCG TG | | / | E-20-M13 |
| | | D770_N771insGF | 2310_2311insGGGTTT | 655155 | E-20-M14 |
| | | N771>GY | 2311A>GGTT | 53189 | E-20-M20 |
| | | V769 D770insGS V | 2308 2309insGCAGCGTGG | 18429 | E-20-M21 |
| | | D770_N771insG | 2310_2311insGGG | / | E-20-M23 |
| | | D770_N771insG | 2310_2311insGGC | 13004 | E-20-M24 |
| | | V769 D770insAS V | 2303 2304insTGTGGCCAG | 20884 | E-20-M28 |
| | | D770_N771insY | 2310_2311insTAC | 123803 0 | E-20-M29 |
| | | D770_N771insT | 2311_2312insCCA | 502300 8 | E-20-M30 |
| | | N771_P772insN | 2313_2314insAAC | 20887 | E-20-M31 |
| | | D770_N771insGD | 2310_2311insGGGGAC | 85795 | E-20-M36 |
| | | D770_N771insGL | 2310_2311insGGGTTA | 48921 | E-20-M37 |
| | | N771>GF | 2311_2312AA>GGGTT | 18431 | E-20-M38 |
| | | V769 D770insMA SVD | 2307 2308insATGGCCAGCG TGGAC | 28638 | E-20-M39 |
| | | N771_P772>SVD NR | 2312_2315ACCC>GCGTGGA CAACCG | 13554 | E-20-M40 |
| | | D770_N771insGT | 2310_2311insGGCACA | 123802 9 | E-20-M41 |
| | | N771>KL | 2312_2313insACT | 643814 7 | E-20-M44 |
| | | D770_N771insGD | 2308_2309insACGGCG | 22955 | E-20-M48 |
| | | D770_N771insAP W | 2310_2311insGCACCGTGG | 20886 | E-20-M50 |
| | | D770_N771insSV E | 2311_2312insGCGTCGAAA | 165174 3 | E-20-M51 |
| | | N771P772insVD N | 2307_2308insGACAACGTG | 20885 | E-20-M52 |
| | | N771>SH | 2311_2312insGTC | 24434 | E-20-M53 |
| 4/ROX | BRAF Exon 15 | V600E | 1799T>A | 476 | BRAF-M1 |
| 5/FAM | | H773_V774insH | 2319_2320insCAC | 12377 | E-20-M3 |
| | EGFR Exon 20 | H773_V774insNP H | 2319_2320insAACCCCCAC | 12381 | E-20-M10 |
| | | H773_V774insQ | 2319_2320insCAG | 131552 | E-20-M12 |
| | | N771_P772insT | 2313_2314insACC | / | E-20-M15 |
| | | N771P772insH | 2314 2315insACC | 123803 1 | E-20-M16 |
| | | P772_H773insQ | 2318_2319insACA | / | E-20-M17 |
| | | H773_V774insY | 2319_2320insTAC | / | E-20-M18 |
| | | H773_V774insPH | 2319_2320insCCCCAC | 12380 | E-20-M19 |
| | | N771_P772insHH | 2311_2312insACCACC | 693120 7 | E-20-M22 |
| | | P772_H773insDNP | 2315_2316insGGACAACCC | 684509 9 | E-20-M25 |
| | | P772_H773insDNP | 2307 2308insGACAACCCC | 696205 0 | E-20-M26 |
| | | D770_N771insNP G | 2310_2311insAACCCCGGC | / | E-20-M27 |
| | | V774_C775insHV | 2321_2322insCCACGT | 18432 | E-20-M32 |
| | | V774_C775insHV | 2322_2323insCACGTG | 22948 | E-20-M33 |
| | | D770>GY | 2308_2309insGTT | 12427 | E-20-M34 |
| | | H773_V774insAH | 2320_2321insCCCACG | 123802 8 | E-20-M35 |
| | | P772_H773insV | 2316_2317insGTT | 255205 | E-20-M42 |
| | | V769 D770insGV V | 2308_2309insGGGTCGTGG | 18430 | E-20-M43 |
| | | H773 V774insGN PH2320_2321insGCAACCCCCA CG | | 643814 7 | E-20-M45 |
| | | V769_D770insAN V | 2303_2304insTGTGGCCAA | 165174 1 | E-20-M46 |
| | | V769_D770insER G | 2308_2309insAGCGTGGAG | 165174 2 | E-20-M47 |
| | | D770_N771>AGG | 2309_2312ACAA>CTGGTGG (Complex) | 12737 | E-20-M49 |
| | | P772_H773insTP | 2316C>AACCCCT | 12388 | E-20-M55 |
| | | H773>PNPY | 2317 2318insCTAACCCCT | 173576 1 | E-20-M56 |
| | | V774_C775insPR | 2322_2323insCCACGT | 417022 3 | E-20-M57 |
| 5/ROX | KRAS Exon 2 | G12C | 34G>T | 516 | KRAS-M6 |
| 6/FAM | HER2 Exon 20 | A775 G776insYV MA | 2325 2326 insTACGTGATGGCT | 12558 | HER2-M1 |
| | | A775 G776insYV MA | 2324 2325 insATACGTGATGGC | 20959 | HER2-M2 |
| | | G776>VC | 2326_2327insTGT | 12553 | HER2-M3 |
| | | G776>VC | 2326_2327insTTT | 12552 | HER2-M8 |
| | | G776R | 2326G>C | / | HER2-M9 |
| | | G776>VC | 2326 2327insTAT | / | HER2-M15 |
| | | G776>VC | 2326 2327insTCT | 85995 | HER2-M16 |
| | | G776V | 2327G>T | 18609 | HER2-M17 |
| | | G776>VV | 2327G>TTGT | 697743 3 | HER2-M18 |
| | | G776>LC | 2326G>TTAT | 20895 | HER2-M19 |
| | | G776>LC | 2326G>CTTT | 12554 | HER2-M20 |
| | | G776>LC | 2326G>TTGT | 19875 | HER2-M21 |
| | | G776>AVGC | 2326_2327insCTGTGGGCT | 690703 3 | HER2-M22 |
| | | G776_V777insL | 2327_2328insTCT | 643815 1 | HER2-M23 |
| | | V777 G778insCG | 2331_2332insTGTGGG | 303939 | HER2-M24 |
| | | G778_S779insG | 2330_2331insGGG | 690642 0 | HER2-M25 |
| | | S779_P780insVGS | 2335_2336insCTGTGGGCT | 681 | HER2-M26 |
| 6/ROX | KRAS Exon 2 | G12A | 35G>C | 522 | KRAS-M2 |
| | | G12V | 35G>T | 520 | KRAS-M3 |
| | | G12R | 34G>C | 518 | KRAS-M5 |
| | | G13C | 37G>T | 527 | KRAS-M14 |
| 7/FAM | HER2 Exon 20 | P780_Y781insGSP | 2339_2340insTGGCTCCCC | 303948 | HER2-M4 |
| | | P780_Y781insGSP | 2339_2340insGGGCTCCCC | 12555 | HER2-M6 |
| | | P780_Y781insGSP | 2340_2341insGGCTCCCCA | 12556 | HER2-M7 |
| | | P780_Y781insGSP | 2339_2340insCGGCTCCCC | 686589 3 | HER2-M10 |
| 7/ROX | KRAS Exon 2 | G12D | 35G>A | 521 | KRAS-M1 |
| | | G12S | 34G>A | 517 | KRAS-M4 |

Another objective of the present disclosure is to provide a method for using the reagent kit, its steps comprise:
(1) extracting DNA and RNA from test samples, the test samples comprise a fresh pathological tissue, a paraffin-embedded tissue or a paraffin section of a tissue;
(2) mixing the RNA with the reverse transcriptase, and adding a reverse transcription reaction solution separately to obtain cDNA;
(3) mixing the DNA and the cDNA with mixed enzyme separately, and adding into PCR 8-tube strips; and
(4) after amplifying by a fluorescent PCR amplification device, judging test results according to a displayed Ct value.

The present disclosure has the following advantages.
1. The present disclosure uses a technology of specific primers and probes and a test mode of a three-color fluorescence channel to establish a real-time PCR system, seven fusion genes comprising ALK, ROS1, RET, NTRK1, NTRK2, NTRK3, MET and four mutation genes comprising EGFR, KRAS, BRAF, HER2 can be simultaneously tested, there are a total of 119 genetic fusions and 112 genetic mutations, it specifically comprises 83 mutations of EGFR gene, 7 mutations of KRAS gene, 1 mutation of BRAF gene, 21 mutations of HER2 gene, 46 fusions of ALK gene, 23 fusions of ROS1 gene, 18 fusions of RET gene, 18 fusions of NTRK1 gene, 5 fusions of NTRK2 gene, 8 fusions of NTRK3 gene and 1 skipping mutation of MET gene (see Table 5 and Table 6 for details).
2. The present disclosure divides 281 sequences (including primers and probes) into groups to be packaged in two PCR 8-tube strips based on a test difficulty of multiple mutation/fusion sites, mutual influence and competition between primers, and other comprehensive factors. The advantages are as follows: (1) multiple mutation types are combined into one tube using double or triple PCR and distinguished using different fluorescently labeled probes to test multiple mutation types in the one tube, an operation is convenient for users, and test costs and test samples are saved at the same time; (2) When mutation types are combined into the one tube, not only a technical difficulty for combining into the one tube is considered, but needs from different drugs are also considered. For the same drug, corresponding targets of the drug are placed in the same reaction tube as much as possible or placed in the same fluorescence channel of different reaction tubes to facilitate result analyses; (3) The reagent kit can be used to test 231 mutation/fusion sites at one time, and operation methods/amplification procedures of all reaction tubes are basically the same, an operation is convenient, time is saved, and it is convenient for clinical application; (4) Each reaction system further comprises an internal control test system, and the internal control of the fusion test tube is used to control a quality of the RNA samples and whether a device and an operation is abnormal; the internal control of the mutation reaction tube is used to control a quality of the DNA samples and whether a device and an operation is abnormal, an external control is used to judge the quality of the DNA samples and participate result analyses. (5) A sensitivity is high, both 1% genetic mutation DNA and 125 copies of fusion gene plasmid DNA can be detected; (6) A test time is short, and only 3 hours are needed to complete a test process; (7) A pre-packaged design is hermetically sealed to improve a safety for a transportation process, and an operation is more convenient.
3. The test sites covered by the reagent kit provided by the present disclosure account for more than 95% of all mutation types of each gene. A total mutation rate in patients with non-small cell lung cancer is as high as 70%, it is a fluorescent PCR product available having the most mutation genes being tested and the mutation types being most integrated to provide a comprehensive reference for clinicians to choose a therapy of a tumor-targeted drug for patients with lung cancer.
4. Compared with the NGS method, the reagent kit provided by the present disclosure is convenient and fast, a sensitivity is high, a specificity is good, result analyses is objective, etc., making it the most popular first-line test method in clinical practice.

### BRIEF DESCRIPTION OF THE DRAWINGS

FIG. 1 is a sensitivity image of an ALK fusion gene, that is, an EML4 exon13 and an ALK exon20 in Embodiment 1.
FIG. 2 is a sensitivity image of a ROS1 fusion gene, that is, a CD74 exon6 and a ROS1 exon32 in Embodiment 1.
FIG. 3 is a sensitivity image of a RET fusion gene, that is, a KIF5B exon16 and a ROS1 exon12 in Embodiment 1.
FIG. 4 is a sensitivity image of an NTRK1 fusion gene, that is, a CD74 exon8 and an NTRK1 exon10 in Embodiment 1.
FIG. 5 is a sensitivity image of an NTRK2 fusion gene, that is, TRIM24 exon12 and an NTRK2 exon15 in Embodiment 1.
FIG. 6 is a sensitivity image of an NTRK3 fusion gene, that is, an ETV6 exon5 and an NTRK3 exon15 in Embodiment 1.
FIG. 7 is a sensitivity image of a MET skipping mutation, that is, a MET exon14 skipping in Embodiment 1.
FIG. 8 is a sensitivity image of a 19del in of a EGFR genetic mutation in Embodiment 1.
FIG. 9 is a sensitivity image of an L858R of the EGFR genetic mutation in Embodiment 1.
FIG. 10 is a sensitivity image of a T790M of the EGFR genetic mutation in Embodiment 1.
FIG. 11 is a sensitivity image of a G12D of a KRAS genetic mutation in Embodiment 1.
FIG. 12 is a sensitivity image of a G12C of the KRAS genetic mutation in Embodiment 1.
FIG. 13 is a sensitivity image of a V600E of a BRAF genetic mutation in Embodiment 1.
FIG. 14 is a sensitivity image of a V769_D770insASV of a HER2 genetic mutation in Embodiment 1.
FIG. 15 is a sensitivity image of a A775_G776insYVMA of the HER2 genetic mutation in Embodiment 1.
FIG.16 is a PCR image of a test result of the ALK fusion gene of a positive clinical sample in Embodiment 2.
FIG. 17 is a PCR image of a test result of the ROS1 fusion gene of the positive clinical sample in Embodiment 2.
FIG. 18 is a PCR image of a test result of the RET fusion gene of the positive clinical sample in Embodiment 2.
FIG. 19 is a PCR image of a test result of the NTRK3 fusion gene of the positive clinical sample in Embodiment 2.
FIG. 20 is a PCR image of a test result of the MET skipping mutation of the positive clinical sample in Embodiment 2.
FIG. 21 is a PCR image of a test result of the 19del of the EGFR genetic mutation of the positive clinical sample in Embodiment 2.
FIG. 22 is a PCR image of a test result of the L858R of the EGFR genetic mutation of the positive clinical sample in Embodiment 2.
FIG. 23 is a PCR image of a test result of the T790M of the EGFR genetic mutation of the positive clinical sample in Embodiment 2.
FIG. 24 is a PCR image of a test result of the KRAS genetic mutation of the positive clinical sample in Embodiment 2.
FIG. 25 is a PCR image of a test result of the G12C of the KRAS genetic mutation of the positive clinical sample in Embodiment 2.
FIG. 26 is a PCR image of a test result of the BRAF genetic mutation of the positive clinical sample in Embodiment 2.
FIG. 27 is a PCR image of a test result of the HER2 genetic mutation of the positive clinical sample in Embodiment 2.

In the aforementioned drawings, abscissas of all drawing are all cycles, and ordinates are all Fluorescence (dR).

### DETAILED DESCRIPTION OF THE EMBODIMENTS

The present disclosure will be further described in combination with the accompanying embodiments. The embodiments are merely used to explain the present disclosure, rather than used to be understood as limitations of the present disclosure. Unspecified techniques and conditions in the embodiments should be performed according to the techniques and the conditions described in the articles in this art or product instructions. Devices and reagents with unspecified makers are all commercial conventional products.

### Embodiment 1

Specific primers and probes are designed according to wild-type gene sequences, that is, human EGFR, BRAF, HER2, KRAS, ALK, ROS1, RET, NTRK1, NTRK2, NTRK3 and MET, published in Cosmic data, and based on driver mutation sites and fusion sites, that is, EGFR, BRAF, HER2, KRAS, ALK, ROS1, RET, NTRK1, NTRK2, NTRK3 and MET (see Tables 1, 7 and 8 for details).

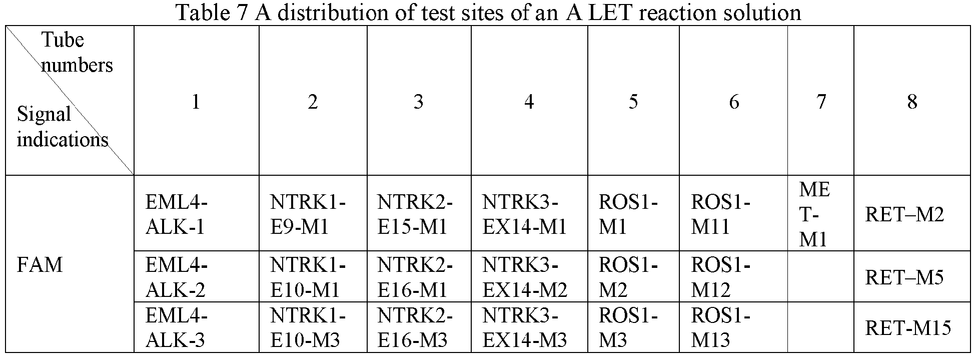

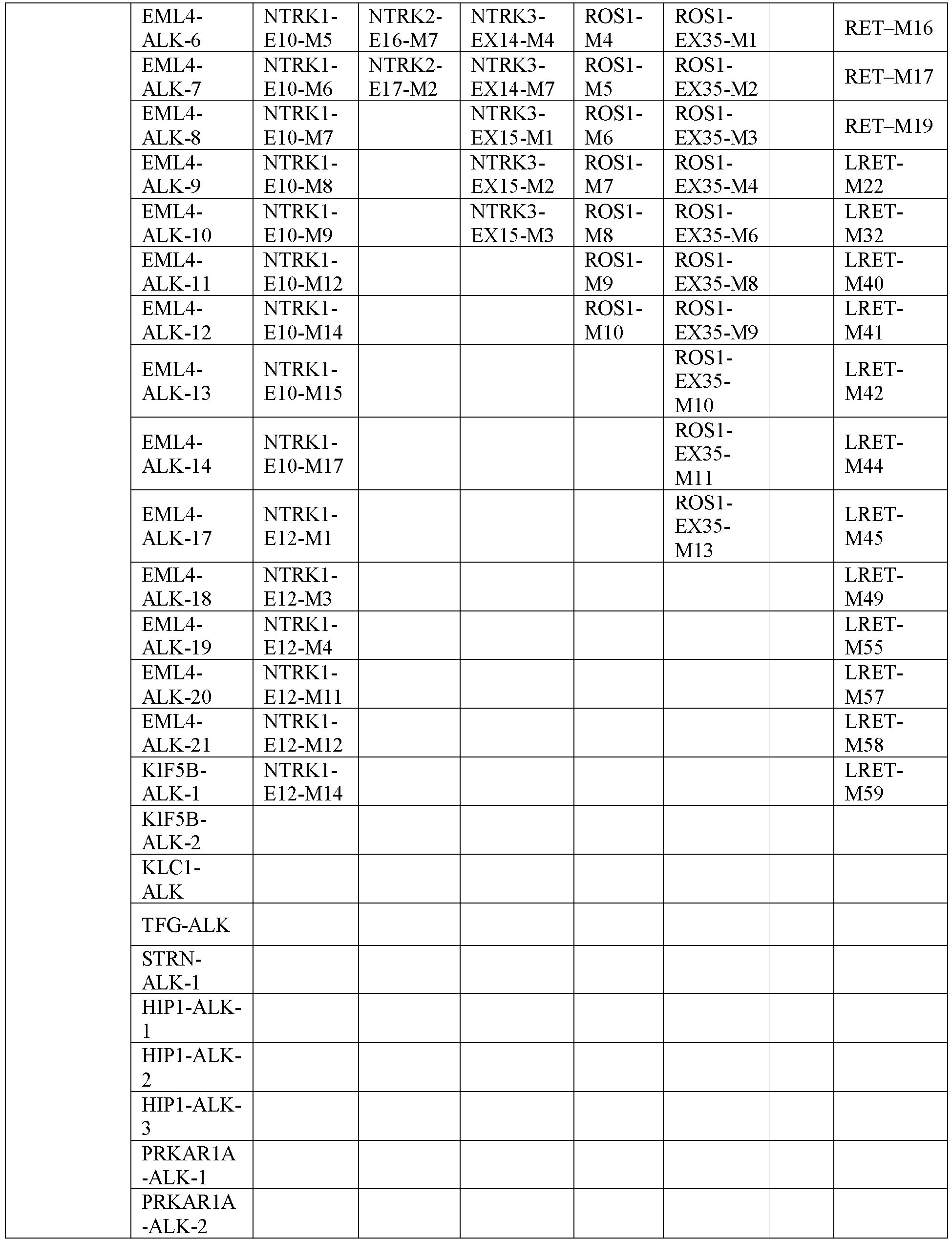

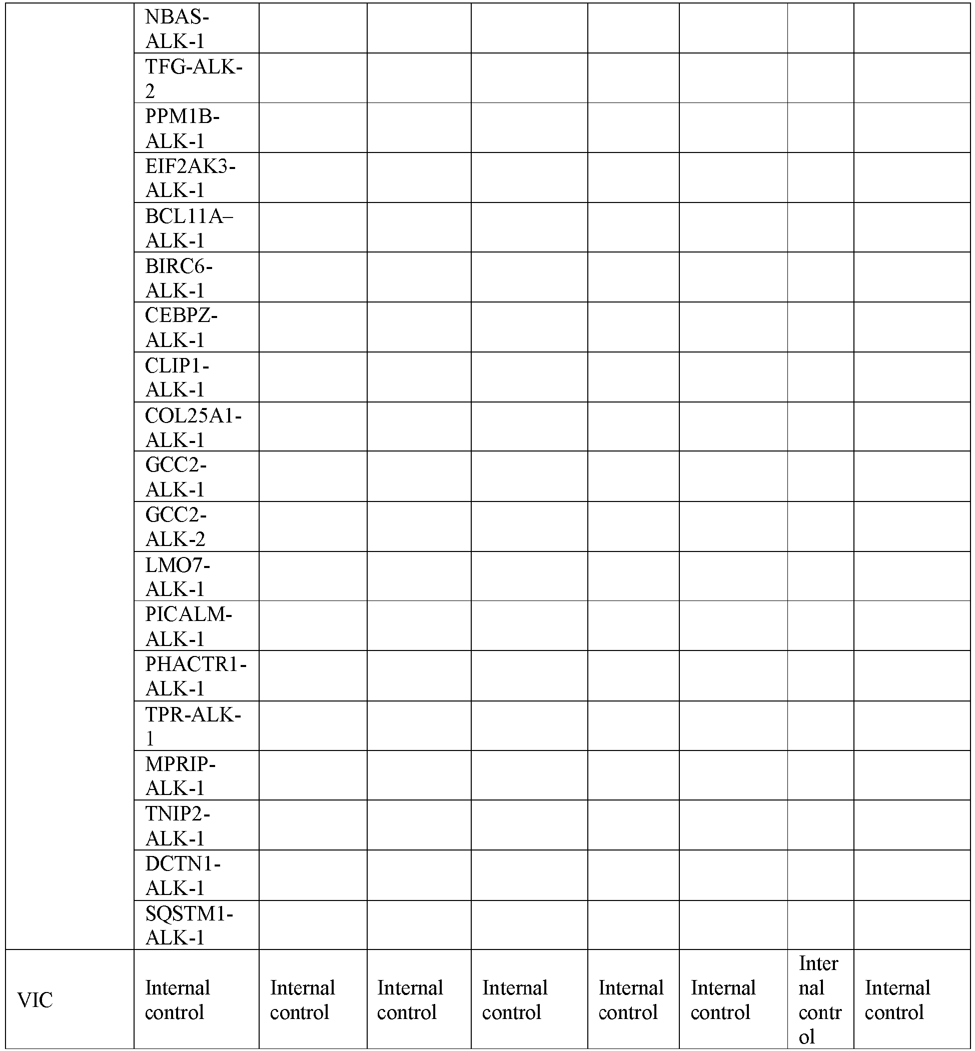

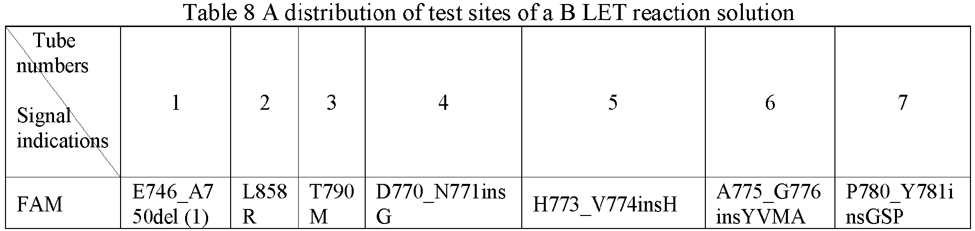

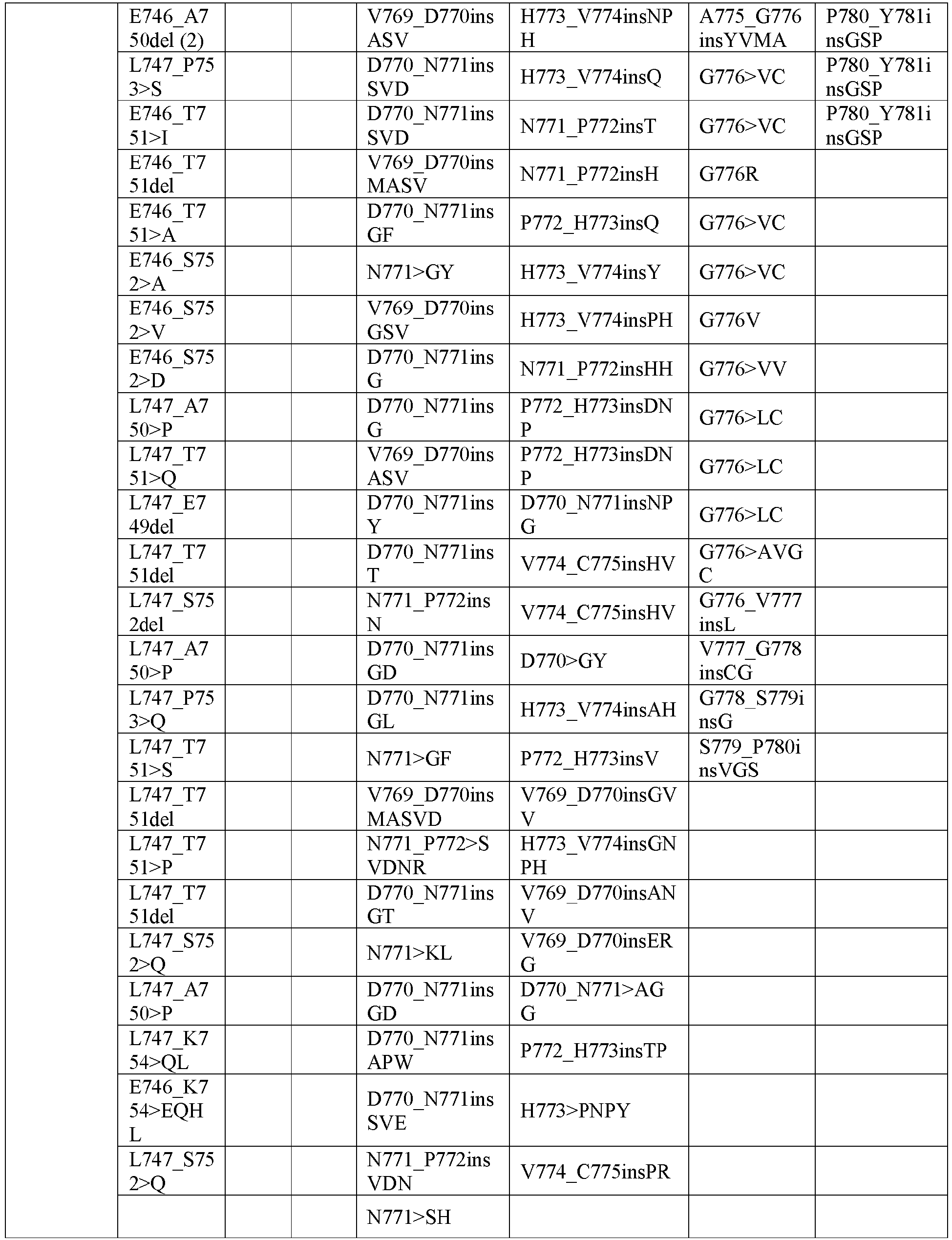

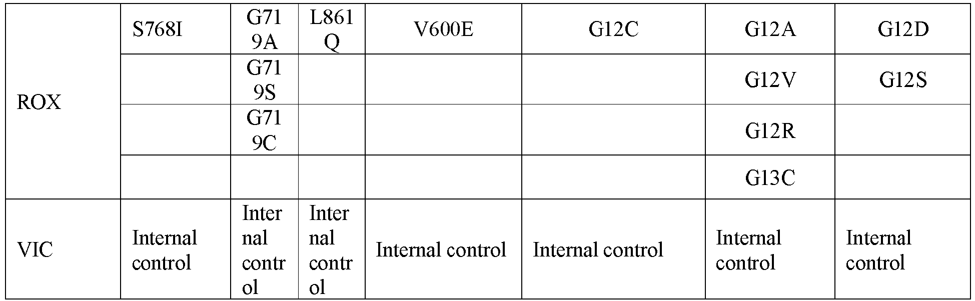

In this embodiment, by way of example, EML4 exon 13 and ALK exon 20 of an ALK fusion gene, CD74 exon 6 and ROS1 exon 32 of a ROS1 fusion gene, KIF5B exon 16 and ROS1 exon 12 of a RET fusion gene, CD74 exon8 and NTRK1 exon10 of an NTRK1 fusion gene, TRIM24 exon12 and NTRK2 exon15 of an NTRK2 fusion gene, ETV6 exon5 and NTRK3 exon15 of an NTRK3 fusion gene, MET exon14 skipping of an MET skipping mutation gene, 19del, L858R, T790M of an EGFR mutation gene, G12D, G12C of a KRAS mutation gene, V600E of a BRAF mutation gene, V769_D770insASV and A775_G776insYVMA of an HER2 mutation gene are used to describe the above-mentioned lung cancer genes using a fluorescent PCR test of the present disclosure.

The experiment uses T7 RNA and various mutant plasmid templates containing the above-mentioned fusion gene, and the fluorescent PCR test comprises the following steps:
(1) Processing and extracting the plasmid and the T7 RNA:
   DNA is extracted from the plasmid by a plasmid extraction mini kit of Tiangen Biotechnology (Cat. No.: DPI03), and the plasmid is transcribed with a TranscriptAid T7 High Yield Transcription Kit (Cat. No.: K0441) to obtain the T7 RNA. The specific operation steps follow instructions of the kit. After the extraction of the DNA and the RNA is complete, concentrations and purities of the DNA and the RNA are immediately tested use an ultraviolet spectrophotometer. An OD260/OD280 of the DNA and the RNA should be between 1.7 and 2.1; and a concentration of the RNA should be between 10 and 100 ng/µL.
(2) Establishing a reaction system of a PCR amplification:
   The template is used as a template for real-time fluorescent PCR amplification, and PCR amplification is processed according to the following amplification system:
   A reaction system of reverse transcription: 5-25µL of purified water, 5-15µL of 5×reverse transcription buffer, 20-150µmol of each primer, and 100-200U of reverse transcriptase, and a total volume is 20-30µL;
   A reaction system of PCR: 15-45µL of purified water, 5-15µL of 10×PCR buffer, 1-10mmol of MgCl₂, 1-20pmol of each pair of primers, 1-20pmol of each pair of probes, 10~100pmol of dNTPs, and 1-10U of Taq enzyme, and a total volume is 30-50µL.
   Reaction conditions of the reverse transcription: 42°C for 60 minutes, 95°C for 5minutes, 1 cycle;
   S-cDNA 1 and S-cDNA 2 are obtained, then mixed with enzyme, respectively added into test tubes, LET PCR 8-tube strips 1-4 for genetic fusion and test tubes, LET PCR 8-tube strips 5-8 for genetic fusion; DNA sample is added to a test tube, that is, LET PCR 8-tube strips 1-8 for genetic mutation for PCR amplification.

Reaction conditions of real-time PCR are as follows:
A first stage: 42°C for 5 minutes, 95°C for 5minutes, 1 cycle;
A second stage: 95°C for 25 seconds, 64°C for 20 seconds, 72°C for 20 seconds, 10 cycles;
A third stage: 93°C for 25 seconds, 60°C for 35 seconds, 72°C for 20 seconds, 36 cycles;
Signal collection: FAM, VIC and ROX signals are collected at 60°C in the third stage, the real-time PCR is performed, and files are saved.

(III) Judgment for test results: the test results are judged according to a Ct value displayed by a fluorescence PCR amplification device (see Tables 9 and 10 for details).
1. That a fluorescence calibration is not selected is confirmed, a single test reaction tube is selected in order for analysis according to tube numbers, and a positive control reaction tube, a sample reaction tube and a negative control tube are selected at the same time, the user can then determine an inflection point of a rise of the amplification curve according to an actual situation, and Ct values of various reaction tubes are obtained.
2. Amplification curves of the FAM, VIC and ROX signals of a negative control:
   No amplification curve of FAM signals of the LET PCR 8-tube strips 1-8 for the genetic fusion and the FAM and ROX signals of the LET PCR 8-tube strips 1-7 for the genetic mutation of the negative control should rise, if the amplification curve of the signal of any tube rise, experimental results are invalid, and a re-test is recommended.
3. Amplification curves of the FAM, VIC and ROX signals of a positive control:
   ① All of amplification curves of FAM signals of the LET PCR 8-tube strips 1-8 for the genetic fusion and the LET PCR 8-tube strips 1-8 for the genetic mutation of the positive control should rise, and a Ct value is generally less than 25 and is possible to fluctuate due to different thresholds.
   ② All of an amplification curve of ROX signals of the LET PCR 8-tube strips 1-8 for the genetic mutation of the positive control should rise, and a Ct value is less than 25.
   ③ All of amplification curves of VIC signals of the LET PCR 8-tube strips 1-8 for the genetic fusion and the LET PCR 8-tube strips 1-7 for the genetic mutation of the positive control should rise, and a Ct value is less than 25.
4. Negative and positive determination for sample RNA genetic fusion:
   ① An amplification curve of VIC signals of the LET PCR 8-tube strips 1-8 for the genetic fusion:
      a. When all of the Ct values of the 8-tube strips are less than 36 and a Ct value of any one of tube strips is less than 26, an analysis is continually performed;
      b. When all of the Ct value of the 8-tube strips is no less than 26 and a Ct value of any one of tube strips is no less than 36, it means that fragmentation of the RNA or RNA degradation or inhibitors in the sample or omission occurs, re-test is recommended, or the RNA is re-extracted and then tested.
   ② An amplification curve of FAM signals of the LET PCR 8-tube strips 1-8 for the genetic fusion:
      a. When a Ct value of FAM signals of any one of tube strips falls in a positive section (see Table 9 for details), the sample is judged that the reaction tube is positive;
      b. When a Ct value of the FAM signals of all tubes fall in a negative section (see Table 9 for details), the sample is judged to be negative or lower than the lower detection limit of this kit. 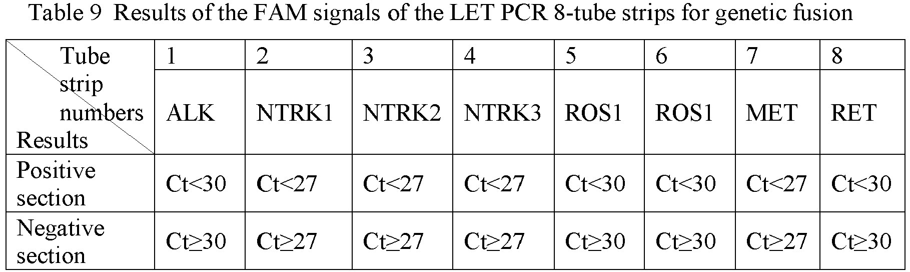 Note: A positive sample of a tube strip 5 may have a cross-reaction amplification in a tube strip 6. Therefore, when positive amplification curves of the tube strip 5 and the tube strip 6 both occur, it is determined that the tube strip 5 has a true positive amplification signal, and the tube strip 6 has a cross-reaction amplification signal.
5. Negative and positive determination of DNA mutations in the sample: in the LET PCR 8-tube strips for the genetic mutation, the Ct values of the FAM and ROX signals of each of the tube strips 1-7 of the sample is first determined, and the Ct values of the FAM and ROX signals of the tube strip 8 of the sample is then determined. Resulting mutation Ct values are also different due to different mutation percentages in the samples. According to different mutation Ct values, the sample test results are divided into negative, a positive section A and a positive section B. See Table 10 for specific judgments.
   ① The amplification curve of the VIC signal of the LET PCR 8-tube strips 1-7 for the genetic mutation of the sample:
      a. When all of the Ct values of the tube strips 1-7 are less than 36, an analysis is continually performed;
      b. When the Ct value of any one of the tube strips 1-7 is no less than 36, it indicates that fragmentation of the DNA or DNA degradation or inhibitors in the sample or omission occurs, re-test is recommended, or the DNA is re-extracted and then tested.
   ② The amplification curve of the FAM and ROX signals of the LET PCR 8-tube strips 1-8 for the genetic mutation tube of the sample:
      a. When a Ct value of FAM and ROX signals of a tube strip 8 is no less than 19 and is no more than 25, an analysis is continually performed;
      b. When the Ct value of the FAM and ROX signals of the tube strip 8 is less than19, it indicates that the DNA addition is excessive, an amount of the DNA addition should be reduced, and the experiment is then performed. However, the signal for the mutation does not rise or fall in the negative section, experimental results are still credible; 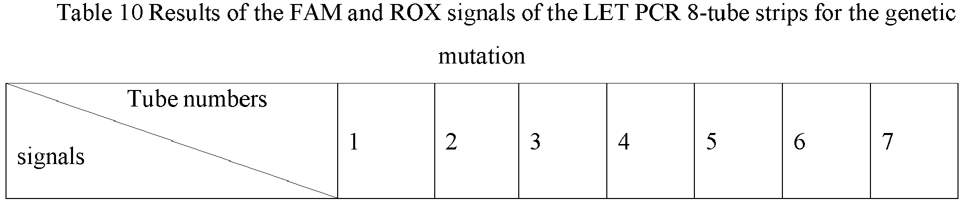 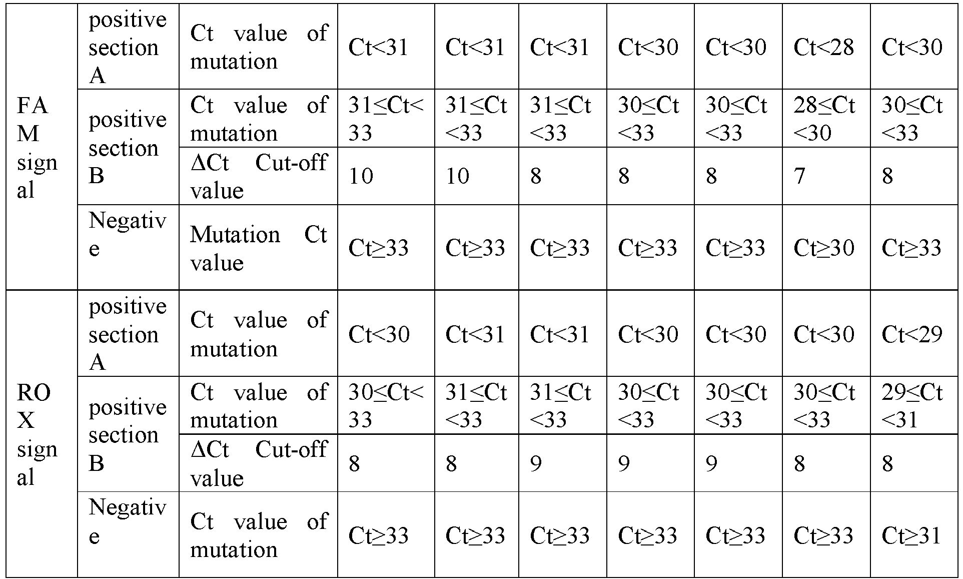
      c. When the Ct value of the FAM and ROX signals of the tube strip 8 is more than 25, it indicates that fragmentation or degradation of the added DNA or inhibitors in the sample or omission occurs. When the signal for the mutation rises and falls in the positive section A, the experimental result is still credible, otherwise an amount increase of the added DNA is recommended, or the DNA is re-extracted and then tested;
      d. When the Ct values of the FAM and ROX signals of all of the tube strips 1-7 of the sample are more than or equal to a negative critical value (that is, they fall in the negative section), the sample is negative or lower than a lower test limitation of this kit;
      e. When the Ct value of the FAM and ROX signals of one reaction tube strip of the tube strips 1-7 is less than the negative critical value, the following judgments are performed:
         i. When the Ct value of the one reaction tube strip is less than a positive critical value (that is, it falls in the positive section A), then the sample is a corresponding positive mutation of the one reaction tube strip;
         ii. When the Ct value of the one reaction tube strip is more than a positive critical value and less than a negative critical Ct value (that is, it falls in the positive section B), the ΔCt value of the reaction tube strip is calculated. When a ΔCt value of the one reaction tube strip is less than a corresponding ΔCt Cut-off value, the sample is also a corresponding positive mutation of the one reaction tube strip, that is, positive section B; otherwise, it is negative or lower than the lower test limitation of the kit.
   ③ Calculation of the ΔCt value: ΔCt value=a Ct value of a FAM (ROX) of mutation-a Ct value of FAM (ROX) of an external control. The Ct value of the FAM (ROX) of the mutation is a corresponding Ct value of mutation signals (FAM or ROX signal) of the tube strips 1-7 of the sample; the Ct value of the external control is a corresponding Ct value of an external signal (FAM or ROX signal) of the tube strip 8 of the sample.
6. Some EGFR, HER2, KRAS positive samples may cause cross signals between several mutation reaction tube strips. When a positive result occurs in 2 or more than 2 reaction tube strips (which is interpreted in Table 10) of the sample, the mutation reaction tube strip with a smallest Ct value is true positive, and the ΔCt value of each reaction tube strip (the Ct value of the mutation-the Ct value of the external control) is then calculated, and whether the other reaction tube strips are cross-signals or not is determined according to a threshold of cross-signal listed in Tables 11-13.
   ① When the ΔCt value is less than the threshold of the cross signal, it is considered to be a true positive signal, and the mutation reaction tube strip should be judged to be positive;
   ② When the ΔCt value is more than or equal to the threshold cross, it is considered to be a cross signal, and the mutation reaction tube strip should be judged as negative. 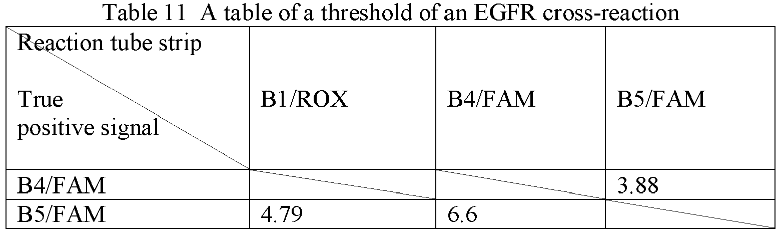 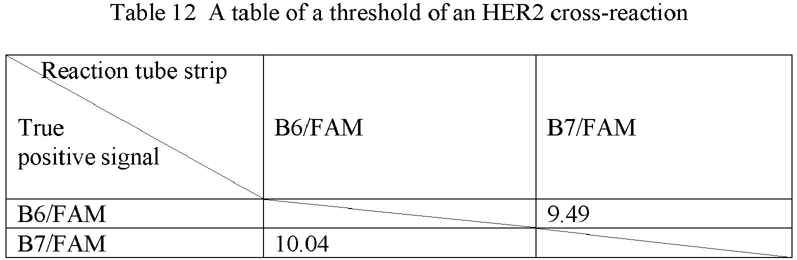 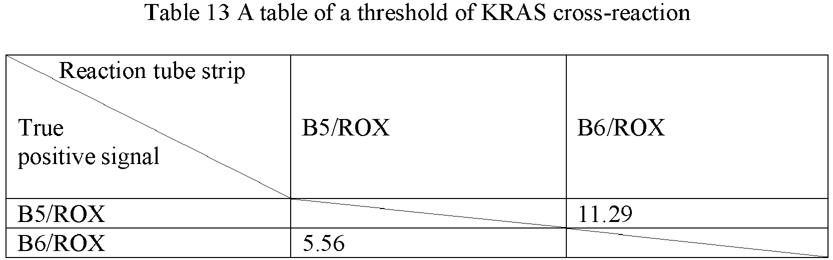
7. The sample may contain 2 or more fusion or mutation types at the same time.

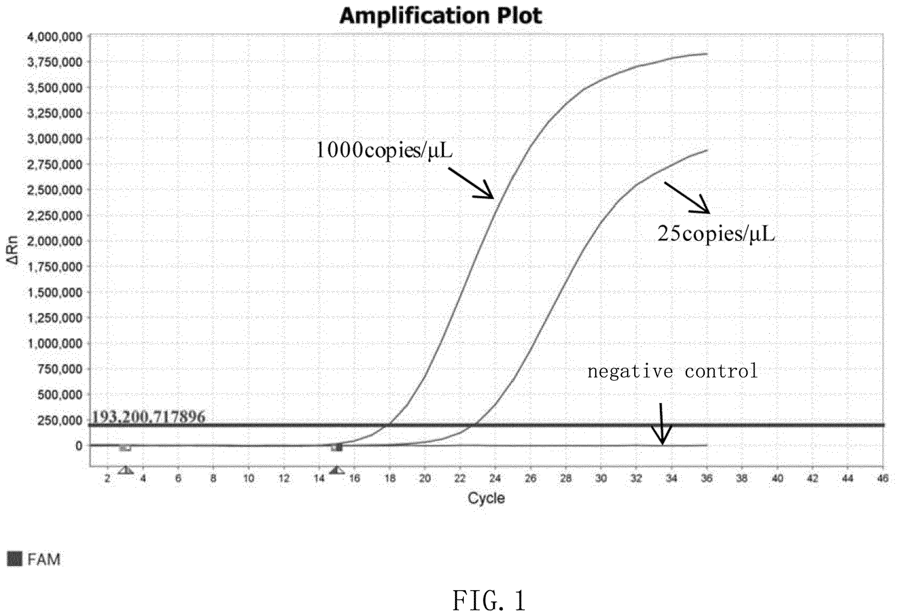

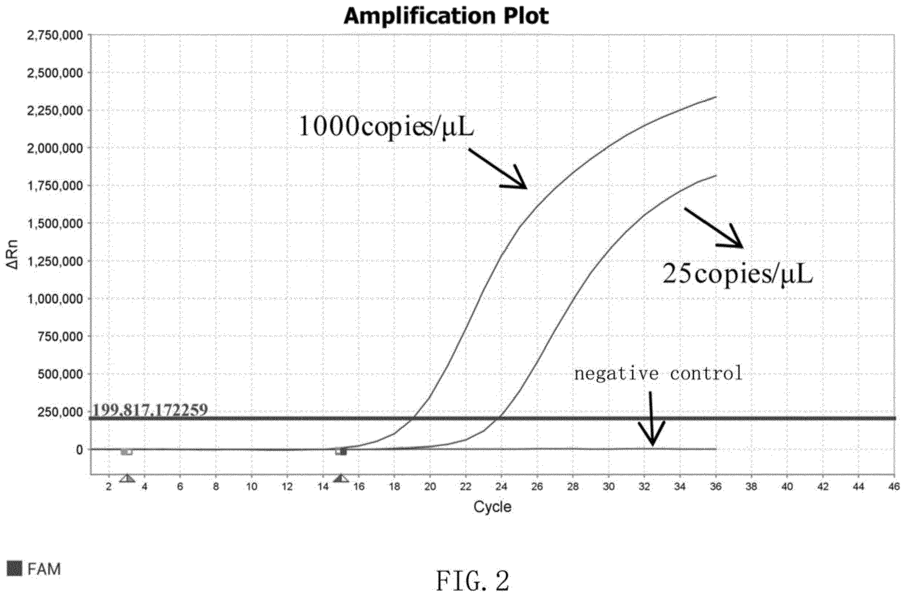

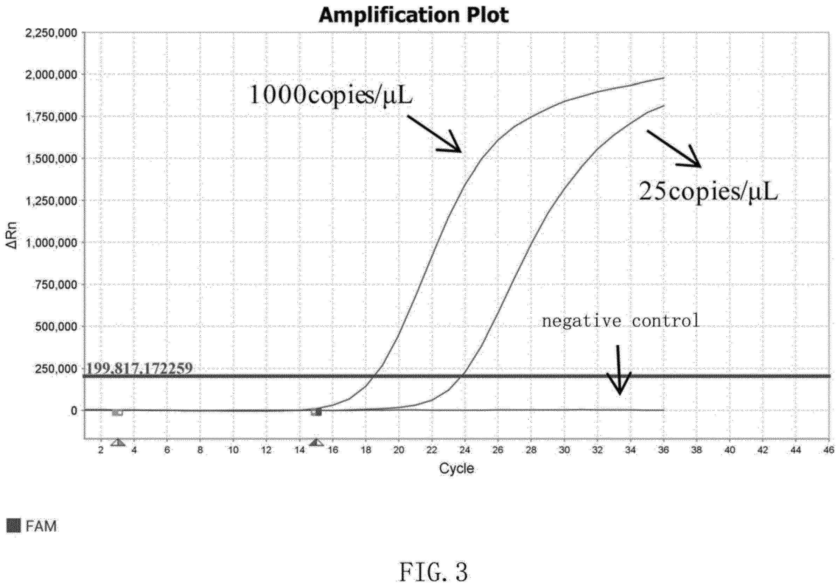

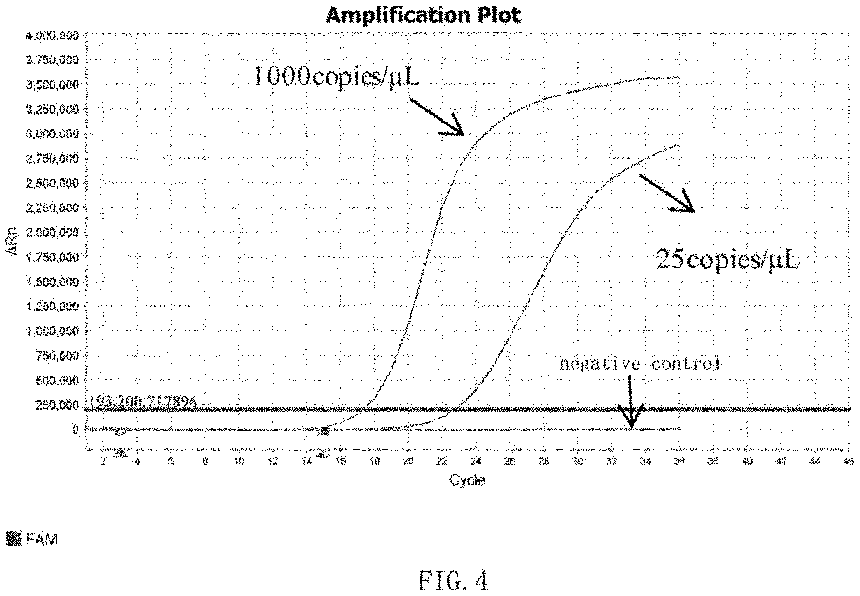

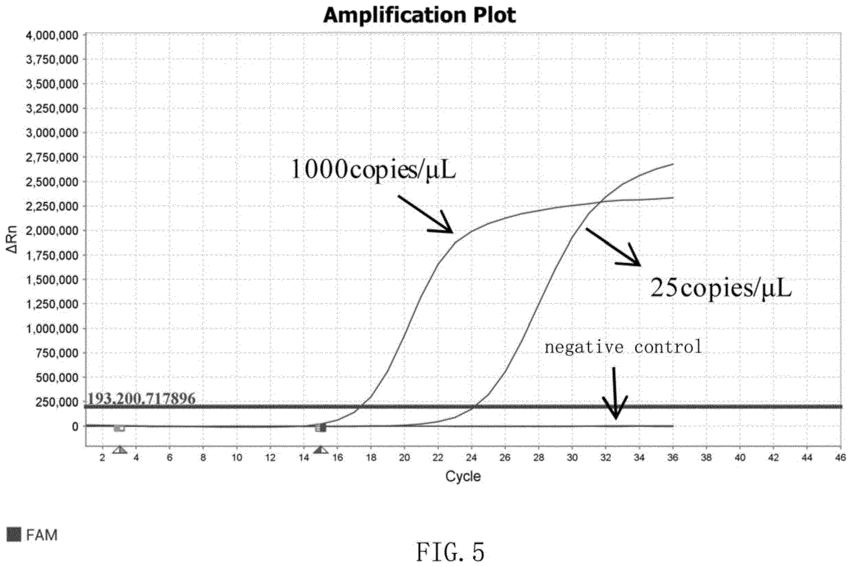

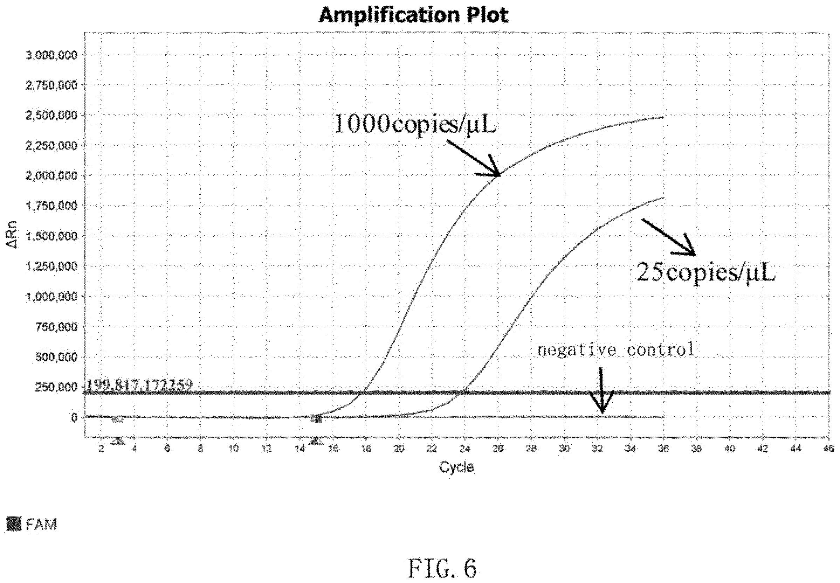

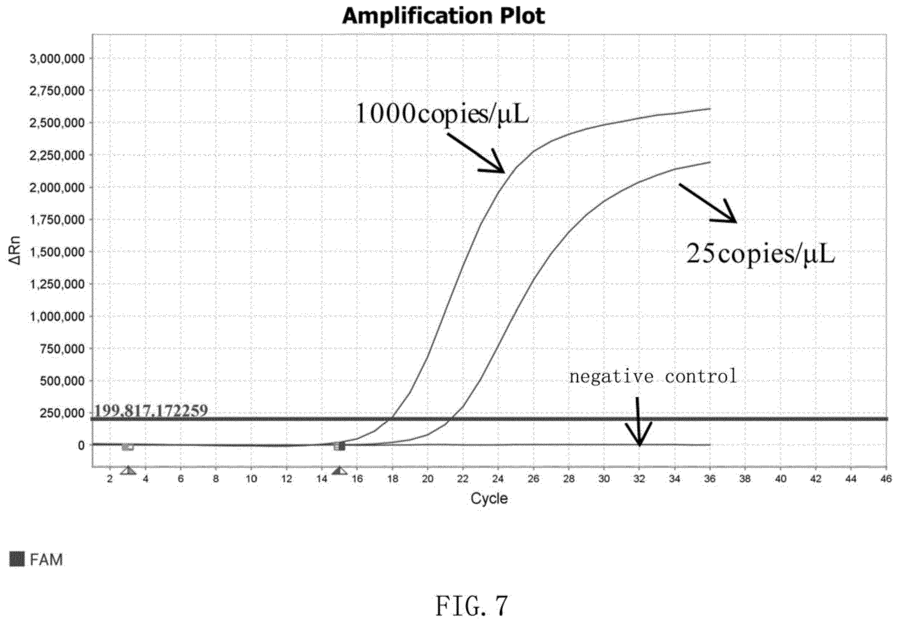

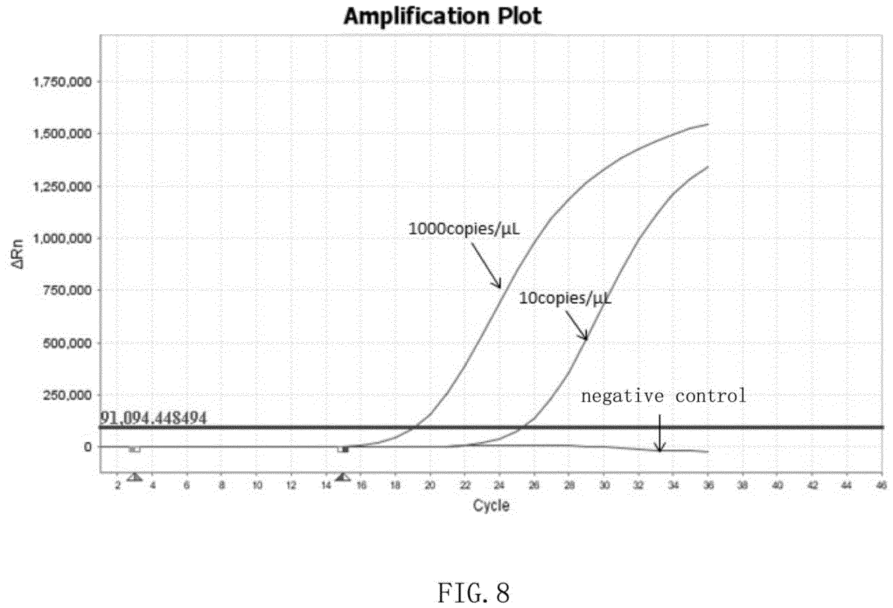

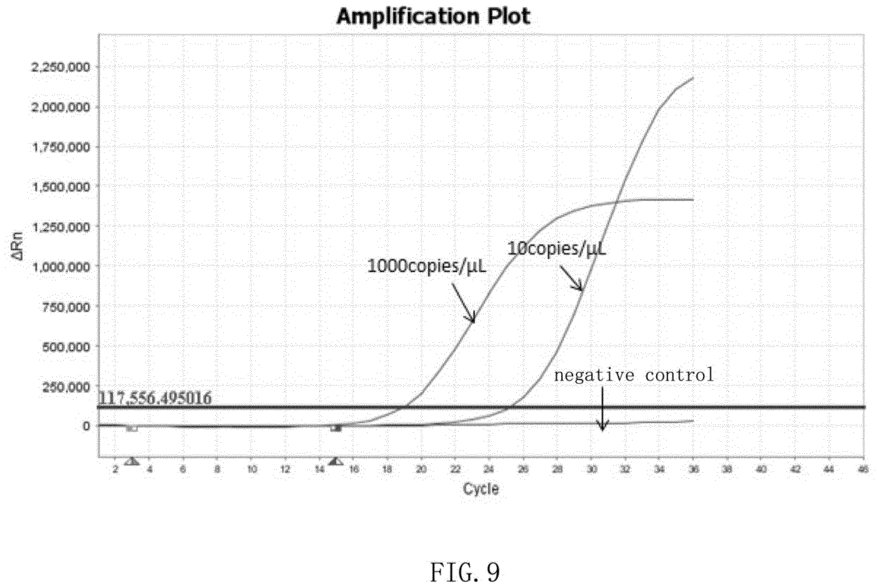

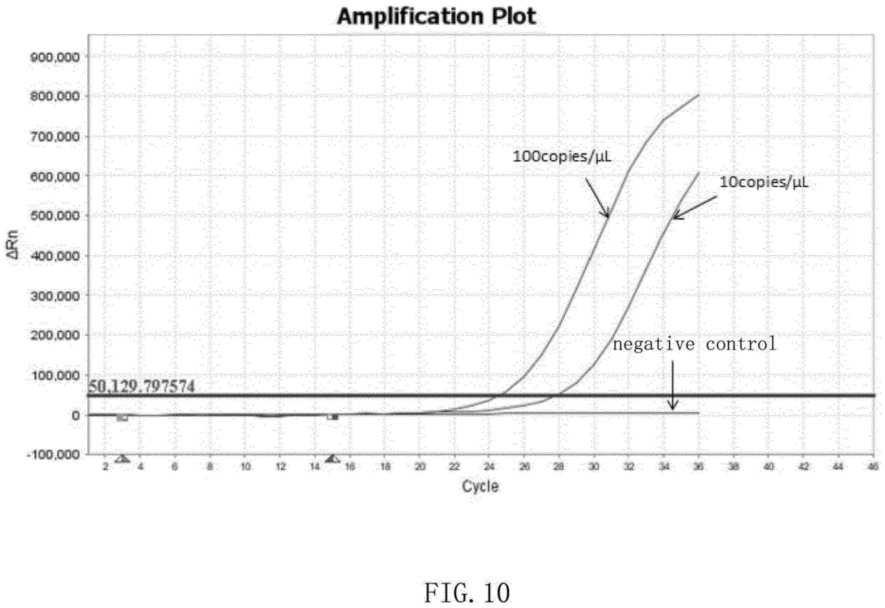

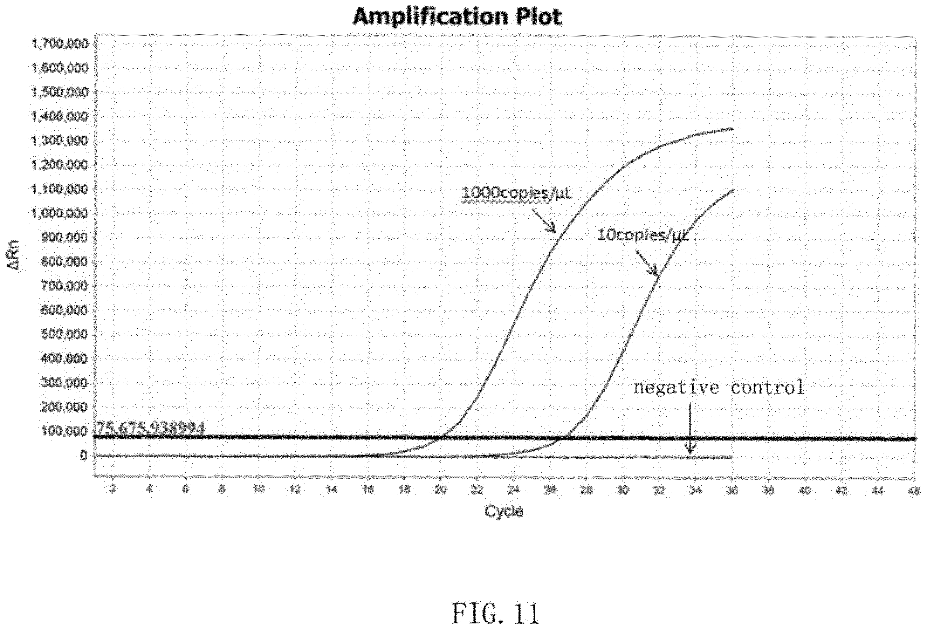

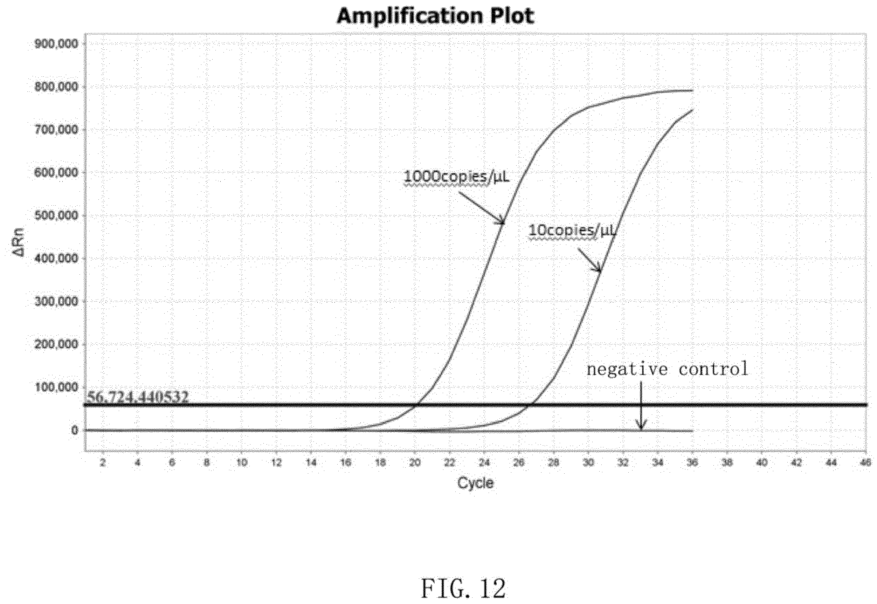

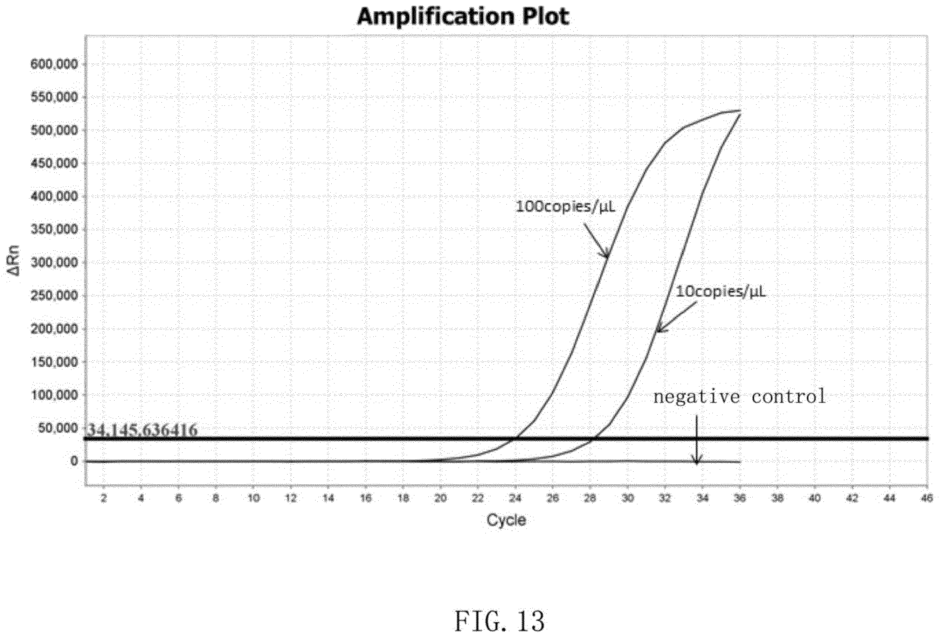

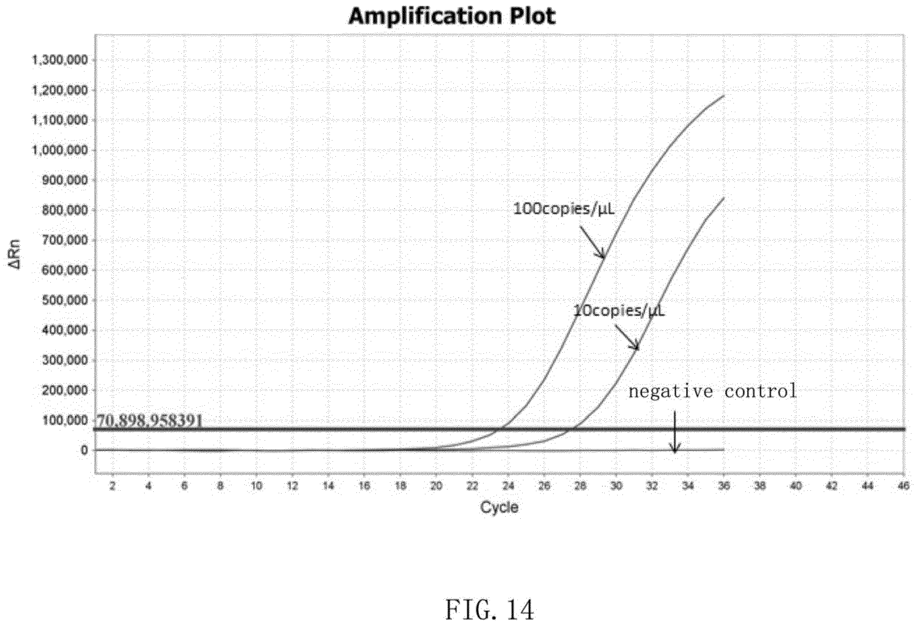

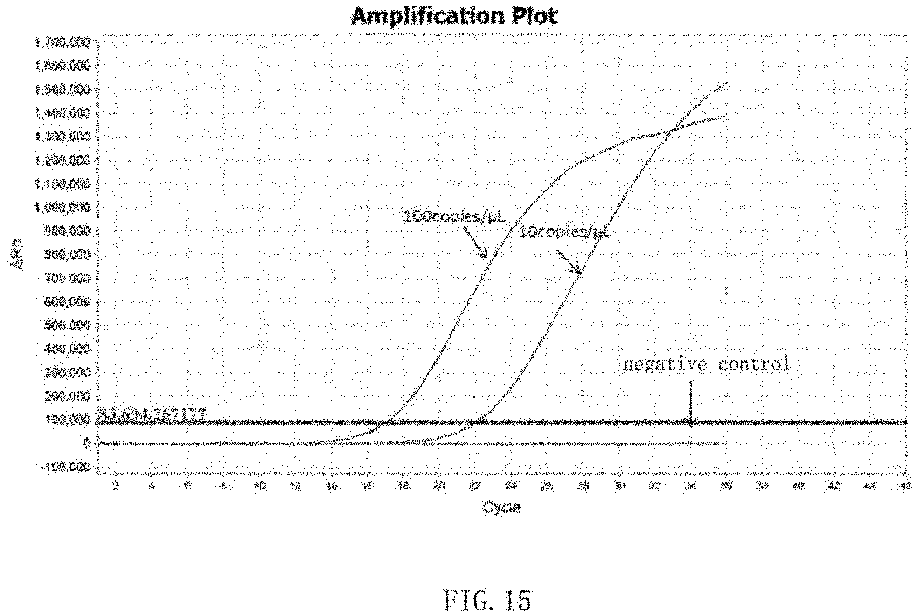

(IV) Sensitivity analysis: DNA and RNA plasmids are taken for gradient dilution, and at least 2 concentrations of each gene are eventually used for test limitation. The results show that the fluorescence PCR method of the present disclosure has high sensitivity, 1% of genetic mutation DNA and 25 copies/µL of fusion gene plasmid can be detected. Part of the results is shown in FIGS. 1-15.

The test result shows that 11 lung cancer genes can be accurately tested at one time using a test system of the present disclosure, and a detection sensitivity can reach 1% of genetic mutation DNA and 25 copies/µL of the fusion gene RNA.

### Embodiment 2

The present disclosure is used to analyze clinical samples, 172 clinical NSCLC FFPE samples were tested for mutations/fusions of 11 lung cancer genes (which comprises some clinical samples with known results) in November 2019 and compared with the NGS method.
I. Extraction of DNA and RNA from test samples: DNA and RNA are extracted using nucleic acid extraction reagents from AMOY DIAGNOSTICS CO., LTD. (type: FFPE DNA/RNA, medical equipment registration number: Minxia equipment No. 20150082, cat. No.: 8.0223601X036G). After the extraction of DNA and RNA is complete, concentrations and purities of the DNA and the RNA are tested by a micro-ultraviolet spectrophotometer. The concentration of the RNA should be more than 10 ng/µL, the concentration of the DNA should be more than 2ng/µL, and OD260/OD280 of the DNA and the RNA should be between 1.7 and 2.1. The aforementioned extracted DNA and RNA are used as amplification templates for testing the 11 lung cancer genes.
II. PCR amplification of the aforementioned DNA and RNA are respectively performed according to the following amplification system:
   A reaction system of reverse transcription: 5-25µL of purified water, 5-15µL of 5×reverse transcription buffer, 20-150µmol of each primer, and 100-200U of reverse transcriptase, and a total volume is 20-30µL;
   A reaction system of PCR: 15-45µL of purified water, 5-15µL of 10×PCR buffer, 1-10mmol of MgCl2, 1-20pmol of each pair of primers, 1-20pmol of each pair of probes, 10-100pmol of dNTPs, and 1-10U of Taq enzyme, and a total volume is is 30-50µL. Reaction conditions for the reverse transcription: 42°C for 60 minutes, 95°C for 5minutes, 1 cycle;
   S-cDNA 1 and S-cDNA 2 are obtained, then mixed with enzyme, respectively added to test tubes, that is, LET PCR 8-tube strips 1-4 and 5-8 for genetic fusion; DNA samples are added to test tubes, that is, LET PCR 8-tube strips 1-8 for genetic mutation, PCR amplification is performed.
   Reaction conditions of real-time PCR are as follows:
      A first stage: 42°C for 5 minutes, 95°C for 5 minutes, 1 cycle;
      A second stage: 95°C for 25 seconds, 64°C for 20 seconds, 72°C for 20 seconds, 10 cycles;
      A third stage: 93°C for 25 seconds, 60°C for 35 seconds, 72°C for 20 seconds, 36 cycles;
   Signal collection: FAM,VIC and ROX signals are collected at 60°C in the third stage, the real-time PCR is performed, and files are saved.
III. Judgment of test results: the test results are judged according to a Ct value displayed by a fluorescence PCR amplification device (the judgment method refers to Embodiment 1).
IV. NGS test. Qualified DNA samples are extracted and then fragmented, and an end is then repaired, and an adaptor is ligated. Capture probes of human pan-tumor 116 gene panel of AMOY DIAGNOSTICS are used to capture a target section. Captured library with qualified quality control are sequenced using the Illumina Novaseq 6000 sequencing platform, and a joint detection data analysis system of human pan-tumor 116 genetic mutation (ADXPAN116-tMut_v0.2.0) of AMOY DIAGNOSTICS is used to analyze data of the sequencing to obtain genetic mutation results of the 116 genes (including EGFR, KRAS, BRAF, HER2, ALK, ROS1, RET, NTRK1, NTRK2, NTRK3, MET, etc.).

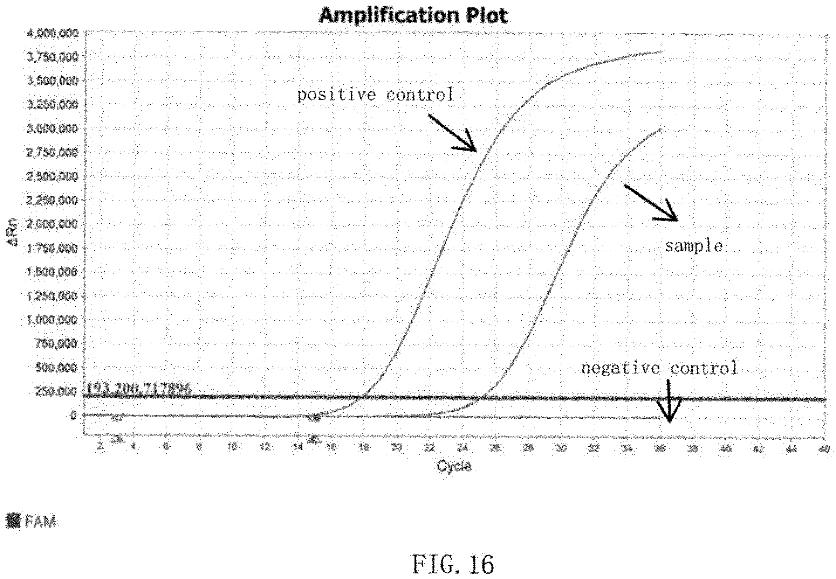

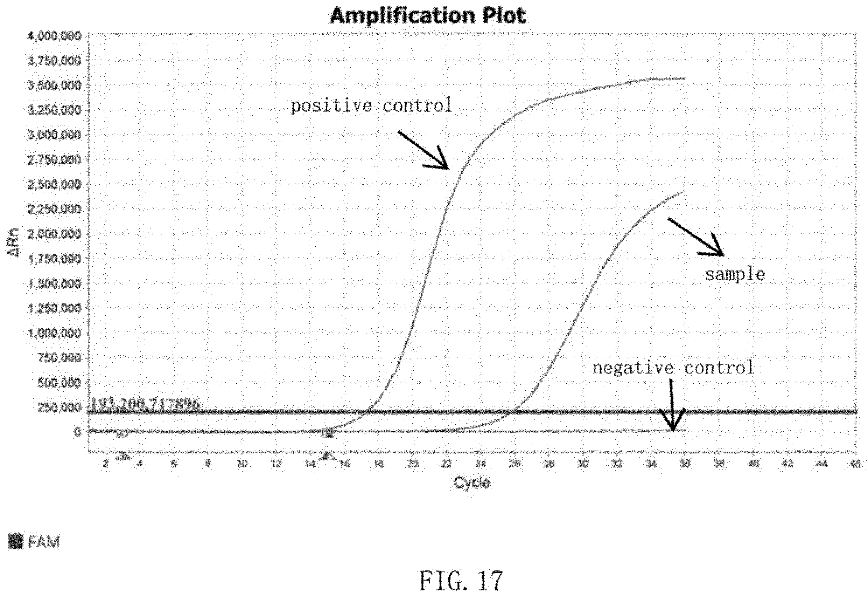

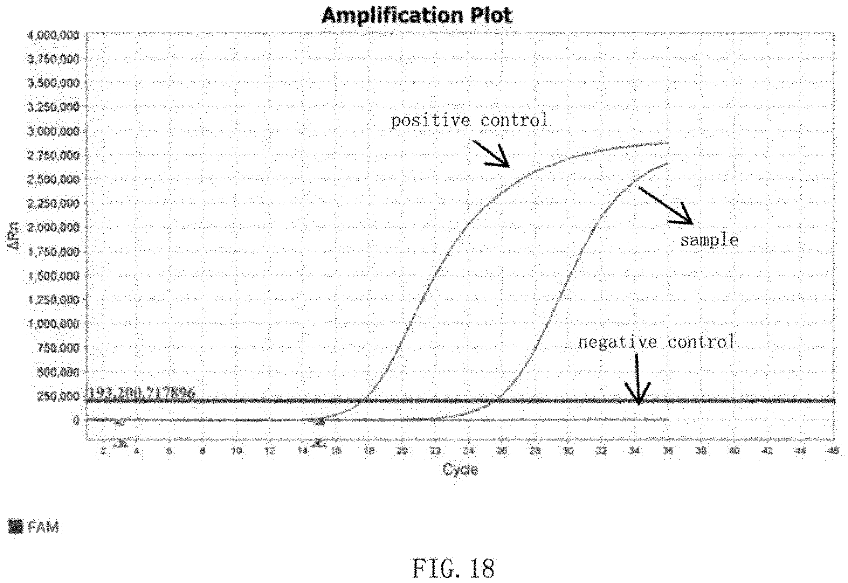

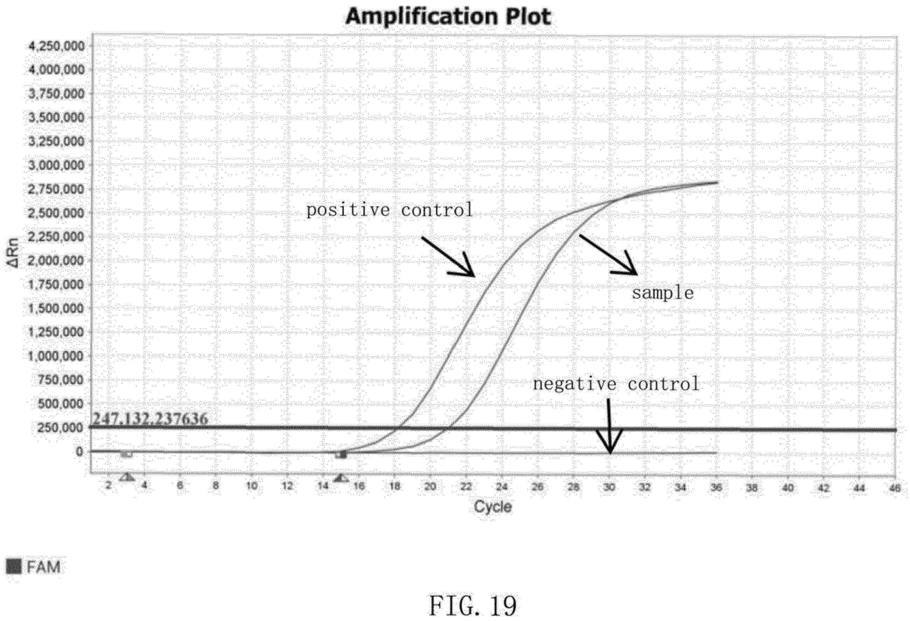

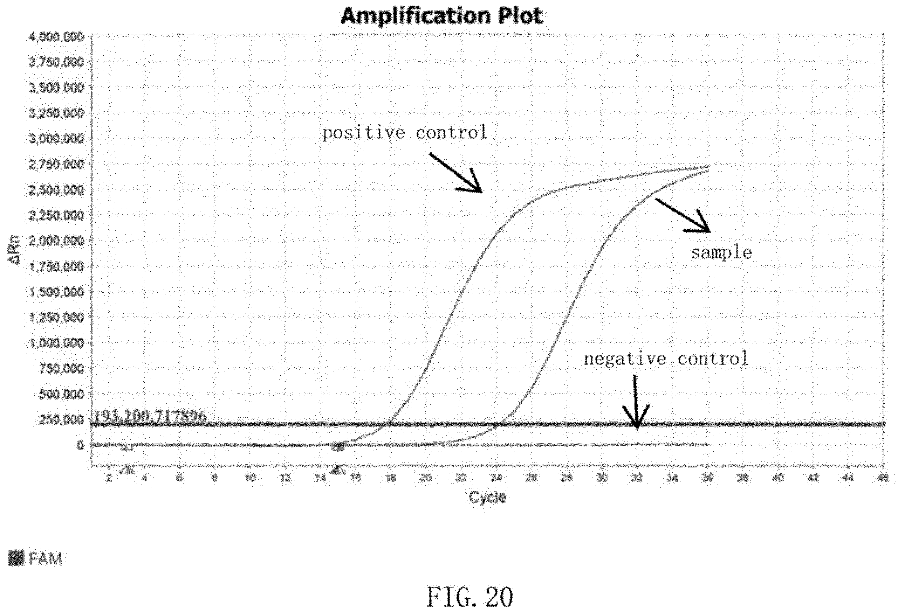

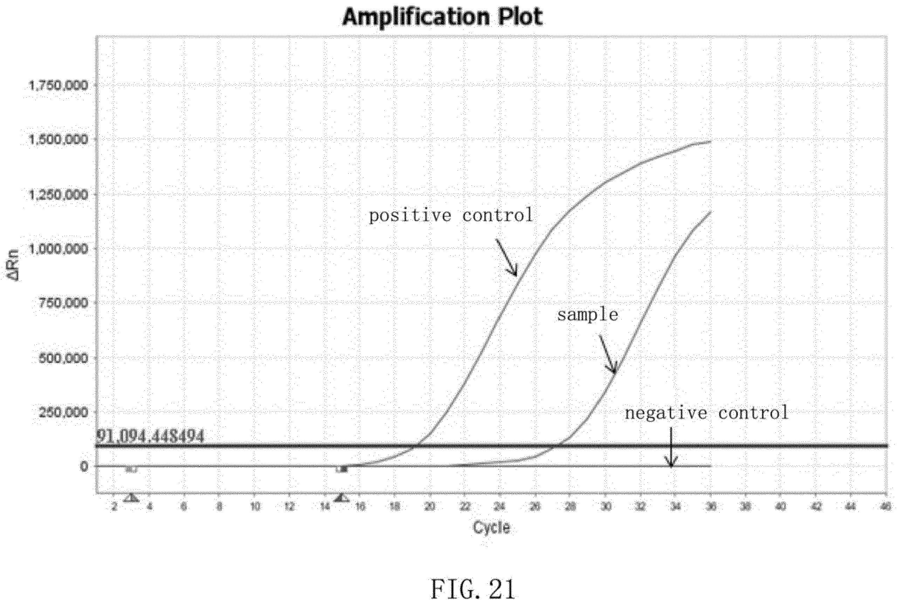

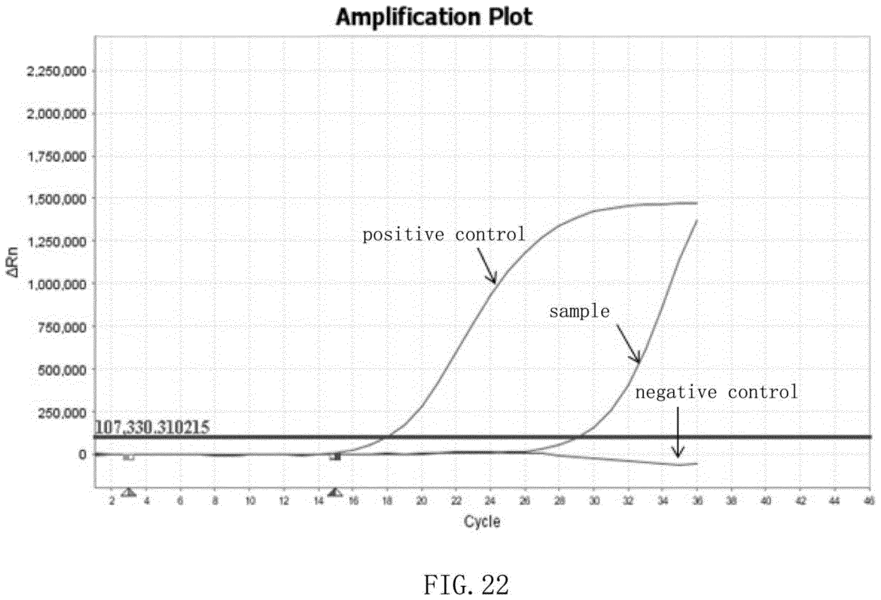

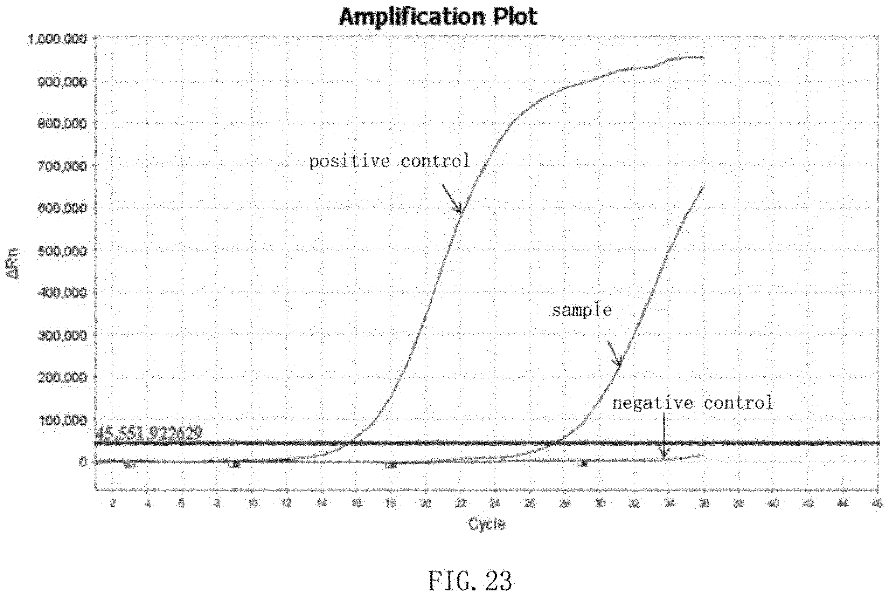

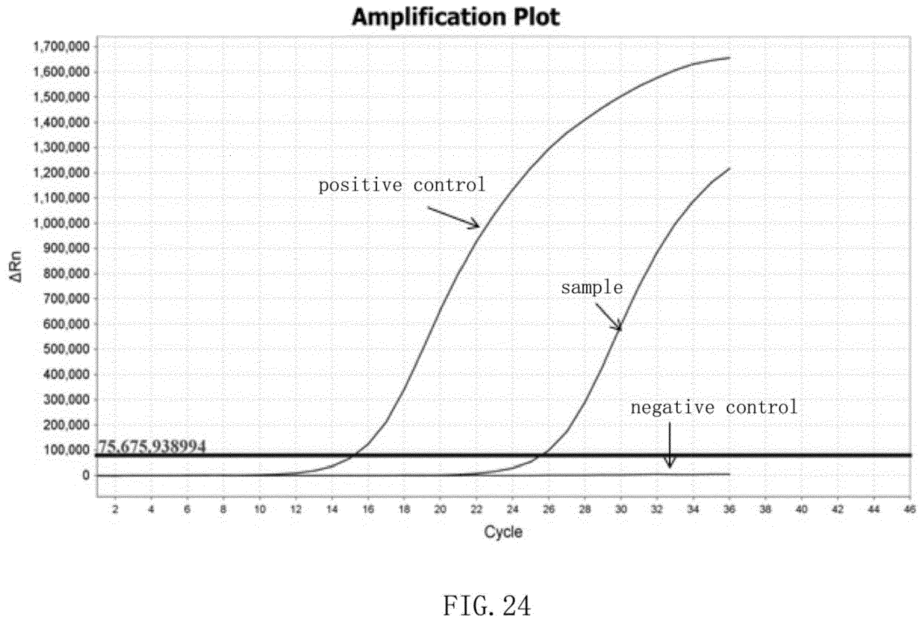

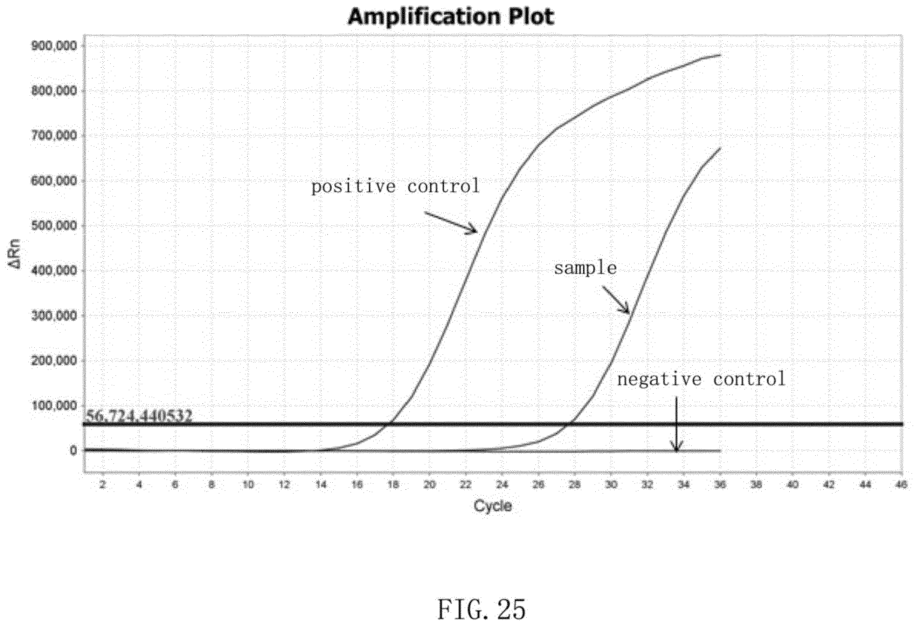

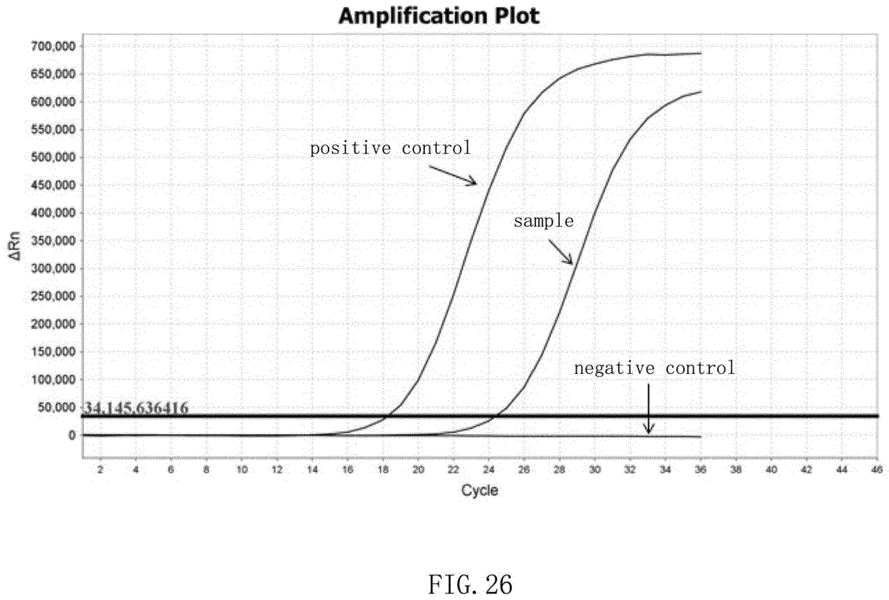

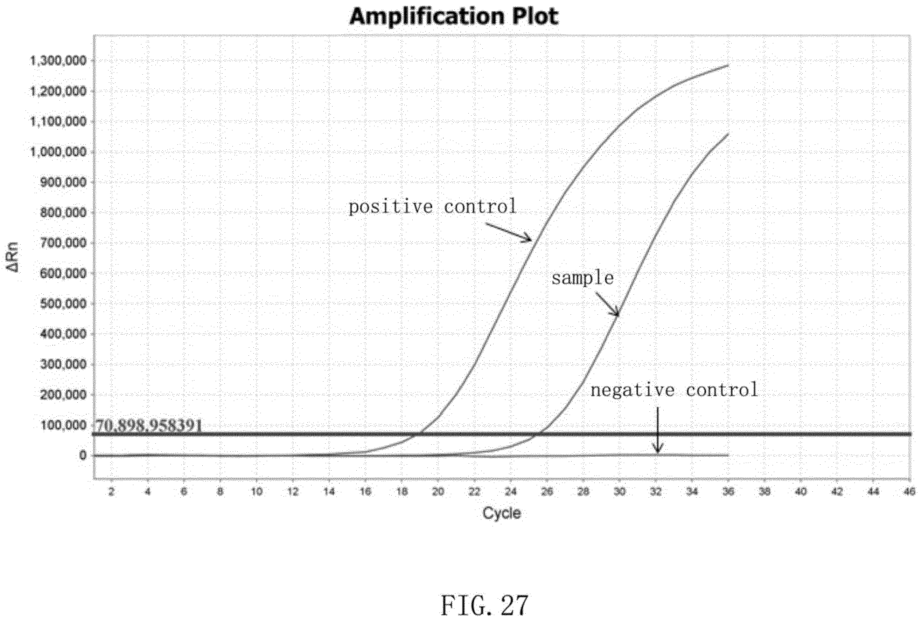

125 positive samples are detected from 172 clinical samples by the kit of the present disclosure. A mutation distribution of the samples is shown in Tables 11 and 12 and FIGS. 16-27.

**Table 11 The mutation distribution of the clinical samples**

| Mutation name | The present disclosure (number of cases) | NGS (number of cases) |
|---|---|---|
| EGFR mutation, positive | 73 | 72 |
| KRAS mutation, positive | 13 | 13 |
| BRAF mutation, positive | 3 | 3 |
| HER2 mutation, positive | 1 | 1 |
| ALK fusion, positive | 11 | 11 |
| ROS1 fusion, positive | 8 | 8 |
| NTRK3 fusion, positive | 4 | 4 |
| RET fusion, positive | 7 | 7 |
| MET Skipping, positive | 7 | 7 |

**Table 12 4×4 table analysis of the test results of the present disclosure and the NGS**

| | NGS | | | |
|---|---|---|---|---|
| The present disclosure | | Positive | Negative | Total |
| | Positive | 124 | 1 | 125 |
| | Negative | 0 | 47 | 47 |
| | Total | 124 | 48 | 172 |

One inconsistent sample is verified using a marketed human EGFR genetic mutation reagent kit (a fluorescence PCR method) (National Machinery registration license 20143402001), and the result is positive for EGFR 19del and is consistent with the test result of the present disclosure.

Test data analysis of the two methods is as follows:
A positive concordance rate=124/124=100.00% (95%CI: 97.64%, 100.76%);
A negative concordance rate=47/48=97.92% (95%CI: 93.88%, 101.96%);
A total concordance rate=171/172=99.42% (95%CI: 98.28%, 100.55%).

Kappa value=0.985, standard error SK=0.0145, Z value=67.965, according to the Z value, a t critical value table is searched using v =∞, a critical value of t is 1.96 (two-sided 0.05) or 1.64 (one-sided 0.05), Z=67.965>1.96, P<0.05, the Kappa value can be considered statistically significant, and the test results of the two kits are consistent.

A total concordance rate of results of the fluorescent PCR method and the NGS is as high as 99.42%, and a sensitivity and a selective test ability of the fluorescent PCR are equivalent to the NGS. Compared with the NGS, 11 genes are quickly tested in convenience using the PCR method, a short time is taken, the samples are saved, and is more conducive to clinical application.

The aforementioned embodiments are merely some embodiments of the present disclosure, and the scope of the disclosure of is not limited thereto. Thus, it is intended that the present disclosure cover any modifications and variations of the presently presented embodiments provided they are made without departing from the appended claims and the specification of the present disclosure.

### INDUSTRIAL APPLICABILITY

The present disclosure is to provide a reagent kit for testing multiple lung cancer genetic mutations at one time, it comprises primers, probes and a distribution method for EGFR, KRAS, BRAF, HER2, ALK, ROS1, RET, NTRK1, NTRK2, NTRK3, MET, other mutation genes for the lung cancer. The present disclosure uses a design of PCR 8-tube strips, every two PCR 8-tube strips are used to test 11 genetic mutations/fusions, one PCR 8-tube strip contains a corresponding fusion test reagent and an internal control reagent; the other PCR 8-tube strip contains a corresponding mutation test reagent. The present disclosure tests 119 genetic fusions and 112 genetic mutations of lung cancer at one time using a fluorescent PCR method, a test time is greatly shortened, an operation is convenient, a result is accurate, and industrial applicability is good.

## Claims

1. A reagent kit for testing a lung cancer genetic mutation, **characterized in that** the reagent kit comprises:
a reagent for testing sites of an A1 group for a lung cancer genetic fusion, the sites of the A1 group comprise EML4-ALK-1 to EML4-ALK-3, EML4-ALK-6 to EML4-ALK-14, EML4-ALK-17 To EML4-ALK-21; KIF5B-ALK-1; KIF5B-ALK-2; KLC1-ALK; TFG-ALK; STRN-ALK-1; HIP1-ALK-1; HIP1-ALK-2; HIP1-ALK-3; PRKAR1A-ALK-1; PRKAR1A-ALK-2; NBAS-ALK-1; TFG-ALK-2; PPM1B-ALK-1; EIF2AK3-ALK-1; BCL11A-ALK-1; BIRC6-ALK-1; CEBPZ-ALK-1; CLIP1-ALK-1; COL25A1-ALK-1; GCC2-ALK-1; GCC2-ALK-2; LMO7-ALK-1; PICALM-ALK-1; PHACTR1-ALK-1; TPR-ALK-1; MPRIP-ALK-1; TNIP2-ALK-1; DCTN1-ALK-1; SQSTM1-ALK-1;
a reagent for testing sites of an A5 group for the lung cancer genetic fusion, the sites of the A5 group comprise ROS1-M1 to ROS1-M10;
a reagent for testing sites of a B1 group of a lung cancer mutation gene, the sites of the B1 group comprise E-19-M1 to E-19-M21; E-19-M24 to E-19-M27; E-20-M2;
a reagent for testing sites of a B2 group of the lung cancer mutation gene, the sites of the B2 group comprise E-21-M1; E-18-M1; E-18-M2; E-18-M3; and
a reagent for testing sites of a B3 group of the lung cancer mutation gene, the sites of the B3 group comprise E-20-M1; E-21-M2.

2. The reagent kit for testing the lung cancer genetic mutation according to claim 1, **characterized in that** a reaction system of the reagent kit comprises:
(1) a reverse transcription reaction system: 5-25µL of purified water, 5-15µL of 5×reverse transcription buffer, 20-150µmol of each primer, and 100-200U of reverse transcriptase, and a total volume is 20-30µL;
(2) a PCR reaction system: 15-45µL of purified water, 5-15µL of 10×PCR buffer, 1-10mmol of MgCl₂, 1-20pmol of each pair of primers, 1-20pmol of each pair of probes, 10∼100pmol of dNTPs, and 1-10U of Taq enzyme, and a total volume is 30-50µL.

3. The reagent kit for testing the lung cancer genetic mutation according to claim 1, **characterized in that** the reagent combination comprises the following primers and probes: the reagent combination for testing the sites of the A1 group fro the lung cancer genetic fusion: SEQ ID NO:1-SEQ ID NO:44.

4. The reagent kit for testing the lung cancer genetic mutation according to claim 1, **characterized in that** the reagent combination comprises the following primers and probes: the reagent combination for testing the sites of the A5 group for the lung cancer genetic fusion: SEQ ID NO:92-SEQ ID NO:104.

5. The reagent kit for testing the lung cancer genetic mutation according to claim 1, **characterized in that** the reagent combination comprises the following primers and probes: the reagent combination for testing the sites of the B1 group for the lung cancer genetic fusion: SEQ ID NO:155-SEQ ID NO:174.

6. The reagent kit for testing the lung cancer genetic mutation according to claim 1, **characterized in that** the reagent combination comprises the following primers and probes: the reagent combination for testing the sites of the B2 group for the lung cancer genetic fusion: SEQ ID NO:175-SEQ ID NO:187.

7. The reagent kit for testing the lung cancer genetic mutation according to claim 1, wherein the reagent combination comprises the following primers and probes: the reagent combination for testing the sites of the B3 group for the lung cancer genetic fusion: SEQ ID NO:188-SEQ ID NO:198.

8. A reagent kit for testing a lung cancer genetic mutation comprises 15 combinations:
a reagent for testing the following sites of an A1 group comprises EML4-ALK-1 to EML4-ALK-3, EML4-ALK-6 to EML4-ALK-14, EML4-ALK-17 to -EML4-ALK-21; KIF5B-ALK-1; KIF5B-ALK-2; KLC1-ALK; TFG-ALK; STRN-ALK-1; HIP1-ALK-1; HIP1-ALK-2; HIP1-ALK-3; PRKAR1A-ALK-1; PRKAR1A -ALK-2; NBAS-ALK-1; TFG-ALK-2; PPM1B-ALK-1; EIF2AK3-ALK-1; BCL11AALK-1; BIRC6-ALK-1; CEBPZ-ALK-1; CLIP1-ALK-1; COL25A1-ALK-1; GCC2-ALK-1; GCC2-ALK-2; LMO7-ALK-1; PICALM-ALK-1; PHACTR1-ALK-1; TPR-ALK-1; MPRIP-ALK-1 ; TNIP2-ALK-1; DCTN1-ALK-1; SQSTM1-ALK-1;
a reagent for testing the following sites of an A2 group comprises NTRK1-E9-M1; NTRK1-E10-M1; NTRK1-E10-M3; NTRK1-E10-M5 to NTRK1-E10-M9; NTRK1-E10-M12; NTRK1-E10-M14; NTRK1-E10-M15; NTRK1-E10-M17; NTRK1-E12-M1; NTRK1-E12-M3; NTRK1-E12-M4; NTRK1-E12-M11; NTRK1-E12-M12; NTRK1-E12- M14;
a reagent for testing the following sites of an A3 group comprises NTRK2-E15-M1; NTRK2-E16-M1; NTRK2-E16-M3; NTRK2-E16-M7; NTRK2-E17-M2;
a reagent for testing the following sites of an A4 group comprises NTRK3-EX14-M1; NTRK3-EX14-M2; NTRK3-EX14-M3; NTRK3-EX14-M4; NTRK3-EX14-M7; NTRK3-EX15-M1; NTRK3- EX15-M2; NTRK3-EX15-M3;
a reagent for testing the following sites of an A5 group comprises ROS1-M1 to ROS1-M10;
a reagent for testing the following sites of an A6 group comprises ROS1-M11; ROS1-M12; ROS1-M13; ROS1-EX35-M1 to ROS1-EX35-M4; ROS1-EX35-M6; ROS1-EX35-M8 to ROS1-EX35-M11; ROS1-EX35-M13;
a reagent for testing the following sites of an A7 group comprises MET-M1;
a reagent for testing the following sites of an A8 group comprises RET-M2; RET-M5; RET-M15; RET-M16; RET-M17; RET-M19; LRET-M22; LRET-M32; LRET-M40; LRET-M41; LRET-M42; LRET-M44; LRET-M45; LRET-M49; LRET-M55; LRET-M57; LRET-M58; LRET-M59;
a reagent for testing the following sites of a B1 group comprises E-19-M1 to E-19-M21; E-19-M24 to E-19-M27; E-20-M2;
a reagent for testing the following sites of a B2 group comprises E-21-M1; E-18-M1; E-18-M2; E-18-M3;
a reagent for testing the following sites of a B3 group comprises E-20-M1; E-21-M2;
a reagent for testing the following sites of a B4 group comprises E-20-M4; E-20-M5; E-20-M8; E-20-M9; E-20-M13; E-20-M14; E-20-M20; E-20-M21; E-20-M23; E-20-M24; E-20-M28 to E-20-M31; E-20-M36 to E-20-M41; E-20- M44; E-20-M48; E-20-M50 to E-20-M53; BRAF-M1;
a reagent for testing the following sites of a B5 group comprises E-20-M3; E-20-M10; E-20-M12; E-20-M15 to E-20-M19; E-20-M22; E-20-M25 to E-20-M27; E- 20-M32 to E-20-M35; E-20-M42; E-20-M43; E-20-M45 to E-20-M47; E-20-M49; E-20-M55 to E-20- M57; KRAS-M6;
a reagent for testing the following sites of a B6 group comprises HER2-M1 to HER2-M3; HER2-M8; HER2-M9; HER2-M15 to HER2-M26; KRAS-M2; KRAS-M3; KRAS-M5; KRAS - M14;
a reagent for testing the following sites of a B7 group comprises HER2-M4; HER2-M6; HER2-M7; HER2-M10; KRAS-M1; KRAS-M4.

9. The reagent kit for testing the lung cancer genetic mutation according to claim 8, **characterized in that** a reaction system of the reagent kit comprises:
(1) a reverse transcription reaction system: 5-25µL of purified water, 5-15µL of 5×reverse transcription buffer, 20-150µmol of each primer, and 100-200U of reverse transcriptase, and a total volume is 20-30µL;
(2) a PCR reaction system: 15-45µL of purified water, 5-15µL of 10×PCR buffer, 1-10mmol of MgCl₂, 1-20pmol of each pair of primers, 1-20pmol of each pair of probes, 10~100pmol of dNTPs, and 1-10U of Taq enzyme, and a total volume is 30-50µL.

10. The kit for testing the lung cancer genetic mutation according to claim 8, wherein the reagent combination comprises the following primers and probes:
the reagent for testing the sites of the A1 group for lung cancer genetic fusion: SEQ ID NO: 1-SEQ ID NO: 44;
the reagent for testing the sites of the A2 group for the lung cancer genetic fusion: SEQ ID NO:45-SEQ ID NO:66;
the reagent for testing the sites of the A3 group for the lung cancer genetic fusion: SEQ ID NO:67-SEQ ID NO:78;
the reagent for testing the sites of the A4 group for the lung cancer genetic fusion: SEQ ID NO:79-SEQ ID NO:91;
the reagent for testing the sites of the A5 group for the lung cancer genetic fusion: SEQ ID NO:92-SEQ ID NO:104;
the reagent for testing the sites of the A6 group for the lung cancer genetic fusion: SEQ ID NO: 105-SEQ ID NO:122;
the reagent for testing the sites of the A7 group for the lung cancer genetic fusion: SEQ ID NO: 123-SEQ ID NO: 131;
the reagent for testing the sites of the A8 group for the lung cancer genetic fusion: SEQ ID NO:132-SEQ ID NO:154;
the reagent for testing the sites of the B1 group for the lung cancer genetic fusion: SEQ ID NO: 155-SEQ ID NO: 174;
the reagent for testing the sites of the B2 group for the lung cancer genetic fusion: SEQ ID NO:175-SEQ ID NO:187;
the reagent for testing the sites of the B3 group for the lung cancer genetic fusion: SEQ ID NO:188-SEQ ID NO:198.
the reagent for testing the sites of the B4 group for the lung cancer genetic fusion: SEQ ID NO:199-SEQ ID NO:220;
the reagent for testing the sites of the B5 group for the lung cancer genetic fusion: SEQ ID NO:221-SEQ ID NO:244;
the reagent for testing the sites of the B6 group for the lung cancer genetic fusion: SEQ ID NO:245-SEQ ID NO:262;
the reagent for testing the sites of the B7 group for the lung cancer genetic fusion: SEQ ID NO:263-SEQ ID NO:273.

11. The reagent kit for testing the lung cancer genetic mutation according to claim 8, **characterized in that**: it further comprises an external control reagent combination for a quality control of a sample.

12. The reagent kit for testing the lung cancer genetic mutation according to claim 11, **characterized in that**: the reagent combination comprises the following primers and probes: SEQ ID NO:274-SEQ ID NO:281.

13. A composition for testing multiple lung cancer genetic mutations, **characterized in that**: the composition comprises 281 sequences of primers and probes that is shown in the following table, and the 281 sequences are grouped and sub-packaged by two PCR 8-tube strips as follows:
| React ion tube | Name of primers and probes | Sequences of mutant primers and probes | Sequence number |
|---|---|---|---|
| | LET-A1-F1 | CAGAAGCCCATTATTCAGAGC | SEQ ID NO.001 |
| | LET-A1-R1 | ACTGCTCTAGCAGACTTTTCTG | SEQ ID NO.002 |
| | LET-A1-P1 | VIC-5 '-TTCACCAGGATCCACCTC-3 '-MGB | SEQ ID NO.003 |
| | LET-A1-F2 | TACTGTAGAGCCCACACCTGG | SEQ ID NO.004 |
| | LET-A1-F3 | TCAACCAAGCAAAAATGTCAACTCG | SEQ ID NO.005 |
| | LET-A1-F4 | GTTACCAAAACTGCAGACAAGCATA | SEQ ID NO.006 |
| | LET-A1-F5 | CCAGACAACAAGTATATAATGTCTAACTCG | SEQ ID NO.007 |
| | LET-A1-F6 | ACACAGACGGGAATGAACAGC | SEQ ID NO.008 |
| | LET-A1-F7 | CTGAAGATCATGTGGCCTCAGT | SEQ ID NO.009 |
| | LET-A1-F8 | TAGGAACGCACTCAGGCAG | SEQ ID NO.010 |
| | LET-A1-F9 | AGGACACTGTGCAGATTTTCATCC | SEQ ID NO.011 |
| LET PCR 8-tube strips-geneti c fusion -1 | LET-A1-F10 | CATAGGAACGCACTCAGGCA | SEQ ID NO.012 |
| | LET-A1-F11 | AAAGAAACTCTTTCATCTGCTGCTAAAAG | SEQ ID NO.013 |
| | LET-A1-F12 | AGGTCAAAGAATATGGCCAGAAGAG | SEQ ID NO.014 |
| | LET-A1-F13 | CGATGCCCTCAGTGAAGAACTA | SEQ ID NO.015 |
| | LET-A1-F14 | CTCACTCGTGCACATGAAAGG | SEQ ID NO.016 |
| | LET-A1-F15 | ACCTGGAGAACCAGGACCTT | SEQ ID NO.017 |
| | LET-A1-F16 | AGAATTGAATCAGGGAGATATGAAGCC | SEQ ID NO.018 |
| | LET-A1-F17 | ATGGTGCCACCACCTGC | SEQ ID NO.019 |
| | LET-A1-F18 | TGGCCTCAACCATTTCCGG | SEQ ID NO.020 |
| | LET-A1-F19 | AGCACTACGAGCTTGCTGG | SEQ ID NO.021 |
| | LET-A1-F20 | TTTCGGTCTCCTTTATCGCAGG | SEQ ID NO.022 |
| | LET-A1-F21 | GAGTTTGTTGAAGTGGGAAGATTGG | SEQ ID NO.023 |
| | LET-A1-F22 | GGGGAAAATATCAGTGCTTCACCAT | SEQ ID NO.024 |
| | LET-A1-F23 | CAGCCACCATATACAGGAGCTC | SEQ ID NO.025 |
| | LET-A1-F24 | CGAGGCGCCCTAGACATC | SEQ ID NO.026 |
| | LET-A1-F25 | GATCCGGTTCCTTGGTGTCAT | SEQ ID NO.027 |
| | LET-A1-F26 | CGGGTTGGTATCCCTTCAGG | SEQ ID NO.028 |
| | LET-A1-F27 | TGATTTACTGTTCTGGCACAGACA | SEQ ID NO.029 |
| | LET-A1-F28 | ATTCTCAGCAGACAATATCGGATCG | SEQ ID NO.030 |
| | LET-A1-F29 | CTTCAACTCAAAGAAAACAAGAGGCAG | SEQ ID NO.031 |
| | LET-A1-F30 | AAGAATCGCAAGAGAAGCACCTT | SEQ ID NO.032 |
| | LET-A1-F31 | TAAAAATTCAAAAACAGAAACAAGAAACCC TACA | SEQ ID NO.033 |
| | LET-A1-F32 | AAGCACAGCTCTTCCAGCTTAA | SEQ ID NO.034 |
| | LET-A1-F33 | ATGGGATATTCCTGGGATCTTCGTA | SEQ ID NO.035 |
| | LET-A1-F34 | CATGAGACCTCCAAACCCCTTT | SEQ ID NO.036 |
| | LET-A1-F35 | GCCTCCCAAACCCACTACC | SEQ ID NO.037 |
| | LET-A1-F36 | CGTATTTTATTGTCACAAACAACAGGAGT | SEQ ID NO.038 |
| | LET-A1-F37 | GGGGAGAAGTCCCCTGACA | SEQ ID NO.039 |
| | LET-A1-F38 | GTCGCCTCTTTGCTGCAC | SEQ ID NO.040 |
| | LET-A1-F39 | TCCTTCAGGCATTGCTACTCTG | SEQ ID NO.041 |
| | LET-A1-F40 | AAGAACGTTGGGGAGAGTGTG | SEQ ID NO.042 |
| | LET-A1-R2 | GGGCTCTGCAGCTCCAT | SEQ ID NO.043 |
| | LET-A1-P2 | FAM-5'-CGGAAGCACCAGGAG-3 '-MGB | SEQ ID NO.044 |
| | LET-A2-F1 | GAAGCCCATTATTCAGAGCG | SEQ ID NO.045 |
| | LET-A2-R1 | TGGTACTGCTCTAGCAGACTT | SEQ ID NO.046 |
| | LET-A2-P1 | VIC-5 '-TTCACCAGGATCCACCTC-3 '-MGB | SEQ ID NO.047 |
| | LET-A2-F2 | GATCGGTAGCCAAGCTGGAA | SEQ ID NO.048 |
| LET PCR 8-tube strips-geneti c fusion -2 | LET-A2-F3 | GTTGGGGAGAGTGTGGCA | SEQ ID NO.049 |
| | LET-A2-F4 | TACATCACAGACTGTTTCCACTCC | SEQ ID NO.050 |
| | LET-A2-F5 | GAAGTCCAAGTTGCTTCTCAGTCT | SEQ ID NO.051 |
| | LET-A2-F6 | TTCTGGGCTGGGTGTGAC | SEQ ID NO.052 |
| | LET-A2-F7 | TTGTTGGGTTTCGAGGCCAA | SEQ ID NO.053 |
| | LET-A2-F8 | TCGAGAGCAAGTTTAAGAAGGAGC | SEQ ID NO.054 |
| | LET-A2-F9 | TATGTCAGCGTTTGGCTTAACAGA | SEQ ID NO.055 |
| | LET-A2-F10 | GCTATGGGGAGGTCAAGGTC | SEQ ID NO.056 |
| | LET-A2-F11 | GAGGAAGATCGCCTTGGAGT | SEQ ID NO.057 |
| | LET-A2-F12 | CTTGCGGGAAAAGGAGAGCT | SEQ ID NO.058 |
| | LET-A2-F13 | GAAGTCCCCTGACAGTGCC | SEQ ID NO.059 |
| | LET-A2-F14 | CCACCAGGAACACCCATCAT | SEQ ID NO.060 |
| | LET-A2-F15 | ACACACGAGCTGACCTCTCT | SEQ ID NO.061 |
| | LET-A2-F16 | TGCCAGCGTGAGAACCAG | SEQ ID NO.062 |
| | LET -A2-R2 | CCCAAAAGGTGTTTCGTCCTTC | SEQ ID NO.063 |
| | LET -A2-R3 | CAATGTCATGAAATGCAGGGACAT | SEQ ID NO.064 |
| | LET -A2-P2 | FAM-5'-CAGCACATCTGGAGACC-3'-MGB | SEQ ID NO.065 |
| | LET -A2-P3 | FAM-5'-GGCTCCAGAGGATG-3'-MGB | SEQ ID NO.066 |
| LET PCR 8-tube strips-geneti c fusion -3 | LET-A3-F1 | CCATTATTCAGAGCGAGTATGGA | SEQ ID NO.067 |
| | LET-A3-R1 | TGGTACTGCTCTAGCAGACTT | SEQ ID NO.068 |
| | LET-A3-PI | VIC-5 '-TTCACCAGGATCCACCTC-3 '-MGB | SEQ ID NO.069 |
| | LET-A3-F2 | CAGTCACCAAATTCATCAGTGCC | SEQ ID NO.070 |
| | LET-A3-F3 | AGAGTGTGGCAGCTGCC | SEQ ID NO.071 |
| | LET-A3-F4 | ATCAGGGAGATATGAAGCCTCCAA | SEQ ID NO.072 |
| | LET-A3-R2 | GGGCCAACACCTTGTCTTGA | SEQ ID NO.073 |
| | LET-A3-R3 | CATTGGAGATGTGATGGAGTGGG | SEQ ID NO.074 |
| | LET-A3-R4 | TCCTTCGCCTAGCTCCCTTT | SEQ ID NO.075 |
| | LET-A3-P2 | FAM-5'-TTTGGATTTGGGAAAGTA-3 '-MGB | SEQ ID NO.076 |
| | LET-A3-P3 | FAM-5'-CGTTATCAGCAATGAT-3'-MGB | SEQ ID NO.077 |
| | LET-A3-P4 | FAM-5'-AGCGACATAACATTGTTC-3 '-MGB | SEQ ID NO.078 |
| LET PCR 8-tube strips-geneti c | LET-A4-F1 | CCATTATTCAGAGCGAGTATGGA | SEQ ID NO.079 |
| | LET-A4-R1 | ATGGTACTGCTCTAGCAGAC | SEQ ID NO.080 |
| | LET-A4-P1 | VIC-5 '-TTCACCAGGATCCACCTC-3 '-MGB | SEQ ID NO.081 |
| | LET-A4-F2 | ACAGCCGGAGGTCATACTG | SEQ ID NO.082 |
| | LET-A4-F3 | CTCCCCGCCTGAAGAGC | SEQ ID NO.083 |
| | LET-A4-F4 | AGATCATGTGGCCTCAGTGAAAA | SEQ ID NO.084 |
| | LET-A4-F5 | AACGTTGGGGAGAGTGTGG | SEQ ID NO.085 |
| fusion -4 | LET-A4-F6 | GCCCAACACTGTACCTCAGTT | SEQ ID NO.086 |
| | LET-A4-F7 | AGCAGATGAGGAAGATCGCC | SEQ ID NO.087 |
| | LET-A4-R2 | GATGTGGTGCAGTGGGC | SEQ ID NO.088 |
| | LET-A4-R3 | CTCACCCAGTTCTCGCTTCA | SEQ ID NO.089 |
| | LET-A4-P2 | FAM-5 '-TGGCTGTCATCAGTGGT-3 '-MGB | SEQ ID NO.090 |
| | LET-A4-P3 | FAM-5'-CATTAAGAGGAGAGACATC-3'-MGB | SEQ ID NO.091 |
| LET PCR 8-tube strips-geneti c fusion -5 | LET-A5-F1 | TATTCAGAGCGAGTATGGAGC | SEQ ID NO.092 |
| | LET-A5-R1 | GTACTGCTCTAGCAGACTTTTCT | SEQ ID NO.093 |
| | LET-A5-P1 | VIC-5 '-TTCACCAGGATCCACCTC-3 '-MGB | SEQ ID NO.094 |
| | LET-A5-F2 | TCGTGTGCTCCCTGGATATTCTTA | SEQ ID NO.095 |
| | LET-A5-F3 | GTCAAGGCTCCTGAGACCTTTG | SEQ ID NO.096 |
| | LET-A5-F4 | CAGGCACTCCTTGGAGCA | SEQ ID NO.097 |
| | LET-A5-F5 | ATCTGATGACTTTGAGCTGTCTGG | SEQ ID NO.098 |
| | LET-A5-F6 | AGGGCAGCAACATCTTTGAGAG | SEQ ID NO.099 |
| | LET-A5-F7 | CGGCTGCAGGACTATGAGG | SEQ ID NO.100 |
| | LET-A5-R2 | TTGAATTTTTACTCCCTTCTAGTAATTTGGGA AT | SEQ ID NO.101 |
| | LET-A5-R3 | TTGTAACAACCAGAAATATTCCAACTATAAT AGTAAGT | SEQ ID NO.102 |
| | LET-A5-P2 | FAM-5'-CCCAAATAAACCAGGCAT-3 '-MGB | SEQ ID NO.103 |
| | LET-A5-P3 | FAM-5′-CCAGAAACAAGTTTCATACTT-3′- MGB | SEQ ID NO.104 |
| LET PCR 8-tube strips-geneti c | LET-A6-F1 | CAGAAGCCCATTATTCAGAGC | SEQ ID NO.105 |
| | LET-A6-R1 | ACTGCTCTAGCAGACTTTTCTG | SEQ ID NO.106 |
| | LET-A6-P1 | VIC-5 '-TTCACCAGGATCCACCTC-3 '-MGB | SEQ ID NO.107 |
| | LET-A6-F2 | CTGAGAGATCGGTAGCCAAGC | SEQ ID NO.108 |
| | LET-A6-F3 | GTCACATCTTCAGGTGCTGGATT | SEQ ID NO.109 |
| | LET-A6-F4 | CCTGGTGCTAGTTGCAAAGACA | SEQ ID NO.110 |
| | LET-A6-F5 | ACGGCTGAAGAAGCAACTGAG | SEQ ID NO.111 |
| | LET-A6-F6 | CAGGCACTCCTTGGAGCAAA | SEQ ID NO.112 |
| fusion -6 | LET-A6-F7 | CACTGAGAAAAGAAGAAGAACAAGCTACA | SEQ ID NO.113 |
| | LET-A6-F8 | CTGCGGCTGCAGGACTAT | SEQ ID NO.114 |
| | LET-A6-F9 | GTTTCACAGTTACTTTCACTTCCCGA | SEQ ID NO.115 |
| | LET-A6-F10 | GAAAAAGAAGCCCTCAATCACCG | SEQ ID NO.116 |
| | LET-A6-F11 | CAAGAAAAATTGGTTTGCAAGATGAAAGG | SEQ ID NO.117 |
| | LET-A6-F12 | GAGATAGTGTCACCTGCCCTACT | SEQ ID NO.118 |
| | LET-A6-F13 | TCTCAAAAAGAAGATAAATATGAGGAAGAA ATCAAGAT | SEQ ID NO.119 |
| | LET-A6-F14 | CTTGGAACCACCTGGAGAACC | SEQ ID NO.120 |
| | LET-A6-R2 | CTGTCACCCCTTCCTTGGC | SEQ ID NO.121 |
| | LET-A6-P2 | FAM-5'-CATAGAAGATTAAAGAATC-3'-MGB | SEQ ID NO.122 |
| LET PCR 8-tube strips-geneti c fusion -7 | LET-A7-F1 | GAAGCCCATTATTCAGAGCG | SEQ ID NO.123 |
| | LET-A7-R1 | ACTGCTCTAGCAGACTTTTCTG | SEQ ID NO.124 |
| | LET-A7-P1 | VIC-5'-TTCACCAGGATCCACCTC-3 '-MGB | SEQ ID NO.125 |
| | LET-A7-F2 | AATGCCTGAGCGGGACATG | SEQ ID NO.126 |
| | LET-A7-R2 | GCCTTGTCCCTCCTTCAAGG | SEQ ID NO.127 |
| | LET-A7-F3 | CAACAGCACTGTTATTACTACTTGGGT | SEQ ID NO.128 |
| | LET-A7-R3 | GATACTGCACTTGTCGGCATG | SEQ ID NO.129 |
| | LET-A7-P2 | FAM-5'-AAGCAAATTAAAGATCAGT-3 '-MGB | SEQ ID NO.130 |
| | LET-A7-Block1 | TTCGCTACGATGCAAGAGTACACACTCCTCA TTTGGATAGGC-NH2 | SEQ ID NO.131 |
| LET PCR 8-tube strips-geneti c fusion -8 | LET-A8-F1 | CAGAAGCCCATTATTCAGAGC | SEQ ID NO.132 |
| | LET-A8-R1 | GTACTGCTCTAGCAGACTTTTCT | SEQ ID NO.133 |
| | LET-A8-P1 | VIC-5 '-TTCACCAGGATCCACCTC-3 '-MGB | SEQ ID NO.134 |
| | LET-A8-F2 | TAAAAGACCTTGCAGAAATAGGAATTGCT | SEQ ID NO.135 |
| | LET-A8-F3 | GGAAGAAAATGAAAAGGAGTTAGCAGC | SEQ ID NO.136 |
| | LET-A8-F4 | CTCCTTTCTTGAAAATAATCTTGAACAGCTC | SEQ ID NO.137 |
| | LET-A8-F5 | CCTGCGCAAACTCTTTGTTCAG | SEQ ID NO.138 |
| | LET-A8-F6 | AGGCACTGCAGGAGGAGA | SEQ ID NO.139 |
| | LET-A8-F7 | TATTGGGCCCTTCCTGGAGAA | SEQ ID NO.140 |
| | LET-A8-F8 | TGTAAAACAAGAAAAAACAGAGGATGGC | SEQ ID NO.141 |
| | LET-A8-F9 | CACTCCCTCCAGCTGGC | SEQ ID NO.142 |
| | LET-A8-F10 | CTGCCTGCCACACATTGTTC | SEQ ID NO.143 |
| | LET-A8-F11 | AAGTGACTCTTCAGATCCCTGCT | SEQ ID NO.144 |
| | LET-A8-F12 | CCACTGGGTCACCCCTTG | SEQ ID NO.145 |
| | LET-A8-F13 | CTGATGGCTTGAAGGCGGAA | SEQ ID NO.146 |
| | LET-A8-F14 | CCTGTCATGAGACCTCCAAACC | SEQ ID NO.147 |
| | LET-A8-F15 | CGAAGTGAAAATAAATTGATTTTAATGAAAA CACAGC | SEQ ID NO.148 |
| | LET-A8-F16 | AAAGGTCGCCTCTTTGCT | SEQ ID NO.149 |
| | LET-A8-F17 | CAGTAAGGCCAAAAATGTGCATACTC | SEQ ID NO.150 |
| | LET-A8-F18 | CCATGAAATGGAAAAGGAGCACTTAAATAA G | SEQ ID NO.151 |
| | LET-A8-F19 | ACCAATCCAGAAAACCTTCCATCG | SEQ ID NO.152 |
| | LET -A8-R2 | TCTCCTAGAGTTTTTCCAAGAACCAAG | SEQ ID NO.153 |
| | LET -A8-P2 | FAM-5'-CCAAAGTGGGAATTC-3 '-MGB | SEQ ID NO.154 |
| LET PCR 8-tube strips-geneti c mutat ion-1 | LET-B1-F1 | GTCTCCAGATCTCAGTAAGGTACG | SEQ ID NO.155 |
| | LET-B1-R1 | GGGAAAGAGTGGTCTCTCATCTC | SEQ ID NO.156 |
| | LET-B1-P1 | VIC-5 '-CTGTCCAAAAGCCATGAA-3 '-MGB | SEQ ID NO.157 |
| | LET-B1-F2 | CCATGCGAAGCCACACTGACG | SEQ ID NO.158 |
| | LET-B1-R2 | GGCACACGTGGGGGTTGTGCACCA | SEQ ID NO.159 |
| | LET-B1-R3 | GGCACACGTGGGGGTTGTGCAAGA | SEQ ID NO.160 |
| | LET-B1-R4 | GGCACACGTGGGGGTTGTGAACGA | SEQ ID NO.161 |
| | LET-B1-R5 | GGCACACGTGGGGGTTGTGCATGA | SEQ ID NO.162 |
| | LET-B1-Block1 | CAGCAGGCGGCACACGTGGGGGTTGTCCAC GA-NH2 | SEQ ID NO.163 |
| | LET-B1-P2 | ROX-5 '-CCATCACGTAGGCTTCCTGG-3 '-BHQ2 | SEQ ID NO.164 |
| | LET-B1-R6 | ATGAGAAAAGGTGGGCCTGAGGT | SEQ ID NO.165 |
| | LET-B1-F3 | TTCCCGTCGCTATCAAGACATCTCCGAAA | SEQ ID NO.166 |
| | LET-B1-F4 | CGTCGCTATCAAGGCATCTCCG | SEQ ID NO.167 |
| | LET-B1-F5 | TCCCGTCGCTATCAAAACATCTCCGAAA | SEQ ID NO.168 |
| | LET-B1-F6 | AATTCCCGTCGCTATCAAGGCTCCGAAAGCC | SEQ ID NO.169 |
| | LET-B1-F7 | CCCGTCGCTATCAAGGAGCAATCTCCGAA | SEQ ID NO.170 |
| | LET-B1-F8 | CCCGTCGCTATCAAGGAAGCAACATCTCC | SEQ ID NO.171 |
| | LET-B1-F9 | CCCGTCGCTATCAAGGAACCGAAAGCC | SEQ ID NO.172 |
| | LET-B1-Block2 | TCCCGTCGCTATCAAGGAATTAAGAGAAGCA AC-NH2 | SEQ ID NO.173 |
| | LET-B1-P3 | FAM-5'-AAGCCAACAAGGAAATCCT-3'-MGB | SEQ ID NO.174 |
| LET PCR 8-tube strips-geneti c mutat ion-2 | LET-B2-F1 | CAACCCTGCCCTGTGCAA | SEQ ID NO.175 |
| | LET-B2-R1 | GGTGGTTCTGGAAGTCCAT | SEQ ID NO.176 |
| | LET-B2-P1 | HEX-5′-AGCATCCAGTGGCGGACATAGTC-3′- BHQ1 | SEQ ID NO.177 |
| | LET-B2-F2 | GCAGCATGTCAAGATCACAGATTTTGTGCG | SEQ ID NO.178 |
| | LET-B2-R2 | ACTTTGCCTCCTTCTGCATGGTATT | SEQ ID NO.179 |
| | LET-B2-P2 | FAM-5′-CAGAAGGAGGCAAAGTAAGGAG-3′- BHQ1 | SEQ ID NO.180 |
| | LET-B2-Block1 | AGATCACAGATTTTGGGCTGGCCAAACTGCT GG-NH2 | SEQ ID NO.181 |
| | LET-B2-R3 | CACCGTGCCGAACGCACCGGAGCT | SEQ ID NO.182 |
| | LET-B2-R4 | TATACGTGCCGAACGCACCGGACCA | SEQ ID NO.183 |
| | LET-B2-F3 | CTGAGGTGACCCTTGTCTCTGTGTTCT | SEQ ID NO.184 |
| | LET-B2-P3 | ROX-5 '-CTCTCTTGAGGATCTTG-3 '-MGB | SEQ ID NO.185 |
| | LET-B2-P4 | ROX-5′-CCCAACCAAGCTCTCTTGAGGATCTT- 3′-BHQ2 | SEQ ID NO.186 |
| | LET-B2-Block2 | ACGCACCGGAGCCCAGCACTTTGT -NH2 | SEQ ID NO.187 |
| LET PCR 8-tube | LET-B3-F1 | GTCTCCAGATCTCAGTAAGGTACG | SEQ ID NO.188 |
| | LET-B3-R1 | GGGAAAGAGTGGTCTCTCATCTC | SEQ ID NO.189 |
| | LET-B3-P1 | VIC-5 '-CTGTCCAAAAGCCATGAA-3 '-MGB | SEQ ID NO.190 |
| | LET-B3-F2 | CAACAGATTTTGGGCTGGCCAGACA | SEQ ID NO.191 |
| strips-geneti c mutat ion-3 | LET-B3-R2 | GTTAAACAATACAGCTAGTGGGAAGGC | SEQ ID NO.192 |
| | LET-B3-P2 | ROX-5′-CAGAAGGAGGCAAAGTAAGGAGG-3′- BHQ2 | SEQ ID NO.193 |
| | LET-B3-F3 | CTACTCCACCGTGCAGCTCATCCT | SEQ ID NO.194 |
| | LET-B3-F4 | ACTACTCCACCGTGCAACTCATCCT | SEQ ID NO.195 |
| | LET-B3-R3 | TATCTCCCTTCCCTGATTACC | SEQ ID NO.196 |
| | LET-B3-Block1 | CTCACCTCCACCGTGCAGCTCATCAC-NH2 | SEQ ID NO.197 |
| | LET-B3-P3 | FAM-5′-CTCATGCCCTTCGGCTGCCTCC-3′- BHQ1 | SEQ ID NO.198 |
| LET PCR 8-tube strips-geneti c mutat ion-4 | LET-B4-F1 | CGTGATGGCCAGCGTGGACGGT | SEQ ID NO.199 |
| | LET-B4-F2 | GATGGCCAGCGTGGACAGCGTGGA | SEQ ID NO.200 |
| | LET-B4-F3 | TGGCCAGCGTGATGGCCAG | SEQ ID NO.201 |
| | LET-B4-F4 | TGGCCAGCGTGGACGGGT | SEQ ID NO.202 |
| | LET-B4-F5 | TGATGGCCAGCGTGGACGGT | SEQ ID NO.203 |
| | LET-B4-F6 | CTACGTGATGGCCAGTGTGG | SEQ ID NO.204 |
| | LET-B4-F7 | TGGCCAGCGTGGACTAC | SEQ ID NO.205 |
| | LET-B4-F8 | TGGCCAGCGTGGACACCA | SEQ ID NO.206 |
| | LET-B4-F9 | ATGGCCAGCGTGGACAAACT | SEQ ID NO.207 |
| | LET-B4-F10 | TGATGGCCAGCGTGGACGCACC | SEQ ID NO.208 |
| | LET-B4-F11 | TGGCCAGCGTGGACAGCGTCG | SEQ ID NO.209 |
| | LET-B4-F12 | TGATGGCCAGCGTGGACAGTC | SEQ ID NO.210 |
| | LET-B4-R1 | GACATAGTCCAGGAGGCAGCCG | SEQ ID NO.211 |
| | LET-B4-P1 | FAM-5 '-CGCCTGCTGGGCATCTGC-3 '-BHQ1 | SEQ ID NO.212 |
| | LET-B4-Block1 | GATGGCCAGCGTGGACAACCCC-NH2 | SEQ ID NO.213 |
| | LET-B4-F13 | CAGTAAAAATAGGTGATTTTGGTCTAGCTAC CGA | SEQ ID NO.214 |
| | LET-B4-R2 | GCATCTCAGGGCCAAAAATTTAATCAGTG | SEQ ID NO.215 |
| | LET -B4-P2 | ROX-5′-CTCGATGGAGTGGGTCCCATCAG-3′- BHQ2 | SEQ ID NO.216 |
| | LET-B4-Block2 | TTTTGGTCTAGCTACAGTGAAATCTCGATGG AGT-NH2 | SEQ ID NO.217 |
| | LET-B4-F14 | TCAGGCCAAAAGTGTGATCCAA | SEQ ID NO.218 |
| | LET -B4-R3 | GGTCTGAGGCTGTTCACTGACTTA | SEQ ID NO.219 |
| | LET -B4-P3 | HEX-5′-TCTGGCAGTTCTCCTCTCCTGCAC-3′- BHQ1 | SEQ ID NO.220 |
| LET PCR 8-tube strips-geneti c mutat ion-5 | LET-B5-F1 | GCCAGCGTGGACAACCCCCACCAC | SEQ ID NO.221 |
| | LET -B5-F2 | CGTGGACAACCCCCAACA | SEQ ID NO.222 |
| | LET -B5-F3 | CGTGGACAACCCCCACTAC | SEQ ID NO.223 |
| | LET -B5-F4 | TGGACAACCCCCACCCCCAC | SEQ ID NO.224 |
| | LET -B5-F5 | CGTGGACAACCCGGACA | SEQ ID NO.225 |
| | LET -B5-F6 | GGACAACCCCCACGGCAACCC | SEQ ID NO.226 |
| | LET -B5-F7 | CAACCCCCACGTGCCACG | SEQ ID NO.227 |
| | LET -B5-F8 | GGCCAGCGTGGACAACACC | SEQ ID NO.228 |
| | LET -B5-F9 | GATGGCCAGCGTGGGTT | SEQ ID NO.229 |
| | LET-B5-F10 | GTGATGGCCAGCGTGGGGGTC | SEQ ID NO.230 |
| | LET-B5-F11 | GCCTACGTGATGGCCAGTGTGGC | SEQ ID NO.231 |
| | LET -B5-F12 | CGTGATGGCCAGCGTGGAGCGTG | SEQ ID NO.232 |
| | LET -B5-F13 | CGTGATGGCCAGCGTGGCTGGT | SEQ ID NO.233 |
| | LET-B5-F14 | GACAACCCCCACGTGCACGT | SEQ ID NO.234 |
| | LET-B5-F15 | CAGCGTGGACAACCCCGTT | SEQ ID NO.235 |
| | LET -B5-R1 | GACATAGTCCAGGAGGCAGCCG | SEQ ID NO.236 |
| | LET-B 5-PI | FAM-5'-CGCCTGCTGGGCATCTGC-3'-BHQ1 | SEQ ID NO.237 |
| | LET-B5-F16 | GATAGTGTATTAACCTTATGTGTGACATGTT C | SEQ ID NO.238 |
| | LET -B5-R2 | CAAGGCACTCTTGCCTACGCGACA | SEQ ID NO.239 |
| | LET -B5-P2 | ROX-5 '-CTGCTGAAAATGACTGAAT-3 '-MGB | SEQ ID NO.240 |
| | LET-B5-Block1 | CACTCTTGCCTACGCCACCAGCTCCAACTAC CA-NH2 | SEQ ID NO.241 |
| | LET-B5-F17 | CTCGGCCTCCCAAGGTGTT | SEQ ID NO.242 |
| | LET-B5-R3 | CCACACAGCAGCAAGTGATAGTT | SEQ ID NO.243 |
| | LET-B5-P3 | VIC-5′-TAGGTGCCTAGCCCATGGTGCCTA-3′- BHQ1 | SEQ ID NO.244 |
| LET PCR 8-tube strips-geneti c mutat ion-6 | LET-B6-F1 | GATAGTGTATTAACCTTATGTGTGACATGTT C | SEQ ID NO.245 |
| | LET-B6-R1 | CGTCAAGGCACTCTTGCCTACGCTACG | SEQ ID NO.246 |
| | LET-B6-R2 | TCGTCAAGGCACTCTTGCCTACGCGAA | SEQ ID NO.247 |
| | LET-B6-R3 | CGTCAAGGCACTCTTGCCTACGTCAG | SEQ ID NO.248 |
| | LET-B6-R4 | TATCGTCAAGGCACTCTTGCCTAAGCA | SEQ ID NO.249 |
| | LET-B6-P1 | ROX-5 '-CTCCAACTACCACAAGTT-3 '-MGB | SEQ ID N0.250 |
| | LET-B6-F2 | ATGGCTGTGGTTTGTGATGGTT | SEQ ID NO.251 |
| | LET-B6-R5 | ACCAGCCATCACGTAAGCCATCAC | SEQ ID NO.252 |
| | LET-B6-R6 | ACATATGGGGAGCCCACACACA | SEQ ID NO.253 |
| | LET-B6-R7 | GAGACATATGGGGAGCCCACACG | SEQ ID NO.254 |
| | LET-B6-R8 | GGAGACATATGGGGAGCCCACAA | SEQ ID NO.255 |
| | LET-B6-R9 | GAGACATATGGGGAGCCCACACAG | SEQ ID NO.256 |
| | LET-B6-R10 | GGAGACATATGGGGAGCCCCTC | SEQ ID NO.257 |
| | LET-B6-P2 | FAM-5′- CGTATGCTTCCTGGGGACAAGGGTACG-3′- BHQ1 | SEQ ID NO.258 |
| | LET-B6-P3 | FAM-5'-CTCTCAGCGTACCCTTG-3'-MGB | SEQ ID NO.259 |
| | LET-B6-F3 | CTCGGCCTCCCAAGGTGTT | SEQ ID NO.260 |
| | LET-B6-R11 | CCACACAGCAGCAAGTGATAGTT | SEQ ID NO.261 |
| | LET-B6-P4 | VIC-5′-TAGGTGCCTAGCCCATGGTGCCTA-3′- BHQ1 | SEQ ID NO.262 |
| LET PCR | LET-B7-F1 | TGAATATAAACTTGTGGTAGTTGGAGCCGA | SEQ ID NO.263 |
| | LET-B7-F2 | GAATATAAACTTGTGGTAGTTGGAGCGA | SEQ ID NO.264 |
| 8-tube strips-geneti c mutat ion-7 | LET-B7-R1 | CAAGATTTACCTCTATTGTTGGATCATATTC | SEQ ID NO.265 |
| | LET-B7-P1 | ROX-5'-CAGCTAATTCAGAATCAT-3'-MGB | SEQ ID NO.266 |
| | LET-B7-Block1 | GACTGAATATAAACTTGTGGTAGTTGGAGCT GG-NH2 | SEQ ID NO.267 |
| | LET-B7-F3 | CTCGGCCTCCCAAGGTGTT | SEQ ID NO.268 |
| | LET-B7-R2 | CCACACAGCAGCAAGTGATAGTT | SEQ ID NO.269 |
| | LET-B7-P2 | VIC-5′-TAGGTGCCTAGCCCATGGTGCCTA-3′- BHQ1 | SEQ ID NO.270 |
| | LET-B7-F4 | TGGTGTGGGCTCCCCTGGCTC | SEQ ID NO.271 |
| | LET-B7-R3 | TCTAAGAGGCAGCCATAGGGCATA | SEQ ID NO.272 |
| | LET-B7-P3 | FAM-5′-CCCATATGTCTCCCGCCTTCTGGGC- 3′-BHQ1 | SEQ ID NO.273 |
| LET PCR 8-tube strips-geneti c mutat ion-8 | LET B8-F1 | CAACCCTGCCCTGTGCAA | SEQ ID NO.274 |
| | LET B8-R1 | GGTGGTTCTGGAAGTCCAT | SEQ ID NO.275 |
| | LET B8-P1 | FAM-5′-AGCATCCAGTGGCGGGACATAGTC- 3′-BHQ1 | SEQ ID NO.276 |
| | LET B8-P2 | ROX-5′-AGCATCCAGTGGCGGACATAGTC- 3′-BHQ2 | SEQ ID NO.277 |
| | LET B8-F2 | AGCTCTCTTGAGGATCTTGAAGGAA | SEQ ID NO.278 |
| | LET B8-R2 | CCTGTGCCAGGGACCTTACCT | SEQ ID NO.279 |
| | LET B8-P3 | FAM-5'-CTCCGGTGCGTTCGGCACG-3 '-BHQ1 | SEQ ID NO.280 |
| | LET B8-P4 | ROX-5'-CTCCGGTGCGTTCGGCACG-3'-BHQ2 | SEQ ID NO.281 |

14. The composition according to claim 13, **characterized in that** a reaction system of primers and probes comprises:
(3) a reverse transcription reaction system: 5-25µL of purified water, 5-15µL of 5×reverse transcription buffer, 20-150µmol of each primer, and 100-200U of reverse transcriptase, and a total volume is 20-30µL;
(4) a PCR reaction system: 15-45µL of purified water, 5-15µL of 10×PCR buffer, 1-10mmol of MgCl₂, 1-20pmol of each pair of primers, 1-20pmol of each pair of probes, 10-100pmol of dNTPs, and 1-10U of Taq enzyme, and a total volume is 30-50µL.

15. A reagent kit, it comprises the 281 sequences of the primers and the probes according to claim 13.

16. The reagent kit according to claim 15, **characterized in that**: the reagent kit comprises two PCR 8-tube strips, and the two PCR 8-tube strips test a lung cancer RNA genetic fusion and a lung cancer DNA genetic mutation respectively.

17. The reagent kit according to claim 15 or 16, **characterized in that**: test reagents for testing the lung cancer RNA genetic fusion in the PCR 8-tube strips comprise: 1 tube of a test reagent of each of ALK, NTRK1, NTRK2 and NTRK3 fusion genes, 2 tubes of a test reagent of a ROS1 fusion gene, 1 tube of a test reagent of an MET exon 14 skipping genetic mutation, and 1 tube of a test reagent of a RET fusion gene.

18. The reagent kit according to claims 15 or 16, **characterized in that**: test reagents for testing the lung cancer DNA genetic mutation in the PCR 8-tube strips comprise: 1 tube of a test reagent of each of EGFR, EGFR, EGFR, EGFR/BRAF, EGFR/KRAS, HER2/KRAS, and HER2/KRAS mutation genes, and 1 tube of a test reagent of an external control.

19. A method for using the reagent kit according to any one of claims 15-18, wherein it comprises:
(1) extracting DNA and RNA from test samples, the test samples comprise a fresh pathological tissue, a paraffin-embedded tissue or a paraffin section of a tissue;
(2) mixing the RNA with the reverse transcriptase, and adding a reverse transcription reaction solution separately to obtain cDNA;
(3) mixing the DNA and the cDNA with mixed enzyme separately, and adding into PCR 8-tube strips;
(4) after amplifying by a fluorescent PCR amplification device, judging test results according to a displayed Ct value.
